# EUROPEAN PATENT SPECIFICATION

(11) **EP 1 801 232 B1**
(45) Date of publication and mention of the grant of the patent: **14.03.2012**
(21) Application number: 05790422.9
(22) Date of filing: 30.09.2005
(51) Int. Cl.: C12N 9/64, C12Q 1/37, G01N 33/573

(54) **METHOD OF SCREENING TRANSMEMBRANE ENZYME INHIBITORY SUBSTANCE**
VERFAHREN ZUM SCREENING EINES TRANSMEMBRANENZYMHEMMSTOFFS
PROCÉDÉ DE TRIAGE D'UNE SUBSTANCE INHIBITRICE D'ENZYME TRANSMEMBRANE

(30) Priority: 01.10.2004 JP 2004290784
(43) Date of publication of application: 27.06.2007
(73) Proprietor: Takeda Pharmaceutical Company Limited, Osaka-shi, Osaka 541-0045 (JP)
(72) Inventor: TARUI, Naoki, Fujisawa, Kanagawa 251-8555 (JP)
(74) Representative: Keller, Günter
(86) International application number: PCT/JP2005/018587
(87) International publication number: WO 2006/038684

(56) References cited:
- WO-A-00/24780
- WO-A-99/43711
- WO-A1-03/055521
- GB-A- 2 367 060
- JP-A- 2002 037 731
- YAN RIQIANG ET AL: "The transmembrane domain of the Alzheimer's beta-secretase (BACE1) determines its late Golgi localization and access to beta-amyloid precursor protein (APP) substrate" JOURNAL OF BIOLOGICAL CHEMISTRY, vol. 276, no. 39, 28 September 2001 (2001-09-28), pages 36788-36796, XP002501363 ISSN: 0021-9258
- COLL RJ ET AL: 'Inhibition of the sarcoplasmic reticulum Ca2+-ATPase by Reactive Red 120.' BIOCHIM BIOPHYS ACTA. vol. 904, no. 2, 1987, pages 227 - 238, XP002993319
- TIAN G ET AL: 'The mechanism of gamma-secretase: multiple inhibitor binding sites for transition state analogs and small molecule inhibitors.' J BIOL CHEM. vol. 278, no. 31, 2003, pages 28968 - 28975, XP002993320
- YANG H ET AL: "Effects of transmembrane domain and glycosylation of BACE on its enzymatic activity and inhibitor binding", ABSTRACTS OF THE ANNUAL MEETING OF THE SOCIETY FOR NEUROSCIENCE, SOCIETY FOR NEUROSCIENCE, WASHINGTON, DC, US, vol. 2003, 1 January 2003 (2003-01-01), XP008097980, ISSN: 0190-5295
- KOPCHO LISA M ET AL: "Comparative studies of active site-ligand interactions among various recombinant constructs of human beta-amyloid precursor protein cleaving enzyme.", ARCHIVES OF BIOCHEMISTRY AND BIOPHYSICS 15 FEB 2003 LNKD- PUBMED:12573291, vol. 410, no. 2, 15 February 2003 (2003-02-15), pages 307-316, ISSN: 0003-9861
- OH M ET AL: "Cell-based assay for beta-secretase activity", ANALYTICAL BIOCHEMISTRY, ACADEMIC PRESS INC, NEW YORK LNKD- DOI:10.1016/J.AB.2003.08.036, vol. 323, no. 1, 1 December 2003 (2003-12-01), pages 7-11, XP004472220, ISSN: 0003-2697
- YAN RIQIANG ET AL: "The transmembrane domain of the Alzheimer's beta-secretase (BACE1) determines its late Golgi localization and access to beta-amyloid precursor protein (APP) substrate", JOURNAL OF BIOLOGICAL CHEMISTRY, THE AMERICAN SOCIETY OF BIOLOGICAL CHEMISTS, INC, US, vol. 276, no. 39, 28 September 2001 (2001-09-28), pages 36788-36796, XP002501363, ISSN: 0021-9258, DOI: 10.1074/JBC.M104350200

### SEQUENCE LISTING

<110> Takeda Pharmaceutical Company Limited
<120> Method of Screening Transmembrane Enzyme Inhibitory Substance
<130> 09828
<150> JP 2004-290784
   <151> 2004-10-01
<160> 29
<170> PatentIn version 3.2
<210> 1
   <211> 1503
   <212> DNA
   <213> Homo sapiens
<220>
   <221> CDS
   <222> (1).. (1503)
<400> 1
   atg gcc caa gcc ctg ccc tgg ctc ctg ctg tgg atg ggc gcg gga gtg      48
   Met Ala Gln Ala Leu Pro Trp Leu Leu Leu Trp Met Gly Ala Gly Val
   1               5                   10                  15
   ctg cct gcc cac ggc acc cag cac ggc atc cgg ctg ccc ctg aga agt      96
   Leu Pro Ala His Gly Thr Gln His Gly Ile Arg Leu Pro Leu Arg Ser
               20                  25                  30
   ggt ctg ggg ggc gcc ccc ctg ggg ctg cgg ctg ccc cgg gag acc gac     144
   Gly Leu Gly Gly Ala Pro Leu Gly Leu Arg Leu Pro Arg Glu Thr Asp
           35                  40                  45
   gaa gag ccc gag gag ccc ggc cgg agg ggc agc ttt gtg gag atg gtg     192
   Glu Glu Pro Glu Glu Pro Gly Arg Arg Gly Ser Phe Val Glu Met Val
       50                  55                  60
   gac aac ctg agg ggc aag tcg ggg cag ggc tac tac gtg gag atg acc     240
   Asp Asn Leu Arg Gly Lys Ser Gly Gln Gly Tyr Tyr Val Glu Met Thr
   65                  70                  75                  80
   gtg ggc agc ccc ccg cag acg ctc aac atc ctg gtg gat aca ggc agc     288
   Val Gly Ser Pro Pro Gln Thr Leu Asn Ile Leu Val Asp Thr Gly Ser
                   85                  90                  95
   agt aac ttt gca gtg ggt gct gcc ccc cac ccc ttc ctg cat cgc tac     336
   Ser Asn Phe Ala Val Gly Ala Ala Pro His Pro Phe Leu His Arg Tyr
               100                 105                 110
   tac cag agg cag ctg tcc agc aca tac cgg gac ctc cgg aag ggt gtg     384
   Tyr Gln Arg Gln Leu Ser Ser Thr Tyr Arg Asp Leu Arg Lys Gly Val
           115                 120                 125
   tat gtg ccc tac acc cag ggc aag tgg gaa ggg gag ctg ggc acc gac     432
   Tyr Val Pro Tyr Thr Gln Gly Lys Trp Glu Gly Glu Leu Gly Thr Asp
       130                 135                 140
   ctg gta agc atc ccc cat ggc ccc aac gtc act gtg cgt gcc aac att     480
   Leu Val Ser Ile Pro His Gly Pro Asn Val Thr Val Arg Ala Asn Ile
   145                 150                 155                 160
   gct gcc atc act gaa tca gac aag ttc ttc atc aac ggc tcc aac tgg     528
   Ala Ala Ile Thr Glu Ser Asp Lys Phe Phe Ile Asn Gly Ser Asn Trp
                   165                 170                 175
   gaa ggc atc ctg ggg ctg gcc tat gct gag att gcc agg cct gac gac     576
   Glu Gly Ile Leu Gly Leu Ala Tyr Ala Glu Ile Ala Arg Pro Asp Asp
               180                 185                 190
   tcc ctg gag cct ttc ttt gac tct ctg gta aag cag acc cac gtt ccc     624
   Ser Leu Glu Pro Phe Phe Asp Ser Leu Val Lys Gln Thr His Val Pro
   195 200 205
   aac ctc ttc tcc ctg cag ctt tgt ggt gct ggc ttc ccc ctc aac cag 672
   Asn Leu Phe Ser Leu Gln Leu Cys Gly Ala Gly Phe Pro Leu Asn Gln
   210 215 220
   tct gaa gtg ctg gcc tct gtc gga ggg agc atg atc att gga ggt atc 720
   Ser Glu Val Leu Ala Ser Val Gly Gly Ser Met Ile Ile Gly Gly Ile
   225 230 235 240
   gac cac tcg ctg tac aca ggc agt ctc tgg tat aca ccc atc cgg cgg 768
   Asp His Ser Leu Tyr Thr Gly Ser Leu Trp Tyr Thr Pro Ile Arg Arg
   245 250 255
   gag tgg tat tat gag gtg atc att gtg cgg gtg gag atc aat gga cag 816
   Glu Trp Tyr Tyr Glu Val Ile Ile Val Arg Val Glu Ile Asn Gly Gln
   260 265 270
   gat ctg aaa atg gac tgc aag gag tac aac tat gac aag agc att gtg 864
   Asp Leu Lys Met Asp Cys Lys Glu Tyr Asn Tyr Asp Lys Ser Ile Val
   275 280 285
   gac agt ggc acc acc aac ctt cgt ttg ccc aag aaa gtg ttt gaa gct 912
   Asp Ser Gly Thr Thr Asn Leu Arg Leu Pro Lys Lys Val Phe Glu Ala
   290 295 300
   gca gtc aaa tcc atc aag gca gcc tcc tcc acg gag aag ttc cct gat 960
   Ala Val Lys Ser Ile Lys Ala Ala Ser Ser Thr Glu Lys Phe Pro Asp
   305 310 315 320
   ggt ttc tgg cta gga gag cag ctg gtg tgc tgg caa gca ggc acc acc 1008
   Gly Phe Trp Leu Gly Glu Gln Leu Val Cys Trp Gln Ala Gly Thr Thr
   325 330 335
   cct tgg aac att ttc cca gtc atc tca ctc tac cta atg ggt gag gtt 1056
   Pro Trp Asn Ile Phe Pro Val Ile Ser Leu Tyr Leu Met Gly Glu Val
   340 345 350
   acc aac cag tcc ttc cgc atc acc atc ctt ccg cag caa tac ctg cgg 1104
   Thr Asn Gln Ser Phe Arg Ile Thr Ile Leu Pro Gln Gln Tyr Leu Arg
   355 360 365
   cca gtg gaa gat gtg gcc acg tcc caa gac gac tgt tac aag ttt gcc 1152
   Pro Val Glu Asp Val Ala Thr Ser Gln Asp Asp Cys Tyr Lys Phe Ala
   370 375 380
   atc tca cag tca tcc acg ggc act gtt atg gga gct gtt atc atg gag 1200
   Ile Ser Gln Ser Ser Thr Gly Thr Val Met Gly Ala Val Ile Met Glu
   385 390 395 400
   ggc ttc tac gtt gtc ttt gat cgg gcc cga aaa cga att ggc ttt gct 1248
   Gly Phe Tyr Val Val Phe Asp Arg Ala Arg Lys Arg Ile Gly Phe Ala
   405 410 415
   gtc agc gct tgc cat gtg cac gat gag ttc agg acg gca gcg gtg gaa 1296
   Val Ser Ala Cys His Val His Asp Glu Phe Arg Thr Ala Ala Val Glu
   420 425 430
   ggc cct ttt gtc acc ttg gac atg gaa gac tgt ggc tac aac att cca 1344
   Gly Pro Phe Val Thr Leu Asp Met Glu Asp Cys Gly Tyr Asn Ile Pro
   435 440 445
   cag aca gat gag tca acc ctc atg acc ata gcc tat gtc atg gct gcc 1392
   Gln Thr Asp Glu Ser Thr Leu Met Thr Ile Ala Tyr Val Met Ala Ala
   450 455 460
   atc tgc gcc ctc ttc atg ctg cca ctc tgc ctc atg gtg tgt cag tgg 1440
   Ile Cys Ala Leu Phe Met Leu Pro Leu Cys Leu Met Val Cys Gln Trp
   465 470 475 480
   cgc tgc ctc cgc tgc ctg cgc cag cag cat gat gac ttt gct gat gac 1488
   Arg Cys Leu Arg Cys Leu Arg Gln Gln His Asp Asp Phe Ala Asp Asp
   485 490 495
   atc tcc ctg ctg aag 1503
   Ile Ser Leu Leu Lys
   500
<210> 2
   <211> 501
   <212> PRT
   <213> Homo sapiens
<400> 2
   Met Ala Gln Ala Leu Pro Trp Leu Leu Leu Trp Met Gly Ala Gly Val
   1 5 10 15
   Leu Pro Ala His Gly Thr Gln His Gly Ile Arg Leu Pro Leu Arg Ser
   20 25 30
   Gly Leu Gly Gly Ala Pro Leu Gly Leu Arg Leu Pro Arg Glu Thr Asp
   35 40 45
   Glu Glu Pro Glu Glu Pro Gly Arg Arg Gly Ser Phe Val Glu Met Val
   50 55 60
   Asp Asn Leu Arg Gly Lys Ser Gly Gln Gly Tyr Tyr Val Glu Met Thr
   65 70 75 80
   Val Gly Ser Pro Pro Gln Thr Leu Asn Ile Leu Val Asp Thr Gly Ser
   85 90 95
   Ser Asn Phe Ala Val Gly Ala Ala Pro His Pro Phe Leu His Arg Tyr
   100 105 110
   Tyr Gln Arg Gln Leu Ser Ser Thr Tyr Arg Asp Leu Arg Lys Gly Val
   115 120 125
   Tyr Val Pro Tyr Thr Gln Gly Lys Trp Glu Gly Glu Leu Gly Thr Asp
   130 135 140
   Leu Val Ser Ile Pro His Gly Pro Asn Val Thr Val Arg Ala Asn Ile
   145 150 155 160
   Ala Ala Ile Thr Glu Ser Asp Lys Phe Phe Ile Asn Gly Ser Asn Trp
   165 170 175
   Glu Gly Ile Leu Gly Leu Ala Tyr Ala Glu Ile Ala Arg Pro Asp Asp
   180 185 190
   Ser Leu Glu Pro Phe Phe Asp Ser Leu Val Lys Gln Thr His Val Pro
   195 200 205
   Asn Leu Phe Ser Leu Gln Leu Cys Gly Ala Gly Phe Pro Leu Asn Gln
   210 215 220
   Ser Glu Val Leu Ala Ser Val Gly Gly Ser Met Ile Ile Gly Gly Ile
   225 230 235 240
   Asp His Ser Leu Tyr Thr Gly Ser Leu Trp Tyr Thr Pro Ile Arg Arg
   245 250 255
   Glu Trp Tyr Tyr Glu Val Ile Ile Val Arg Val Glu Ile Asn Gly Gln
   260 265 270
   Asp Leu Lys Met Asp Cys Lys Glu Tyr Asn Tyr Asp Lys Ser Ile Val
   275 280 285
   Asp Ser Gly Thr Thr Asn Leu Arg Leu Pro Lys Lys Val Phe Glu Ala
   290 295 300
   Ala Val Lys Ser Ile Lys Ala Ala Ser Ser Thr Glu Lys Phe Pro Asp
   305 310 315 320
   Gly Phe Trp Leu Gly Glu Gln Leu Val Cys Trp Gln Ala Gly Thr Thr
   325 330 335
   Pro Trp Asn Ile Phe Pro Val Ile Ser Leu Tyr Leu Met Gly Glu Val
   340 345 350
   Thr Asn Gln Ser Phe Arg Ile Thr Ile Leu Pro Gln Gln Tyr Leu Arg
   355 360 365
   Pro Val Glu Asp Val Ala Thr Ser Gln Asp Asp Cys Tyr Lys Phe Ala
   370 375 380
   Ile Ser Gln Ser Ser Thr Gly Thr Val Met Gly Ala Val Ile Met Glu
   385 390 395 400
   Gly Phe Tyr Val Val Phe Asp Arg Ala Arg Lys Arg Ile Gly Phe Ala
   405 410 415
   Val Ser Ala Cys His Val His Asp Glu Phe Arg Thr Ala Ala Val Glu
   420 425 430
   Gly Pro Phe Val Thr Leu Asp Met Glu Asp Cys Gly Tyr Asn Ile Pro
   435 440 445
   Gln Thr Asp Glu Ser Thr Leu Met Thr Ile Ala Tyr Val Met Ala Ala
   450 455 460
   Ile Cys Ala Leu Phe Met Leu Pro Leu Cys Leu Met Val Cys Gln Trp
   465 470 475 480
   Arg Cys Leu Arg Cys Leu Arg Gln Gln His Asp Asp Phe Ala Asp Asp
   485 490 495
   Ile Ser Leu Leu Lys
   500
<210> 3
   <211> 24
   <212> DNA
   <213> Artificial
<220>
   <223> Oligonucleotide encoding Flag peptide.
<220>
   <221> CDS
   <222> (1).. (24)
<400> 3
   gat tac aag gat gac gac gat aag 24
   Asp Tyr Lys Asp Asp Asp Asp Lys
   1 5
<210> 4
   <211> 8
   <212> PRT
   <213> Artificial
<220>
   <223> Synthetic Construct
<400> 4
   Asp Tyr Lys Asp Asp Asp Asp Lys
   1 5
<210> 5
   <211> 18
   <212> DNA
   <213> Artificial
<220>
   <223> Primer.
<400> 5
   ggcaccacca accttcgt 18
<210> 6
   <211> 55
   <212> DNA
   <213> Artificial
<220>
   <223> Primer.
<400> 6
   ggtacctact tatcgtcgtc atccttgtaa tccttcagca gggagatgtc atcag 55
<210> 7
   <211> 20
   <212> DNA
   <213> Artificial
<220>
   <223> Primer.
<400> 7
   taatacgact cactataggg 20
<210> 8
   <211> 24
   <212> DNA
   <213> Artificial
<220>
   <223> Primer.
<400> 8
   ggcgcccccc agaccacttc tcag 24
<210> 9
   <211> 1527
   <212> DNA
   <213> Recombinant DNA
<220>
   <221> CDS
   <222> (1).. (1527)
<220>
   <221> misc_feature
   <222> (1504).. (1527)
   <223> Flag-tag.
<400> 9
   atg gcc caa gcc ctg ccc tgg ctc ctg ctg tgg atg ggc gcg gga gtg        48
   Met Ala Gln Ala Leu Pro Trp Leu Leu Leu Trp Met Gly Ala Gly Val
   1               5                   10                  15
   ctg cct gcc cac ggc acc cag cac ggc atc cgg ctg ccc ctg aga agt        96
   Leu Pro Ala His Gly Thr Gln His Gly Ile Arg Leu Pro Leu Arg Ser
               20                  25                  30
   ggt ctg ggg ggc gcc ccc ctg ggg ctg cgg ctg ccc cgg gag acc gac       144
   Gly Leu Gly Gly Ala Pro Leu Gly Leu Arg Leu Pro Arg Glu Thr Asp
           35                  40                  45
   gaa gag ccc gag gag ccc ggc cgg agg ggc agc ttt gtg gag atg gtg       192
   Glu Glu Pro Glu Glu Pro Gly Arg Arg Gly Ser Phe Val Glu Met Val
       50                  55                  60
   gac aac ctg agg ggc aag tcg ggg cag ggc tac tac gtg gag atg acc       240
   Asp Asn Leu Arg Gly Lys Ser Gly Gln Gly Tyr Tyr Val Glu Met Thr
   65                  70                  75                  80
   gtg ggc agc ccc ccg cag acg ctc aac atc ctg gtg gat aca ggc agc       288
   Val Gly Ser Pro Pro Gln Thr Leu Asn Ile Leu Val Asp Thr Gly Ser
                   85                  90                  95
   agt aac ttt gca gtg ggt gct gcc ccc cac ccc ttc ctg cat cgc tac       336
   Ser Asn Phe Ala Val Gly Ala Ala Pro His Pro Phe Leu His Arg Tyr
               100                 105                 110
   tac cag agg cag ctg tcc agc aca tac cgg gac ctc cgg aag ggt gtg       384
   Tyr Gln Arg Gln Leu Ser Ser Thr Tyr Arg Asp Leu Arg Lys Gly Val
           115                 120                 125
   tat gtg ccc tac acc cag ggc aag tgg gaa ggg gag ctg ggc acc gac       432
   Tyr Val Pro Tyr Thr Gln Gly Lys Trp Glu Gly Glu Leu Gly Thr Asp
       130                 135                 140
   ctg gta agc atc ccc cat ggc ccc aac gtc act gtg cgt gcc aac att       480
   Leu Val Ser Ile Pro His Gly Pro Asn Val Thr Val Arg Ala Asn Ile
   145                 150                 155                 160
   gct gcc atc act gaa tca gac aag ttc ttc atc aac ggc tcc aac tgg       528
   Ala Ala Ile Thr Glu Ser Asp Lys Phe Phe Ile Asn Gly Ser Asn Trp
                   165                 170                 175
   gaa ggc atc ctg ggg ctg gcc tat gct gag att gcc agg cct gac gac       576
   Glu Gly Ile Leu Gly Leu Ala Tyr Ala Glu Ile Ala Arg Pro Asp Asp
               180                 185                 190
   tcc ctg gag cct ttc ttt gac tct ctg gta aag cag acc cac gtt ccc       624
   Ser Leu Glu Pro Phe Phe Asp Ser Leu Val Lys Gln Thr His Val Pro
           195                 200                 205
   aac ctc ttc tcc ctg cag ctt tgt ggt gct ggc ttc ccc ctc aac cag       672
   Asn Leu Phe Ser Leu Gln Leu Cys Gly Ala Gly Phe Pro Leu Asn Gln
       210                 215                 220
   tct gaa gtg ctg gcc tct gtc gga ggg agc atg atc att gga ggt atc       720
   Ser Glu Val Leu Ala Ser Val Gly Gly Ser Met Ile Ile Gly Gly Ile
   225                 230                 235                 240
   gac cac tcg ctg tac aca ggc agt ctc tgg tat aca ccc atc cgg cgg       768
   Asp His Ser Leu Tyr Thr Gly Ser Leu Trp Tyr Thr Pro Ile Arg Arg
                   245                 250                 255
   gag tgg tat tat gag gtg atc att gtg cgg gtg gag atc aat gga cag       816
   Glu Trp Tyr Tyr Glu Val Ile Ile Val Arg Val Glu Ile Asn Gly Gln
               260                 265                 270
   gat ctg aaa atg gac tgc aag gag tac aac tat gac aag agc att gtg       864
   Asp Leu Lys Met Asp Cys Lys Glu Tyr Asn Tyr Asp Lys Ser Ile Val
           275                 280                 285
   gac agt ggc acc acc aac ctt cgt ttg ccc aag aaa gtg ttt gaa gct      912
   Asp Ser Gly Thr Thr Asn Leu Arg Leu Pro Lys Lys Val Phe Glu Ala
       290                 295                 300
   gca gtc aaa tcc atc aag gca gcc tcc tcc acg gag aag ttc cct gat      960
   Ala Val Lys Ser Ile Lys Ala Ala Ser Ser Thr Glu Lys Phe Pro Asp
   305                 310                 315                 320
   ggt ttc tgg cta gga gag cag ctg gtg tgc tgg caa gca ggc acc acc     1008
   Gly Phe Trp Leu Gly Glu Gln Leu Val Cys Trp Gln Ala Gly Thr Thr
                   325                 330                 335
   cct tgg aac att ttc cca gtc atc tca ctc tac cta atg ggt gag gtt     1056
   Pro Trp Asn Ile Phe Pro Val Ile Ser Leu Tyr Leu Met Gly Glu Val
               340                 345                 350
   acc aac cag tcc ttc cgc atc acc atc ctt ccg cag caa tac ctg cgg     1104
   Thr Asn Gln Ser Phe Arg Ile Thr Ile Leu Pro Gln Gln Tyr Leu Arg
           355                 360                 365
   cca gtg gaa gat gtg gcc acg tcc caa gac gac tgt tac aag ttt gcc     1152
   Pro Val Glu Asp Val Ala Thr Ser Gln Asp Asp Cys Tyr Lys Phe Ala
       370                 375                 380
   atc tca cag tca tcc acg ggc act gtt atg gga gct gtt atc atg gag     1200
   Ile Ser Gln Ser Ser Thr Gly Thr Val Met Gly Ala Val Ile Met Glu
   385                 390                 395                 400
   ggc ttc tac gtt gtc ttt gat cgg gcc cga aaa cga att ggc ttt gct     1248
   Gly Phe Tyr Val Val Phe Asp Arg Ala Arg Lys Arg Ile Gly Phe Ala
                   405                 410                 415
   gtc agc gct tgc cat gtg cac gat gag ttc agg acg gca gcg gtg gaa     1296
   Val Ser Ala Cys His Val His Asp Glu Phe Arg Thr Ala Ala Val Glu
               420                 425                 430
   ggc cct ttt gtc acc ttg gac atg gaa gac tgt ggc tac aac att cca     1344
   Gly Pro Phe Val Thr Leu Asp Met Glu Asp Cys Gly Tyr Asn Ile Pro
           435                 440                 445
   cag aca gat gag tca acc ctc atg acc ata gcc tat gtc atg gct gcc     1392
   Gln Thr Asp Glu Ser Thr Leu Met Thr Ile Ala Tyr Val Met Ala Ala
       450                 455                 460
   atc tgc gcc ctc ttc atg ctg cca ctc tgc ctc atg gtg tgt cag tgg     1440
   Ile Cys Ala Leu Phe Met Leu Pro Leu Cys Leu Met Val Cys Gln Trp
   465                 470                 475                 480
   cgc tgc ctc cgc tgc ctg cgc cag cag cat gat gac ttt gct gat gac     1488
   Arg Cys Leu Arg Cys Leu Arg Gln Gln His Asp Asp Phe Ala Asp Asp
                   485                 490                 495
   atc tcc ctg ctg aag gat tac aag gat gac gac gat aag                 1527
   Ile Ser Leu Leu Lys Asp Tyr Lys Asp Asp Asp Asp Lys
               500                 505
<210> 10
   <211> 509
   <212> PRT
   <213> Recombinant DNA
<400> 10
   Met Ala Gln Ala Leu Pro Trp Leu Leu Leu Trp Met Gly Ala Gly Val
   1               5                   10                  15
   Leu Pro Ala His Gly Thr Gln His Gly Ile Arg Leu Pro Leu Arg Ser
               20                  25                  30
   Gly Leu Gly Gly Ala Pro Leu Gly Leu Arg Leu Pro Arg Glu Thr Asp
   35 40 45
   Glu Glu Pro Glu Glu Pro Gly Arg Arg Gly Ser Phe Val Glu Met Val
   50 55 60
   Asp Asn Leu Arg Gly Lys Ser Gly Gln Gly Tyr Tyr Val Glu Met Thr
   65 70 75 80
   Val Gly Ser Pro Pro Gln Thr Leu Asn Ile Leu Val Asp Thr Gly Ser
   85 90 95
   Ser Asn Phe Ala Val Gly Ala Ala Pro His Pro Phe Leu His Arg Tyr
   100 105 110
   Tyr Gln Arg Gln Leu Ser Ser Thr Tyr Arg Asp Leu Arg Lys Gly Val
   115 120 125
   Tyr Val Pro Tyr Thr Gln Gly Lys Trp Glu Gly Glu Leu Gly Thr Asp
   130 135 140
   Leu Val Ser Ile Pro His Gly Pro Asn Val Thr Val Arg Ala Asn Ile
   145 150 155 160
   Ala Ala Ile Thr Glu Ser Asp Lys Phe Phe Ile Asn Gly Ser Asn Trp
   165 170 175
   Glu Gly Ile Leu Gly Leu Ala Tyr Ala Glu Ile Ala Arg Pro Asp Asp
   180 185 190
   Ser Leu Glu Pro Phe Phe Asp Ser Leu Val Lys Gln Thr His Val Pro
   195 200 205
   Asn Leu Phe Ser Leu Gln Leu Cys Gly Ala Gly Phe Pro Leu Asn Gln
   210 215 220
   Ser Glu Val Leu Ala Ser Val Gly Gly Ser Met Ile Ile Gly Gly Ile
   225 230 235 240
   Asp His Ser Leu Tyr Thr Gly Ser Leu Trp Tyr Thr Pro Ile Arg Arg
   245 250 255
   Glu Trp Tyr Tyr Glu Val Ile Ile Val Arg Val Glu Ile Asn Gly Gln
   260 265 270
   Asp Leu Lys Met Asp Cys Lys Glu Tyr Asn Tyr Asp Lys Ser Ile Val
   275 280 285
   Asp Ser Gly Thr Thr Asn Leu Arg Leu Pro Lys Lys Val Phe Glu Ala
   290 295 300
   Ala Val Lys Ser Ile Lys Ala Ala Ser Ser Thr Glu Lys Phe Pro Asp
   305 310 315 320
   Gly Phe Trp Leu Gly Glu Gln Leu Val Cys Trp Gln Ala Gly Thr Thr
   325 330 335
   Pro Trp Asn Ile Phe Pro Val Ile Ser Leu Tyr Leu Met Gly Glu Val
   340 345 350
   Thr Asn Gln Ser Phe Arg Ile Thr Ile Leu Pro Gln Gln Tyr Leu Arg
   355 360 365
   Pro Val Glu Asp Val Ala Thr Ser Gln Asp Asp Cys Tyr Lys Phe Ala
   370 375 380
   Ile Ser Gln Ser Ser Thr Gly Thr Val Met Gly Ala Val Ile Met Glu
   385 390 395 400
   Gly Phe Tyr Val Val Phe Asp Arg Ala Arg Lys Arg Ile Gly Phe Ala
   405 410 415
   Val Ser Ala Cys His Val His Asp Glu Phe Arg Thr Ala Ala Val Glu
   420 425 430
   Gly Pro Phe Val Thr Leu Asp Met Glu Asp Cys Gly Tyr Asn Ile Pro
   435 440 445
   Gln Thr Asp Glu Ser Thr Leu Met Thr Ile Ala Tyr Val Met Ala Ala
   450 455 460
   Ile Cys Ala Leu Phe Met Leu Pro Leu Cys Leu Met Val Cys Gln Trp
   465 470 475 480
   Arg Cys Leu Arg Cys Leu Arg Gln Gln His Asp Asp Phe Ala Asp Asp
   485 490 495
   Ile Ser Leu Leu Lys Asp Tyr Lys Asp Asp Asp Asp Lys
   500 505
<210> 11
   <211> 11
   <212> PRT
   <213> Artificial
<220>
   <223> Synthetic substrate for beta-secretase.
<220>
   <221> MOD_RES
   <222> (1).. (1)
   <223> N-methylanthranoyl-L-serine.
<220>
   <221> MOD_RES
   <222> (9).. (9)
   <223> 2.4-dinitrophenyl-L-lisine.
<220>
   <221> MOD_RES
   <222> (11).. (11)
   <223> AMIDATION
<400> 11
   Ser Glu Val Lys Met Asp Ala Glu Lys Arg Arg
   1 5 10
<210> 12
   <211> 30
   <212> DNA
   <213> Artificial
<220>
   <223> Primer.
<400> 12
   gctggctagc gtttaaacgg gccctctaga 30
<210> 13
   <211> 52
   <212> DNA
   <213> Artificial
<220>
   <223> Primer.
<400> 13
   ttttggtacc tacttatcgt cgtcatcctt gtaatcggtt gactcatctg tc 52
<210> 14
   <211> 1386
   <212> DNA
   <213> Recombinant DNA
<220>
   <221> CDS
   <222> (1).. (1386)
<220>
   <221> misc_feature
   <222> (1363).. (1386)
   <223> Flag-tag.
<400> 14
   atg gcc caa gcc ctg ccc tgg ctc ctg ctg tgg atg ggc gcg gga gtg       48
   Met Ala Gln Ala Leu Pro Trp Leu Leu Leu Trp Met Gly Ala Gly Val
   1               5                   10                  15
   ctg cct gcc cac ggc acc cag cac ggc atc cgg ctg ccc ctg aga agt       96
   Leu Pro Ala His Gly Thr Gln His Gly Ile Arg Leu Pro Leu Arg Ser
               20                  25                  30
   ggt ctg ggg ggc gcc ccc ctg ggg ctg cgg ctg ccc cgg gag acc gac      144
   Gly Leu Gly Gly Ala Pro Leu Gly Leu Arg Leu Pro Arg Glu Thr Asp
           35                  40                  45
   gaa gag ccc gag gag ccc ggc cgg agg ggc agc ttt gtg gag atg gtg      192
   Glu Glu Pro Glu Glu Pro Gly Arg Arg Gly Ser Phe Val Glu Met Val
       50                  55                  60
   gac aac ctg agg ggc aag tcg ggg cag ggc tac tac gtg gag atg acc      240
   Asp Asn Leu Arg Gly Lys Ser Gly Gln Gly Tyr Tyr Val Glu Met Thr
   65                  70                  75                  80
   gtg ggc agc ccc ccg cag acg ctc aac atc ctg gtg gat aca ggc agc      288
   Val Gly Ser Pro Pro Gln Thr Leu Asn Ile Leu Val Asp Thr Gly Ser
                   85                  90                  95
   agt aac ttt gca gtg ggt gct gcc ccc cac ccc ttc ctg cat cgc tac      336
   Ser Asn Phe Ala Val Gly Ala Ala Pro His Pro Phe Leu His Arg Tyr
               100                 105                 110
   tac cag agg cag ctg tcc agc aca tac cgg gac ctc cgg aag ggt gtg      384
   Tyr Gln Arg Gln Leu Ser Ser Thr Tyr Arg Asp Leu Arg Lys Gly Val
           115                 120                 125
   tat gtg ccc tac acc cag ggc aag tgg gaa ggg gag ctg ggc acc gac      432
   Tyr Val Pro Tyr Thr Gln Gly Lys Trp Glu Gly Glu Leu Gly Thr Asp
       130                 135                 140
   ctg gta agc atc ccc cat ggc ccc aac gtc act gtg cgt gcc aac att      480
   Leu Val Ser Ile Pro His Gly Pro Asn Val Thr Val Arg Ala Asn Ile
   145                 150                 155                 160
   gct gcc atc act gaa tca gac aag ttc ttc atc aac ggc tcc aac tgg      528
   Ala Ala Ile Thr Glu Ser Asp Lys Phe Phe Ile Asn Gly Ser Asn Trp
                   165                 170                 175
   gaa ggc atc ctg ggg ctg gcc tat gct gag att gcc agg cct gac gac      576
   Glu Gly Ile Leu Gly Leu Ala Tyr Ala Glu Ile Ala Arg Pro Asp Asp
               180                 185                 190
   tcc ctg gag cct ttc ttt gac tct ctg gta aag cag acc cac gtt ccc      624
   Ser Leu Glu Pro Phe Phe Asp Ser Leu Val Lys Gln Thr His Val Pro
           195                 200                 205
   aac ctc ttc tcc ctg cag ctt tgt ggt gct ggc ttc ccc ctc aac cag      672
   Asn Leu Phe Ser Leu Gln Leu Cys Gly Ala Gly Phe Pro Leu Asn Gln
       210                 215                 220
   tct gaa gtg ctg gcc tct gtc gga ggg agc atg atc att gga ggt atc      720
   Ser Glu Val Leu Ala Ser Val Gly Gly Ser Met Ile Ile Gly Gly Ile
   225                 230                 235                 240
   gac cac tcg ctg tac aca ggc agt ctc tgg tat aca ccc atc cgg cgg      768
   Asp His Ser Leu Tyr Thr Gly Ser Leu Trp Tyr Thr Pro Ile Arg Arg
                   245                 250                 255
   gag tgg tat tat gag gtg atc att gtg cgg gtg gag atc aat gga cag      816
   Glu Trp Tyr Tyr Glu Val Ile Ile Val Arg Val Glu Ile Asn Gly Gln
               260                 265                 270
   gat ctg aaa atg gac tgc aag gag tac aac tat gac aag agc att gtg      864
   Asp Leu Lys Met Asp Cys Lys Glu Tyr Asn Tyr Asp Lys Ser Ile Val
           275                 280                 285
   gac agt ggc acc acc aac ctt cgt ttg ccc aag aaa gtg ttt gaa gct      912
   Asp Ser Gly Thr Thr Asn Leu Arg Leu Pro Lys Lys Val Phe Glu Ala
       290                 295                 300
   gca gtc aaa tcc atc aag gca gcc tcc tcc acg gag aag ttc cct gat      960
   Ala Val Lys Ser Ile Lys Ala Ala Ser Ser Thr Glu Lys Phe Pro Asp
   305                 310                 315                 320
   ggt ttc tgg cta gga gag cag ctg gtg tgc tgg caa gca ggc acc acc     1008
   Gly Phe Trp Leu Gly Glu Gln Leu Val Cys Trp Gln Ala Gly Thr Thr
                   325                 330                 335
   cct tgg aac att ttc cca gtc atc tca ctc tac cta atg ggt gag gtt     1056
   Pro Trp Asn Ile Phe Pro Val Ile Ser Leu Tyr Leu Met Gly Glu Val
               340                 345                 350
   acc aac cag tcc ttc cgc atc acc atc ctt ccg cag caa tac ctg cgg     1104
   Thr Asn Gln Ser Phe Arg Ile Thr Ile Leu Pro Gln Gln Tyr Leu Arg
           355                 360                 365
   cca gtg gaa gat gtg gcc acg tcc caa gac gac tgt tac aag ttt gcc     1152
   Pro Val Glu Asp Val Ala Thr Ser Gln Asp Asp Cys Tyr Lys Phe Ala
       370                 375                 380
   atc tca cag tca tcc acg ggc act gtt atg gga gct gtt atc atg gag     1200
   Ile Ser Gln Ser Ser Thr Gly Thr Val Met Gly Ala Val Ile Met Glu
   385                 390                 395                 400
   ggc ttc tac gtt gtc ttt gat cgg gcc cga aaa cga att ggc ttt gct     1248
   Gly Phe Tyr Val Val Phe Asp Arg Ala Arg Lys Arg Ile Gly Phe Ala
                   405                 410                 415
   gtc agc gct tgc cat gtg cac gat gag ttc agg acg gca gcg gtg gaa     1296
   Val Ser Ala Cys His Val His Asp Glu Phe Arg Thr Ala Ala Val Glu
               420                 425                 430
   ggc cct ttt gtc acc ttg gac atg gaa gac tgt ggc tac aac att cca     1344
   Gly Pro Phe Val Thr Leu Asp Met Glu Asp Cys Gly Tyr Asn Ile Pro
           435                 440                 445
   cag aca gat gag tca acc gat tac aag gat gac gac gat aag             1386
   Gln Thr Asp Glu Ser Thr Asp Tyr Lys Asp Asp Asp Asp Lys
       450                 455                 460
<210> 15
   <211> 462
   <212> PRT
   <213> Recombinant DNA
<400> 15
   Met Ala Gln Ala Leu Pro Trp Leu Leu Leu Trp Met Gly Ala Gly Val
   1 5 10 15
   Leu Pro Ala His Gly Thr Gln His Gly Ile Arg Leu Pro Leu Arg Ser
   20 25 30
   Gly Leu Gly Gly Ala Pro Leu Gly Leu Arg Leu Pro Arg Glu Thr Asp
   35 40 45
   Glu Glu Pro Glu Glu Pro Gly Arg Arg Gly Ser Phe Val Glu Met Val
   50 55 60
   Asp Asn Leu Arg Gly Lys Ser Gly Gln Gly Tyr Tyr Val Glu Met Thr
   65 70 75 80
   Val Gly Ser Pro Pro Gln Thr Leu Asn Ile Leu Val Asp Thr Gly Ser
   85 90 95
   Ser Asn Phe Ala Val Gly Ala Ala Pro His Pro Phe Leu His Arg Tyr
   100 105 110
   Tyr Gln Arg Gln Leu Ser Ser Thr Tyr Arg Asp Leu Arg Lys Gly Val
   115 120 125
   Tyr Val Pro Tyr Thr Gln Gly Lys Trp Glu Gly Glu Leu Gly Thr Asp
   130 135 140
   Leu Val Ser Ile Pro His Gly Pro Asn Val Thr Val Arg Ala Asn Ile
   145 150 155 160
   Ala Ala Ile Thr Glu Ser Asp Lys Phe Phe Ile Asn Gly Ser Asn Trp
   165 170 175
   Glu Gly Ile Leu Gly Leu Ala Tyr Ala Glu Ile Ala Arg Pro Asp Asp
   180 185 190
   Ser Leu Glu Pro Phe Phe Asp Ser Leu Val Lys Gln Thr His Val Pro
   195 200 205
   Asn Leu Phe Ser Leu Gln Leu Cys Gly Ala Gly Phe Pro Leu Asn Gln
   210 215 220
   Ser Glu Val Leu Ala Ser Val Gly Gly Ser Met Ile Ile Gly Gly Ile
   225 230 235 240
   Asp His Ser Leu Tyr Thr Gly Ser Leu Trp Tyr Thr Pro Ile Arg Arg
   245 250 255
   Glu Trp Tyr Tyr Glu Val Ile Ile Val Arg Val Glu Ile Asn Gly Gln
   260 265 270
   Asp Leu Lys Met Asp Cys Lys Glu Tyr Asn Tyr Asp Lys Ser Ile Val
   275 280 285
   Asp Ser Gly Thr Thr Asn Leu Arg Leu Pro Lys Lys Val Phe Glu Ala
   290 295 300
   Ala Val Lys Ser Ile Lys Ala Ala Ser Ser Thr Glu Lys Phe Pro Asp
   305 310 315 320
   Gly Phe Trp Leu Gly Glu Gln Leu Val Cys Trp Gln Ala Gly Thr Thr
   325 330 335
   Pro Trp Asn Ile Phe Pro Val Ile Ser Leu Tyr Leu Met Gly Glu Val
   340 345 350
   Thr Asn Gln Ser Phe Arg Ile Thr Ile Leu Pro Gln Gln Tyr Leu Arg
   355 360 365
   Pro Val Glu Asp Val Ala Thr Ser Gln Asp Asp Cys Tyr Lys Phe Ala
   370 375 380
   Ile Ser Gln Ser Ser Thr Gly Thr Val Met Gly Ala Val Ile Met Glu
   385 390 395 400
   Gly Phe Tyr Val Val Phe Asp Arg Ala Arg Lys Arg Ile Gly Phe Ala
   405 410 415
   Val Ser Ala Cys His Val His Asp Glu Phe Arg Thr Ala Ala Val Glu
   420 425 430
   Gly Pro Phe Val Thr Leu Asp Met Glu Asp Cys Gly Tyr Asn Ile Pro
   435 440 445
   Gln Thr Asp Glu Ser Thr Asp Tyr Lys Asp Asp Asp Asp Lys
   450 455 460
<210> 16
   <211> 52
   <212> DNA
   <213> Artificial
<220>
   <223> Primer.
<400> 16 52
   ttttggtacc tacttatcgt cgtcatcctt gtaatcgcag agtggcagca tg 52
<210> 17
   <211> 1446
   <212> DNA
   <213> Recombinant DNA
<220>
   <221> CDS
   <222> (1).. (1446)
<220>
   <221> misc_feature
   <222> (1423).. (1446)
   <223> Flag-tag.
<400> 17
   atg gcc caa gcc ctg ccc tgg ctc ctg ctg tgg atg ggc gcg gga gtg      48
   Met Ala Gln Ala Leu Pro Trp Leu Leu Leu Trp Met Gly Ala Gly Val
   1               5                   10                  15
   ctg cct gcc cac ggc acc cag cac ggc atc cgg ctg ccc ctg aga agt      96
   Leu Pro Ala His Gly Thr Gln His Gly Ile Arg Leu Pro Leu Arg Ser
               20                  25                  30
   ggt ctg ggg ggc gcc ccc ctg ggg ctg cgg ctg ccc cgg gag acc gac     144
   Gly Leu Gly Gly Ala Pro Leu Gly Leu Arg Leu Pro Arg Glu Thr Asp
           35                  40                  45
   gaa gag ccc gag gag ccc ggc cgg agg ggc agc ttt gtg gag atg gtg     192
   Glu Glu Pro Glu Glu Pro Gly Arg Arg Gly Ser Phe Val Glu Met Val
       50                  55                  60
   gac aac ctg agg ggc aag tcg ggg cag ggc tac tac gtg gag atg acc     240
   Asp Asn Leu Arg Gly Lys Ser Gly Gln Gly Tyr Tyr Val Glu Met Thr
   65                  70                  75                  80
   gtg ggc agc ccc ccg cag acg ctc aac atc ctg gtg gat aca ggc agc     288
   Val Gly Ser Pro Pro Gln Thr Leu Asn Ile Leu Val Asp Thr Gly Ser
                   85                  90                  95
   agt aac ttt gca gtg ggt gct gcc ccc cac ccc ttc ctg cat cgc tac     336
   Ser Asn Phe Ala Val Gly Ala Ala Pro His Pro Phe Leu His Arg Tyr
               100                 105                 110
   tac cag agg cag ctg tcc agc aca tac cgg gac ctc cgg aag ggt gtg     384
   Tyr Gln Arg Gln Leu Ser Ser Thr Tyr Arg Asp Leu Arg Lys Gly Val
           115                 120                 125
   tat gtg ccc tac acc cag ggc aag tgg gaa ggg gag ctg ggc acc gac     432
   Tyr Val Pro Tyr Thr Gln Gly Lys Trp Glu Gly Glu Leu Gly Thr Asp
       130                 135                 140
   ctg gta agc atc ccc cat ggc ccc aac gtc act gtg cgt gcc aac att     480
   Leu Val Ser Ile Pro His Gly Pro Asn Val Thr Val Arg Ala Asn Ile
   145                 150                 155                 160
   gct gcc atc act gaa tca gac aag ttc ttc atc aac ggc tcc aac tgg     528
   Ala Ala Ile Thr Glu Ser Asp Lys Phe Phe Ile Asn Gly Ser Asn Trp
                   165                 170                 175
   gaa ggc atc ctg ggg ctg gcc tat gct gag att gcc agg cct gac gac     576
   Glu Gly Ile Leu Gly Leu Ala Tyr Ala Glu Ile Ala Arg Pro Asp Asp
               180                 185                 190
   tcc ctg gag cct ttc ttt gac tct ctg gta aag cag acc cac gtt ccc     624
   Ser Leu Glu Pro Phe Phe Asp Ser Leu Val Lys Gln Thr His Val Pro
           195                 200                 205
   aac ctc ttc tcc ctg cag ctt tgt ggt gct ggc ttc ccc ctc aac cag     672
   Asn Leu Phe Ser Leu Gln Leu Cys Gly Ala Gly Phe Pro Leu Asn Gln
       210                 215                 220
   tct gaa gtg ctg gcc tct gtc gga ggg agc atg atc att gga ggt atc     720
   Ser Glu Val Leu Ala Ser Val Gly Gly Ser Met Ile Ile Gly Gly Ile
   225                 230                 235                 240
   gac cac tcg ctg tac aca ggc agt ctc tgg tat aca ccc atc cgg cgg     768
   Asp His Ser Leu Tyr Thr Gly Ser Leu Trp Tyr Thr Pro Ile Arg Arg
                   245                 250                 255
   gag tgg tat tat gag gtg atc att gtg cgg gtg gag atc aat gga cag     816
   Glu Trp Tyr Tyr Glu Val Ile Ile Val Arg Val Glu Ile Asn Gly Gln
               260                 265                 270
   gat ctg aaa atg gac tgc aag gag tac aac tat gac aag agc att gtg     864
   Asp Leu Lys Met Asp Cys Lys Glu Tyr Asn Tyr Asp Lys Ser Ile Val
           275                 280                 285
   gac agt ggc acc acc aac ctt cgt ttg ccc aag aaa gtg ttt gaa gct     912
   Asp Ser Gly Thr Thr Asn Leu Arg Leu Pro Lys Lys Val Phe Glu Ala
       290                 295                 300
   gca gtc aaa tcc atc aag gca gcc tcc tcc acg gag aag ttc cct gat        960
   Ala Val Lys Ser Ile Lys Ala Ala Ser Ser Thr Glu Lys Phe Pro Asp
   305                 310                 315                 320
   ggt ttc tgg cta gga gag cag ctg gtg tgc tgg caa gca ggc acc acc       1008
   Gly Phe Trp Leu Gly Glu Gln Leu Val Cys Trp Gln Ala Gly Thr Thr
                   325                 330                 335
   cct tgg aac att ttc cca gtc atc tca ctc tac cta atg ggt gag gtt       1056
   Pro Trp Asn Ile Phe Pro Val Ile Ser Leu Tyr Leu Met Gly Glu Val
               340                 345                 350
   acc aac cag tcc ttc cgc atc acc atc ctt ccg cag caa tac ctg cgg       1104
   Thr Asn Gln Ser Phe Arg Ile Thr Ile Leu Pro Gln Gln Tyr Leu Arg
           355                 360                 365
   cca gtg gaa gat gtg gcc acg tcc caa gac gac tgt tac aag ttt gcc       1152
   Pro Val Glu Asp Val Ala Thr Ser Gln Asp Asp Cys Tyr Lys Phe Ala
       370                 375                 380
   atc tca cag tca tcc acg ggc act gtt atg gga gct gtt atc atg gag       1200
   Ile Ser Gln Ser Ser Thr Gly Thr Val Met Gly Ala Val Ile Met Glu
   385                 390                 395                 400
   ggc ttc tac gtt gtc ttt gat cgg gcc cga aaa cga att ggc ttt gct       1248
   Gly Phe Tyr Val Val Phe Asp Arg Ala Arg Lys Arg Ile Gly Phe Ala
                   405                 410                 415
   gtc agc gct tgc cat gtg cac gat gag ttc agg acg gca gcg gtg gaa       1296
   Val Ser Ala Cys His Val His Asp Glu Phe Arg Thr Ala Ala Val Glu
               420                 425                 430
   ggc cct ttt gtc acc ttg gac atg gaa gac tgt ggc tac aac att cca       1344
   Gly Pro Phe Val Thr Leu Asp Met Glu Asp Cys Gly Tyr Asn Ile Pro
           435                 440                 445
   cag aca gat gag tca acc ctc atg acc ata gcc tat gtc atg gct gcc       1392
   Gln Thr Asp Glu Ser Thr Leu Met Thr Ile Ala Tyr Val Met Ala Ala
       450                 455                 460
   atc tgc gcc ctc ttc atg ctg cca ctc tgc gat tac aag gat gac gac       1440
   Ile Cys Ala Leu Phe Met Leu Pro Leu Cys Asp Tyr Lys Asp Asp Asp
   465                 470                 475                 480
   gat aag                                                               1446
   Asp Lys
<210> 18
   <211> 482
   <212> PRT
   <213> Recombinant DNA
<400> 18
   Met Ala Gln Ala Leu Pro Trp Leu Leu Leu Trp Met Gly Ala Gly Val
   1 5 10 15
   Leu Pro Ala His Gly Thr Gln His Gly Ile Arg Leu Pro Leu Arg Ser
   20 25 30
   Gly Leu Gly Gly Ala Pro Leu Gly Leu Arg Leu Pro Arg Glu Thr Asp
   35 40 45
   Glu Glu Pro Glu Glu Pro Gly Arg Arg Gly Ser Phe Val Glu Met Val
   50 55 60
   Asp Asn Leu Arg Gly Lys Ser Gly Gln Gly Tyr Tyr Val Glu Met Thr
   65 70 75 80
   Val Gly Ser Pro Pro Gln Thr Leu Asn Ile Leu Val Asp Thr Gly Ser
   85 90 95
   Ser Asn Phe Ala Val Gly Ala Ala Pro His Pro Phe Leu His Arg Tyr
   100 105 110
   Tyr Gln Arg Gln Leu Ser Ser Thr Tyr Arg Asp Leu Arg Lys Gly Val
   115 120 125
   Tyr Val Pro Tyr Thr Gln Gly Lys Trp Glu Gly Glu Leu Gly Thr Asp
   130 135 140
   Leu Val Ser Ile Pro His Gly Pro Asn Val Thr Val Arg Ala Asn Ile
   145 150 155 160
   Ala Ala Ile Thr Glu Ser Asp Lys Phe Phe Ile Asn Gly Ser Asn Trp
   165 170 175
   Glu Gly Ile Leu Gly Leu Ala Tyr Ala Glu Ile Ala Arg Pro Asp Asp
   180 185 190
   Ser Leu Glu Pro Phe Phe Asp Ser Leu Val Lys Gln Thr His Val Pro
   195 200 205
   Asn Leu Phe Ser Leu Gln Leu Cys Gly Ala Gly Phe Pro Leu Asn Gln
   210 215 220
   Ser Glu Val Leu Ala Ser Val Gly Gly Ser Met Ile Ile Gly Gly Ile
   225 230 235 240
   Asp His Ser Leu Tyr Thr Gly Ser Leu Trp Tyr Thr Pro Ile Arg Arg
   245 250 255
   Glu Trp Tyr Tyr Glu Val Ile Ile Val Arg Val Glu Ile Asn Gly Gln
   260 265 270
   Asp Leu Lys Met Asp Cys Lys Glu Tyr Asn Tyr Asp Lys Ser Ile Val
   275 280 285
   Asp Ser Gly Thr Thr Asn Leu Arg Leu Pro Lys Lys Val Phe Glu Ala
   290 295 300
   Ala Val Lys Ser Ile Lys Ala Ala Ser Ser Thr Glu Lys Phe Pro Asp
   305 310 315 320
   Gly Phe Trp Leu Gly Glu Gln Leu Val Cys Trp Gln Ala Gly Thr Thr
   325 330 335
   Pro Trp Asn Ile Phe Pro Val Ile Ser Leu Tyr Leu Met Gly Glu Val
   340 345 350
   Thr Asn Gln Ser Phe Arg Ile Thr Ile Leu Pro Gln Gln Tyr Leu Arg
   355 360 365
   Pro Val Glu Asp Val Ala Thr Ser Gln Asp Asp Cys Tyr Lys Phe Ala
   370 375 380
   Ile Ser Gln Ser Ser Thr Gly Thr Val Met Gly Ala Val Ile Met Glu
   385 390 395 400
   Gly Phe Tyr Val Val Phe Asp Arg Ala Arg Lys Arg Ile Gly Phe Ala
   405 410 415
   Val Ser Ala Cys His Val His Asp Glu Phe Arg Thr Ala Ala Val Glu
   420 425 430
   Gly Pro Phe Val Thr Leu Asp Met Glu Asp Cys Gly Tyr Asn Ile Pro
   435 440 445
   Gln Thr Asp Glu Ser Thr Leu Met Thr Ile Ala Tyr Val Met Ala Ala
   450 455 460
   Ile Cys Ala Leu Phe Met Leu Pro Leu Cys Asp Tyr Lys Asp Asp Asp
   465 470 475 480
   Asp Lys
<210> 19
   <211> 41
   <212> DNA
   <213> Artificial
<220>
   <223> Primer.
<400> 19
   catctgcgcc ctcttcatgc tggattacaa ggatgacgac g 41
<210> 20
   <211> 41
   <212> DNA
   <213> Artificial
<220>
   <223> Primer.
<400> 20
   cgtcgtcatc cttgtaatcc agcatgaaga gggcgcagat g 41
<210> 21
   <211> 1437
   <212> DNA
   <213> Recombinant DNA
<220>
   <221> CDS
   <222> (1).. (1437)
<220>
   <221> misc_feature
   <222> (1414).. (1437)
   <223> Flag-tag.
<400> 21
   atg gcc caa gcc ctg ccc tgg ctc ctg ctg tgg atg ggc gcg gga gtg      48
   Met Ala Gln Ala Leu Pro Trp Leu Leu Leu Trp Met Gly Ala Gly Val
   1               5                   10                  15
   ctg cct gcc cac ggc acc cag cac ggc atc cgg ctg ccc ctg aga agt      96
   Leu Pro Ala His Gly Thr Gln His Gly Ile Arg Leu Pro Leu Arg Ser
               20                  25                  30
   ggt ctg ggg ggc gcc ccc ctg ggg ctg cgg ctg ccc cgg gag acc gac      144
   Gly Leu Gly Gly Ala Pro Leu Gly Leu Arg Leu Pro Arg Glu Thr Asp
           35                  40                  45
   gaa gag ccc gag gag ccc ggc cgg agg ggc agc ttt gtg gag atg gtg      192
   Glu Glu Pro Glu Glu Pro Gly Arg Arg Gly Ser Phe Val Glu Met Val
       50                  55                  60
   gac aac ctg agg ggc aag tcg ggg cag ggc tac tac gtg gag atg acc      240
   Asp Asn Leu Arg Gly Lys Ser Gly Gln Gly Tyr Tyr Val Glu Met Thr
   65                  70                  75                  80
   gtg ggc agc ccc ccg cag acg ctc aac atc ctg gtg gat aca ggc agc      288
   Val Gly Ser Pro Pro Gln Thr Leu Asn Ile Leu Val Asp Thr Gly Ser
                   85                  90                  95
   agt aac ttt gca gtg ggt gct gcc ccc cac ccc ttc ctg cat cgc tac      336
   Ser Asn Phe Ala Val Gly Ala Ala Pro His Pro Phe Leu His Arg Tyr
               100                 105                 110
   tac cag agg cag ctg tcc agc aca tac cgg gac ctc cgg aag ggt gtg      384
   Tyr Gln Arg Gln Leu Ser Ser Thr Tyr Arg Asp Leu Arg Lys Gly Val
           115                 120                 125
   tat gtg ccc tac acc cag ggc aag tgg gaa ggg gag ctg ggc acc gac      432
   Tyr Val Pro Tyr Thr Gln Gly Lys Trp Glu Gly Glu Leu Gly Thr Asp
       130                 135                 140
   ctg gta agc atc ccc cat ggc ccc aac gtc act gtg cgt gcc aac att      480
   Leu Val Ser Ile Pro His Gly Pro Asn Val Thr Val Arg Ala Asn Ile
   145                 150                 155                 160
   gct gcc atc act gaa tca gac aag ttc ttc atc aac ggc tcc aac tgg      528
   Ala Ala Ile Thr Glu Ser Asp Lys Phe Phe Ile Asn Gly Ser Asn Trp
                   165                 170                 175
   gaa ggc atc ctg ggg ctg gcc tat gct gag att gcc agg cct gac gac      576
   Glu Gly Ile Leu Gly Leu Ala Tyr Ala Glu Ile Ala Arg Pro Asp Asp
               180                 185                 190
   tcc ctg gag cct ttc ttt gac tct ctg gta aag cag acc cac gtt ccc      624
   Ser Leu Glu Pro Phe Phe Asp Ser Leu Val Lys Gln Thr His Val Pro
           195                 200                 205
   aac ctc ttc tcc ctg cag ctt tgt ggt gct ggc ttc ccc ctc aac cag      672
   Asn Leu Phe Ser Leu Gln Leu Cys Gly Ala Gly Phe Pro Leu Asn Gln
       210                 215                 220
   tct gaa gtg ctg gcc tct gtc gga ggg agc atg atc att gga ggt atc      720
   Ser Glu Val Leu Ala Ser Val Gly Gly Ser Met Ile Ile Gly Gly Ile
   225                 230                 235                 240
   gac cac tcg ctg tac aca ggc agt ctc tgg tat aca ccc atc cgg cgg      768
   Asp His Ser Leu Tyr Thr Gly Ser Leu Trp Tyr Thr Pro Ile Arg Arg
                   245                 250                 255
   gag tgg tat tat gag gtg atc att gtg cgg gtg gag atc aat gga cag      816
   Glu Trp Tyr Tyr Glu Val Ile Ile Val Arg Val Glu Ile Asn Gly Gln
               260                 265                 270
   gat ctg aaa atg gac tgc aag gag tac aac tat gac aag agc att gtg      864
   Asp Leu Lys Met Asp Cys Lys Glu Tyr Asn Tyr Asp Lys Ser Ile Val
           275                 280                 285
   gac agt ggc acc acc aac ctt cgt ttg ccc aag aaa gtg ttt gaa gct      912
   Asp Ser Gly Thr Thr Asn Leu Arg Leu Pro Lys Lys Val Phe Glu Ala
       290                 295                 300
   gca gtc aaa tcc atc aag gca gcc tcc tcc acg gag aag ttc cct gat      960
   Ala Val Lys Ser Ile Lys Ala Ala Ser Ser Thr Glu Lys Phe Pro Asp
   305                 310                 315                 320
   ggt ttc tgg cta gga gag cag ctg gtg tgc tgg caa gca ggc acc acc     1008
   Gly Phe Trp Leu Gly Glu Gln Leu Val Cys Trp Gln Ala Gly Thr Thr
                   325                 330                 335
   cct tgg aac att ttc cca gtc atc tca ctc tac cta atg ggt gag gtt      1056
   Pro Trp Asn Ile Phe Pro Val Ile Ser Leu Tyr Leu Met Gly Glu Val
               340                 345                 350
   acc aac cag tcc ttc cgc atc acc atc ctt ccg cag caa tac ctg cgg      1104
   Thr Asn Gln Ser Phe Arg Ile Thr Ile Leu Pro Gln Gln Tyr Leu Arg
           355                 360                 365
   cca gtg gaa gat gtg gcc acg tcc caa gac gac tgt tac aag ttt gcc      1152
   Pro Val Glu Asp Val Ala Thr Ser Gln Asp Asp Cys Tyr Lys Phe Ala
       370                 375                 380
   atc tca cag tca tcc acg ggc act gtt atg gga gct gtt atc atg gag      1200
   Ile Ser Gln Ser Ser Thr Gly Thr Val Met Gly Ala Val Ile Met Glu
   385                 390                 395                 400
   ggc ttc tac gtt gtc ttt gat cgg gcc cga aaa cga att ggc ttt gct      1248
   Gly Phe Tyr Val Val Phe Asp Arg Ala Arg Lys Arg Ile Gly Phe Ala
                   405                 410                 415
   gtc agc gct tgc cat gtg cac gat gag ttc agg acg gca gcg gtg gaa      1296
   Val Ser Ala Cys His Val His Asp Glu Phe Arg Thr Ala Ala Val Glu
               420                 425                 430
   ggc cct ttt gtc acc ttg gac atg gaa gac tgt ggc tac aac att cca      1344
   Gly Pro Phe Val Thr Leu Asp Met Glu Asp Cys Gly Tyr Asn Ile Pro
           435                 440                 445
   cag aca gat gag tca acc ctc atg acc ata gcc tat gtc atg gct gcc      1392
   Gln Thr Asp Glu Ser Thr Leu Met Thr Ile Ala Tyr Val Met Ala Ala
       450                 455                 460
   atc tgc gcc ctc ttc atg ctg gat tac aag gat gac gac gat aag          1437
   Ile Cys Ala Leu Phe Met Leu Asp Tyr Lys Asp Asp Asp Asp Lys
   465                 470                 475
<210> 22
   <211> 479
   <212> PRT
   <213> Recombinant DNA
<400> 22
   Met Ala Gln Ala Leu Pro Trp Leu Leu Leu Trp Met Gly Ala Gly Val
   1 5 10 15
   Leu Pro Ala His Gly Thr Gln His Gly Ile Arg Leu Pro Leu Arg Ser
   20 25 30
   Gly Leu Gly Gly Ala Pro Leu Gly Leu Arg Leu Pro Arg Glu Thr Asp
   35 40 45
   Glu Glu Pro Glu Glu Pro Gly Arg Arg Gly Ser Phe Val Glu Met Val
   50 55 60
   Asp Asn Leu Arg Gly Lys Ser Gly Gln Gly Tyr Tyr Val Glu Met Thr
   65 70 75 80
   Val Gly Ser Pro Pro Gln Thr Leu Asn Ile Leu Val Asp Thr Gly Ser
   85 90 95
   Ser Asn Phe Ala Val Gly Ala Ala Pro His Pro Phe Leu His Arg Tyr
   100 105 110
   Tyr Gln Arg Gln Leu Ser Ser Thr Tyr Arg Asp Leu Arg Lys Gly Val
   115 120 125
   Tyr Val Pro Tyr Thr Gln Gly Lys Trp Glu Gly Glu Leu Gly Thr Asp
   130 135 140
   Leu Val Ser Ile Pro His Gly Pro Asn Val Thr Val Arg Ala Asn Ile
   145 150 155 160
   Ala Ala Ile Thr Glu Ser Asp Lys Phe Phe Ile Asn Gly Ser Asn Trp
   165 170 175
   Glu Gly Ile Leu Gly Leu Ala Tyr Ala Glu Ile Ala Arg Pro Asp Asp
   180 185 190
   Ser Leu Glu Pro Phe Phe Asp Ser Leu Val Lys Gln Thr His Val Pro
   195 200 205
   Asn Leu Phe Ser Leu Gln Leu Cys Gly Ala Gly Phe Pro Leu Asn Gln
   210 215 220
   Ser Glu Val Leu Ala Ser Val Gly Gly Ser Met Ile Ile Gly Gly Ile
   225 230 235 240
   Asp His Ser Leu Tyr Thr Gly Ser Leu Trp Tyr Thr Pro Ile Arg Arg
   245 250 255
   Glu Trp Tyr Tyr Glu Val Ile Ile Val Arg Val Glu Ile Asn Gly Gln
   260 265 270
   Asp Leu Lys Met Asp Cys Lys Glu Tyr Asn Tyr Asp Lys Ser Ile Val
   275 280 285
   Asp Ser Gly Thr Thr Asn Leu Arg Leu Pro Lys Lys Val Phe Glu Ala
   290 295 300
   Ala Val Lys Ser Ile Lys Ala Ala Ser Ser Thr Glu Lys Phe Pro Asp
   305 310 315 320
   Gly Phe Trp Leu Gly Glu Gln Leu Val Cys Trp Gln Ala Gly Thr Thr
   325 330 335
   Pro Trp Asn Ile Phe Pro Val Ile Ser Leu Tyr Leu Met Gly Glu Val
   340 345 350
   Thr Asn Gln Ser Phe Arg Ile Thr Ile Leu Pro Gln Gln Tyr Leu Arg
   355 360 365
   Pro Val Glu Asp Val Ala Thr Ser Gln Asp Asp Cys Tyr Lys Phe Ala
   370 375 380
   Ile Ser Gln Ser Ser Thr Gly Thr Val Met Gly Ala Val Ile Met Glu
   385 390 395 400
   Gly Phe Tyr Val Val Phe Asp Arg Ala Arg Lys Arg Ile Gly Phe Ala
   405 410 415
   Val Ser Ala Cys His Val His Asp Glu Phe Arg Thr Ala Ala Val Glu
   420 425 430
   Gly Pro Phe Val Thr Leu Asp Met Glu Asp Cys Gly Tyr Asn Ile Pro
   435 440 445
   Gln Thr Asp Glu Ser Thr Leu Met Thr Ile Ala Tyr Val Met Ala Ala
   450 455 460
   Ile Cys Ala Leu Phe Met Leu Asp Tyr Lys Asp Asp Asp Asp Lys
   465 470 475
<210> 23
   <211> 52
   <212> DNA
   <213> Artificial
<220>
   <223> Primer.
<400> 23
   ttttggtacc tacttatcgt cgtcatcctt gtaatcgaag agggcgcaga tg 52
<210> 24
   <211> 1431
   <212> DNA
   <213> Recombinant DNA
<220>
   <221> CDS
   <222> (1).. (1431)
<220>
   <221> misc_feature
   <222> (1408)..(1431)
   <223> Flag-tag.
<400> 24
   atg gcc caa gcc ctg ccc tgg ctc ctg ctg tgg atg ggc gcg gga gtg      48
   Met Ala Gln Ala Leu Pro Trp Leu Leu Leu Trp Met Gly Ala Gly Val
   1               5                   10                  15
   ctg cct gcc cac ggc acc cag cac ggc atc cgg ctg ccc ctg aga agt      96
   Leu Pro Ala His Gly Thr Gln His Gly Ile Arg Leu Pro Leu Arg Ser
               20                  25                  30
   ggt ctg ggg ggc gcc ccc ctg ggg ctg cgg ctg ccc cgg gag acc gac     144
   Gly Leu Gly Gly Ala Pro Leu Gly Leu Arg Leu Pro Arg Glu Thr Asp
           35                  40                  45
   gaa gag ccc gag gag ccc ggc cgg agg ggc agc ttt gtg gag atg gtg     192
   Glu Glu Pro Glu Glu Pro Gly Arg Arg Gly Ser Phe Val Glu Met Val
       50                  55                  60
   gac aac ctg agg ggc aag tcg ggg cag ggc tac tac gtg gag atg acc     240
   Asp Asn Leu Arg Gly Lys Ser Gly Gln Gly Tyr Tyr Val Glu Met Thr
   65                  70                  75                  80
   gtg ggc agc ccc ccg cag acg ctc aac atc ctg gtg gat aca ggc agc     288
   Val Gly Ser Pro Pro Gln Thr Leu Asn Ile Leu Val Asp Thr Gly Ser
                   85                  90                  95
   agt aac ttt gca gtg ggt gct gcc ccc cac ccc ttc ctg cat cgc tac     336
   Ser Asn Phe Ala Val Gly Ala Ala Pro His Pro Phe Leu His Arg Tyr
               100                 105                 110
   tac cag agg cag ctg tcc agc aca tac cgg gac ctc cgg aag ggt gtg     384
   Tyr Gln Arg Gln Leu Ser Ser Thr Tyr Arg Asp Leu Arg Lys Gly Val
           115                 120                 125
   tat gtg ccc tac acc cag ggc aag tgg gaa ggg gag ctg ggc acc gac     432
   Tyr Val Pro Tyr Thr Gln Gly Lys Trp Glu Gly Glu Leu Gly Thr Asp
       130                 135                 140
   ctg gta agc atc ccc cat ggc ccc aac gtc act gtg cgt gcc aac att      480
   Leu Val Ser Ile Pro His Gly Pro Asn Val Thr Val Arg Ala Asn Ile
   145                 150                 155                 160
   gct gcc atc act gaa tca gac aag ttc ttc atc aac ggc tcc aac tgg      528
   Ala Ala Ile Thr Glu Ser Asp Lys Phe Phe Ile Asn Gly Ser Asn Trp
                   165                 170                 175
   gaa ggc atc ctg ggg ctg gcc tat gct gag att gcc agg cct gac gac      576
   Glu Gly Ile Leu Gly Leu Ala Tyr Ala Glu Ile Ala Arg Pro Asp Asp
               180                 185                 190
   tcc ctg gag cct ttc ttt gac tct ctg gta aag cag acc cac gtt ccc      624
   Ser Leu Glu Pro Phe Phe Asp Ser Leu Val Lys Gln Thr His Val Pro
           195                 200                 205
   aac ctc ttc tcc ctg cag ctt tgt ggt gct ggc ttc ccc ctc aac cag      672
   Asn Leu Phe Ser Leu Gln Leu Cys Gly Ala Gly Phe Pro Leu Asn Gln
       210                 215                 220
   tct gaa gtg ctg gcc tct gtc gga ggg agc atg atc att gga ggt atc      720
   Ser Glu Val Leu Ala Ser Val Gly Gly Ser Met Ile Ile Gly Gly Ile
   225                 230                 235                 240
   gac cac tcg ctg tac aca ggc agt ctc tgg tat aca ccc atc cgg cgg      768
   Asp His Ser Leu Tyr Thr Gly Ser Leu Trp Tyr Thr Pro Ile Arg Arg
                   245                 250                 255
   gag tgg tat tat gag gtg atc att gtg cgg gtg gag atc aat gga cag      816
   Glu Trp Tyr Tyr Glu Val Ile Ile Val Arg Val Glu Ile Asn Gly Gln
               260                 265                 270
   gat ctg aaa atg gac tgc aag gag tac aac tat gac aag agc att gtg      864
   Asp Leu Lys Met Asp Cys Lys Glu Tyr Asn Tyr Asp Lys Ser Ile Val
           275                 280                 285
   gac agt ggc acc acc aac ctt cgt ttg ccc aag aaa gtg ttt gaa gct      912
   Asp Ser Gly Thr Thr Asn Leu Arg Leu Pro Lys Lys Val Phe Glu Ala
       290                 295                 300
   gca gtc aaa tcc atc aag gca gcc tcc tcc acg gag aag ttc cct gat      960
   Ala Val Lys Ser Ile Lys Ala Ala Ser Ser Thr Glu Lys Phe Pro Asp
   305                 310                 315                 320
   ggt ttc tgg cta gga gag cag ctg gtg tgc tgg caa gca ggc acc acc     1008
   Gly Phe Trp Leu Gly Glu Gln Leu Val Cys Trp Gln Ala Gly Thr Thr
                   325                 330                 335
   cct tgg aac att ttc cca gtc atc tca ctc tac cta atg ggt gag gtt     1056
   Pro Trp Asn Ile Phe Pro Val Ile Ser Leu Tyr Leu Met Gly Glu Val
               340                 345                 350
   acc aac cag tcc ttc cgc atc acc atc ctt ccg cag caa tac ctg cgg     1104
   Thr Asn Gln Ser Phe Arg Ile Thr Ile Leu Pro Gln Gln Tyr Leu Arg
           355                 360                 365
   cca gtg gaa gat gtg gcc acg tcc caa gac gac tgt tac aag ttt gcc     1152
   Pro Val Glu Asp Val Ala Thr Ser Gln Asp Asp Cys Tyr Lys Phe Ala
       370                 375                 380
   atc tca cag tca tcc acg ggc act gtt atg gga gct gtt atc atg gag     1200
   Ile Ser Gln Ser Ser Thr Gly Thr Val Met Gly Ala Val Ile Met Glu
   385                 390                 395                 400
   ggc ttc tac gtt gtc ttt gat cgg gcc cga aaa cga att ggc ttt gct     1248
   Gly Phe Tyr Val Val Phe Asp Arg Ala Arg Lys Arg Ile Gly Phe Ala
                   405                 410                 415
   gtc agc gct tgc cat gtg cac gat gag ttc agg acg gca gcg gtg gaa     1296
   Val Ser Ala Cys His Val His Asp Glu Phe Arg Thr Ala Ala Val Glu
               420                 425                 430
   ggc cct ttt gtc acc ttg gac atg gaa gac tgt ggc tac aac att cca     1344
   Gly Pro Phe Val Thr Leu Asp Met Glu Asp Cys Gly Tyr Asn Ile Pro
           435                 440                 445
   cag aca gat gag tca acc ctc atg acc ata gcc tat gtc atg gct gcc      1392
   Gln Thr Asp Glu Ser Thr Leu Met Thr Ile Ala Tyr Val Met Ala Ala
       450                 455                 460
   atc tgc gcc ctc ttc gat tac aag gat gac gac gat aag                  1431
   Ile Cys Ala Leu Phe Asp Tyr Lys Asp Asp Asp Asp Lys
   465                 470                 475
<210> 25
   <211> 477
   <212> PRT
   <213> Recombinant DNA
<400> 25
   Met Ala Gln Ala Leu Pro Trp Leu Leu Leu Trp Met Gly Ala Gly Val
   1 5 10 15
   Leu Pro Ala His Gly Thr Gln His Gly Ile Arg Leu Pro Leu Arg Ser
   20 25 30
   Gly Leu Gly Gly Ala Pro Leu Gly Leu Arg Leu Pro Arg Glu Thr Asp
   35 40 45
   Glu Glu Pro Glu Glu Pro Gly Arg Arg Gly Ser Phe Val Glu Met Val
   50 55 60
   Asp Asn Leu Arg Gly Lys Ser Gly Gln Gly Tyr Tyr Val Glu Met Thr
   65 70 75 80
   Val Gly Ser Pro Pro Gln Thr Leu Asn Ile Leu Val Asp Thr Gly Ser
   85 90 95
   Ser Asn Phe Ala Val Gly Ala Ala Pro His Pro Phe Leu His Arg Tyr
   100 105 110
   Tyr Gln Arg Gln Leu Ser Ser Thr Tyr Arg Asp Leu Arg Lys Gly Val
   115 120 125
   Tyr Val Pro Tyr Thr Gln Gly Lys Trp Glu Gly Glu Leu Gly Thr Asp
   130 135 140
   Leu Val Ser Ile Pro His Gly Pro Asn Val Thr Val Arg Ala Asn Ile
   145 150 155 160
   Ala Ala Ile Thr Glu Ser Asp Lys Phe Phe Ile Asn Gly Ser Asn Trp
   165 170 175
   Glu Gly Ile Leu Gly Leu Ala Tyr Ala Glu Ile Ala Arg Pro Asp Asp
   180 185 190
   Ser Leu Glu Pro Phe Phe Asp Ser Leu Val Lys Gln Thr His Val Pro
   195 200 205
   Asn Leu Phe Ser Leu Gln Leu Cys Gly Ala Gly Phe Pro Leu Asn Gln
   210 215 220
   Ser Glu Val Leu Ala Ser Val Gly Gly Ser Met Ile Ile Gly Gly Ile
   225 230 235 240
   Asp His Ser Leu Tyr Thr Gly Ser Leu Trp Tyr Thr Pro Ile Arg Arg
   245 250 255
   Glu Trp Tyr Tyr Glu Val Ile Ile Val Arg Val Glu Ile Asn Gly Gln
   260 265 270
   Asp Leu Lys Met Asp Cys Lys Glu Tyr Asn Tyr Asp Lys Ser Ile Val
   275 280 285
   Asp Ser Gly Thr Thr Asn Leu Arg Leu Pro Lys Lys Val Phe Glu Ala
   290 295 300
   Ala Val Lys Ser Ile Lys Ala Ala Ser Ser Thr Glu Lys Phe Pro Asp
   305 310 315 320
   Gly Phe Trp Leu Gly Glu Gln Leu Val Cys Trp Gln Ala Gly Thr Thr
   325 330 335
   Pro Trp Asn Ile Phe Pro Val Ile Ser Leu Tyr Leu Met Gly Glu Val
   340 345 350
   Thr Asn Gln Ser Phe Arg Ile Thr Ile Leu Pro Gln Gln Tyr Leu Arg
   355 360 365
   Pro Val Glu Asp Val Ala Thr Ser Gln Asp Asp Cys Tyr Lys Phe Ala
   370 375 380
   Ile Ser Gln Ser Ser Thr Gly Thr Val Met Gly Ala Val Ile Met Glu
   385 390 395 400
   Gly Phe Tyr Val Val Phe Asp Arg Ala Arg Lys Arg Ile Gly Phe Ala
   405 410 415
   Val Ser Ala Cys His Val His Asp Glu Phe Arg Thr Ala Ala Val Glu
   420 425 430
   Gly Pro Phe Val Thr Leu Asp Met Glu Asp Cys Gly Tyr Asn Ile Pro
   435 440 445
   Gln Thr Asp Glu Ser Thr Leu Met Thr Ile Ala Tyr Val Met Ala Ala
   450 455 460
   Ile Cys Ala Leu Phe Asp Tyr Lys Asp Asp Asp Asp Lys
   465 470 475
<210> 26
   <211> 40
   <212> DNA
   <213> Artificial
<220>
   <223> Primer.
<400> 26
   gcctatgtca tggctgccat cgattacaag gatgacgacg 40
<210> 27
   <211> 40
   <212> DNA
   <213> Artificial
<220>
   <223> Primer.
<400> 27
   cgtcgtcatc cttgtaatcg atggcagcca tgacataggc 40
<210> 28
   <211> 1419
   <212> DNA
   <213> Recombinant DNA
<220>
   <221> CDS
   <222> (1).. (1419)
<220>
   <221> misc_feature
   <222> (1396).. (1419)
   <223> Flag-tag.
<400> 28
   atg gcc caa gcc ctg ccc tgg ctc ctg ctg tgg atg ggc gcg gga gtg      48
   Met Ala Gln Ala Leu Pro Trp Leu Leu Leu Trp Met Gly Ala Gly Val
   1               5                   10                  15
   ctg cct gcc cac ggc acc cag cac ggc atc cgg ctg ccc ctg aga agt      96
   Leu Pro Ala His Gly Thr Gln His Gly Ile Arg Leu Pro Leu Arg Ser
               20                  25                  30
   ggt ctg ggg ggc gcc ccc ctg ggg ctg cgg ctg ccc cgg gag acc gac     144
   Gly Leu Gly Gly Ala Pro Leu Gly Leu Arg Leu Pro Arg Glu Thr Asp
           35                  40                  45
   gaa gag ccc gag gag ccc ggc cgg agg ggc agc ttt gtg gag atg gtg     192
   Glu Glu Pro Glu Glu Pro Gly Arg Arg Gly Ser Phe Val Glu Met Val
       50                  55                  60
   gac aac ctg agg ggc aag tcg ggg cag ggc tac tac gtg gag atg acc     240
   Asp Asn Leu Arg Gly Lys Ser Gly Gln Gly Tyr Tyr Val Glu Met Thr
   65                  70                  75                  80
   gtg ggc agc ccc ccg cag acg ctc aac atc ctg gtg gat aca ggc agc     288
   Val Gly Ser Pro Pro Gln Thr Leu Asn Ile Leu Val Asp Thr Gly Ser
                   85                  90                  95
   agt aac ttt gca gtg ggt gct gcc ccc cac ccc ttc ctg cat cgc tac     336
   Ser Asn Phe Ala Val Gly Ala Ala Pro His Pro Phe Leu His Arg Tyr
               100                 105                 110
   tac cag agg cag ctg tcc agc aca tac cgg gac ctc cgg aag ggt gtg     384
   Tyr Gln Arg Gln Leu Ser Ser Thr Tyr Arg Asp Leu Arg Lys Gly Val
           115                 120                 125
   tat gtg ccc tac acc cag ggc aag tgg gaa ggg gag ctg ggc acc gac     432
   Tyr Val Pro Tyr Thr Gln Gly Lys Trp Glu Gly Glu Leu Gly Thr Asp
       130                 135                 140
   ctg gta agc atc ccc cat ggc ccc aac gtc act gtg cgt gcc aac att     480
   Leu Val Ser Ile Pro His Gly Pro Asn Val Thr Val Arg Ala Asn Ile
   145                 150                 155                 160
   gct gcc atc act gaa tca gac aag ttc ttc atc aac ggc tcc aac tgg     528
   Ala Ala Ile Thr Glu Ser Asp Lys Phe Phe Ile Asn Gly Ser Asn Trp
                   165                 170                 175
   gaa ggc atc ctg ggg ctg gcc tat gct gag att gcc agg cct gac gac     576
   Glu Gly Ile Leu Gly Leu Ala Tyr Ala Glu Ile Ala Arg Pro Asp Asp
               180                 185                 190
   tcc ctg gag cct ttc ttt gac tct ctg gta aag cag acc cac gtt ccc     624
   Ser Leu Glu Pro Phe Phe Asp Ser Leu Val Lys Gln Thr His Val Pro
           195                 200                 205
   aac ctc ttc tcc ctg cag ctt tgt ggt gct ggc ttc ccc ctc aac cag      672
   Asn Leu Phe Ser Leu Gln Leu Cys Gly Ala Gly Phe Pro Leu Asn Gln
       210                 215                 220
   tct gaa gtg ctg gcc tct gtc gga ggg agc atg atc att gga ggt atc      720
   Ser Glu Val Leu Ala Ser Val Gly Gly Ser Met Ile Ile Gly Gly Ile
   225                 230                 235                 240
   gac cac tcg ctg tac aca ggc agt ctc tgg tat aca ccc atc cgg cgg      768
   Asp His Ser Leu Tyr Thr Gly Ser Leu Trp Tyr Thr Pro Ile Arg Arg
                   245                 250                 255
   gag tgg tat tat gag gtg atc att gtg cgg gtg gag atc aat gga cag      816
   Glu Trp Tyr Tyr Glu Val Ile Ile Val Arg Val Glu Ile Asn Gly Gln
               260                 265                 270
   gat ctg aaa atg gac tgc aag gag tac aac tat gac aag agc att gtg      864
   Asp Leu Lys Met Asp Cys Lys Glu Tyr Asn Tyr Asp Lys Ser Ile Val
           275                 280                 285
   gac agt ggc acc acc aac ctt cgt ttg ccc aag aaa gtg ttt gaa gct      912
   Asp Ser Gly Thr Thr Asn Leu Arg Leu Pro Lys Lys Val Phe Glu Ala
       290                 295                 300
   gca gtc aaa tcc atc aag gca gcc tcc tcc acg gag aag ttc cct gat      960
   Ala Val Lys Ser Ile Lys Ala Ala Ser Ser Thr Glu Lys Phe Pro Asp
   305                 310                 315                 320
   ggt ttc tgg cta gga gag cag ctg gtg tgc tgg caa gca ggc acc acc     1008
   Gly Phe Trp Leu Gly Glu Gln Leu Val Cys Trp Gln Ala Gly Thr Thr
                   325                 330                 335
   cct tgg aac att ttc cca gtc atc tca ctc tac cta atg ggt gag gtt     1056
   Pro Trp Asn Ile Phe Pro Val Ile Ser Leu Tyr Leu Met Gly Glu Val
               340                 345                 350
   acc aac cag tcc ttc cgc atc acc atc ctt ccg cag caa tac ctg cgg     1104
   Thr Asn Gln Ser Phe Arg Ile Thr Ile Leu Pro Gln Gln Tyr Leu Arg
           355                 360                 365
   cca gtg gaa gat gtg gcc acg tcc caa gac gac tgt tac aag ttt gcc     1152
   Pro Val Glu Asp Val Ala Thr Ser Gln Asp Asp Cys Tyr Lys Phe Ala
       370                 375                 380
   atc tca cag tca tcc acg ggc act gtt atg gga gct gtt atc atg gag     1200
   Ile Ser Gln Ser Ser Thr Gly Thr Val Met Gly Ala Val Ile Met Glu
   385                 390                 395                 400
   ggc ttc tac gtt gtc ttt gat cgg gcc cga aaa cga att ggc ttt gct     1248
   Gly Phe Tyr Val Val Phe Asp Arg Ala Arg Lys Arg Ile Gly Phe Ala
                   405                 410                 415
   gtc agc gct tgc cat gtg cac gat gag ttc agg acg gca gcg gtg gaa     1296
   Val Ser Ala Cys His Val His Asp Glu Phe Arg Thr Ala Ala Val Glu
               420                 425                 430
   ggc cct ttt gtc acc ttg gac atg gaa gac tgt ggc tac aac att cca     1344
   Gly Pro Phe Val Thr Leu Asp Met Glu Asp Cys Gly Tyr Asn Ile Pro
           435                 440                 445
   cag aca gat gag tca acc ctc atg acc ata gcc tat gtc atg gct gcc     1392
   Gln Thr Asp Glu Ser Thr Leu Met Thr Ile Ala Tyr Val Met Ala Ala
       450                 455                 460
   atc gat tac aag gat gac gac gat aag                                 1419
   Ile Asp Tyr Lys Asp Asp Asp Asp Lys
   465                 470
<210> 29
   <211> 473
   <212> PRT
   <213> Recombinant DNA
<400> 29
   Met Ala Gln Ala Leu Pro Trp Leu Leu Leu Trp Met Gly Ala Gly Val
   1               5                   10                  15
   Leu Pro Ala His Gly Thr Gln His Gly Ile Arg Leu Pro Leu Arg Ser
               20                  25                  30
   Gly Leu Gly Gly Ala Pro Leu Gly Leu Arg Leu Pro Arg Glu Thr Asp
           35                  40                  45
   Glu Glu Pro Glu Glu Pro Gly Arg Arg Gly Ser Phe Val Glu Met Val
       50                  55                  60
   Asp Asn Leu Arg Gly Lys Ser Gly Gln Gly Tyr Tyr Val Glu Met Thr
   65                  70                  75                  80
   Val Gly Ser Pro Pro Gln Thr Leu Asn Ile Leu Val Asp Thr Gly Ser
                   85                  90                  95
   Ser Asn Phe Ala Val Gly Ala Ala Pro His Pro Phe Leu His Arg Tyr
               100                 105                 110
   Tyr Gln Arg Gln Leu Ser Ser Thr Tyr Arg Asp Leu Arg Lys Gly Val
           115                 120                 125
   Tyr Val Pro Tyr Thr Gln Gly Lys Trp Glu Gly Glu Leu Gly Thr Asp
       130                 135                 140
   Leu Val Ser Ile Pro His Gly Pro Asn Val Thr Val Arg Ala Asn Ile
   145                 150                 155                 160
   Ala Ala Ile Thr Glu Ser Asp Lys Phe Phe Ile Asn Gly Ser Asn Trp
                   165                 170                 175
   Glu Gly Ile Leu Gly Leu Ala Tyr Ala Glu Ile Ala Arg Pro Asp Asp
               180                 185                 190
   Ser Leu Glu Pro Phe Phe Asp Ser Leu Val Lys Gln Thr His Val Pro
           195                 200                 205
   Asn Leu Phe Ser Leu Gln Leu Cys Gly Ala Gly Phe Pro Leu Asn Gln
       210                 215                 220
   Ser Glu Val Leu Ala Ser Val Gly Gly Ser Met Ile Ile Gly Gly Ile
   225                 230                 235                 240
   Asp His Ser Leu Tyr Thr Gly Ser Leu Trp Tyr Thr Pro Ile Arg Arg
                   245                 250                 255
   Glu Trp Tyr Tyr Glu Val Ile Ile Val Arg Val Glu Ile Asn Gly Gln
               260                 265                 270
   Asp Leu Lys Met Asp Cys Lys Glu Tyr Asn Tyr Asp Lys Ser Ile Val
           275                 280                 285
   Asp Ser Gly Thr Thr Asn Leu Arg Leu Pro Lys Lys Val Phe Glu Ala
       290                 295                 300
   Ala Val Lys Ser Ile Lys Ala Ala Ser Ser Thr Glu Lys Phe Pro Asp
   305 310 315 320
   Gly Phe Trp Leu Gly Glu Gln Leu Val Cys Trp Gln Ala Gly Thr Thr
   325 330 335
   Pro Trp Asn Ile Phe Pro Val Ile Ser Leu Tyr Leu Met Gly Glu Val
   340 345 350
   Thr Asn Gln Ser Phe Arg Ile Thr Ile Leu Pro Gln Gln Tyr Leu Arg
   355 360 365
   Pro Val Glu Asp Val Ala Thr Ser Gln Asp Asp Cys Tyr Lys Phe Ala
   370 375 380
   Ile Ser Gln Ser Ser Thr Gly Thr Val Met Gly Ala Val Ile Met Glu
   385 390 395 400
   Gly Phe Tyr Val Val Phe Asp Arg Ala Arg Lys Arg Ile Gly Phe Ala
   405 410 415
   Val Ser Ala Cys His Val His Asp Glu Phe Arg Thr Ala Ala Val Glu
   420 425 430
   Gly Pro Phe Val Thr Leu Asp Met Glu Asp Cys Gly Tyr Asn Ile Pro
   435 440 445
   Gln Thr Asp Glu Ser Thr Leu Met Thr Ile Ala Tyr Val Met Ala Ala
   450 455 460
   Ile Asp Tyr Lys Asp Asp Asp Asp Lys
   465 470

## Description

### Technical Field

The present invention relates to a screening method for a substance inhibiting a transmembrane enzyme by specifically binding to a transmembrane region of the enzyme.

### Background Art

Alzheimer's disease is a neurodegenerative disease characterized by the formation of senile plaques and neurofibrillary tangles, as well as neuron degenerations/deficits. The senil plaque, the most characteristic of Alzheimer's disease, is formed by the deposition of biological constituents in the brain, wherein a β amyloid protein (hereinafter, sometimes abbreviated as Aβ) is a main constituent thereof. Aβ consisting of 40 or 42 amino acids (hereinafter, abbreviated as Aβ 1-40 and Aβ 1-42, respectively) are known to exhibit toxicity to neurons. Accordingly, a pharmaceutical agent inhibiting the production and/or secretion of Aβ is effective for the prophylaxis and/or treatment of diseases due to Aβ (e.g., Alzheimer's disease, Down syndrome and the like).

Aβ 1-40 and Aβ 1-42 are produced from their precursor protein, APP (Amyloid Precursor Protein), by cleavage by a β-secretase and a γ-secretase. It is believed that a pharmaceutical agent inhibiting these enzymes inhibits the production and/or secretion of Aβ, so that it can exert a fundamental prophylactic and/or therapeutic effect on a patient with the increased Aβ protein in the brain, such as an individual likely to have a genetic predisposition to develop the diseases due to Aβ (e.g., Alzheimer's disease (AD), Down syndrome and the like), particularly familial Alzheimer's disease (FAD) and the like, and a patient with the increased Aβ protein in the brain due to a trauma and the like.

In 1999, 4 pharmaceutical company groups almost simultaneously have reported the isolation of cDNA for BACE1 (Beta-site APP Cleaving Enzyme 1; also referred as Asp2, memapsin 2), a protease responsible for β-secretase activity (for review, see Varghese J. et al., J. Med. Chem., Vol. 46, 22, pp.4625-4630 (2003)). BACE1 is a single transmembrane protein consisting of 501 amino acids and a typical aspartic protease with an active center being two aspartic acids in the extracellular side (the lumenal side, in the case of the endoplasmic reticulum or Golgi apparatus). Subsequently, it has been demonstrated that the production of Aβ completely disappears in BACE1-knock out mice, revealing that BACE1 is a β-secretase itself. Moreover, because the KO mice exhibit no severe developmental abnormality and there is little change in the gene expression profile, it is expected that a β-secretase inhibitor will be a therapeutic drug for AD, which is safer and has less side effects than a γ-secretase inhibitor (it is known that a γ-secretase cleaves Notch 1 involving in differentiation of immunocyte as well as APP, and inhibiting the cleavage of Notch 1 leads to immune abnormalities).

Because β-secretase is an aspartic protease, a peptidic inhibitor having a statin, hydroxyethylene, or hydroxyethylcarbonyl structure, which can function as a transition state mimic has been developed (for review, see Varghese J. et al., J. Med. Chem., Vol. 46, 22, pp.4625-4630 (2003)). However, the peptidic inhibitor is problematic in the *in vivo* kinetics and the translocation efficiency into the brain, and in many cases, it requires high concentration to exert the drug efficacy *in vivo.*

A low-molecular-weight, non-peptidic inhibitor not only can overcome the above-mentioned problems, but also may have a possibility to find out the novel site of inhibitory action. Such low-molecular-weight compounds have been gradually developed (WO 01/87293, WO 02/88101 and WO 02/96897), and include for example, one having possibility to directly act on the active center of β-secretase (WO 02/88101) and one having possibility to shift the APP processing from by β-secretase cleavage to by α-secretase cleavage (WO 02/96897), as suggested by a computer simulation. And, it has been reported that green tea catechins inhibit β-secretase activity non-competitively to the substrate (Jeon S.Y. et al., Bioorg. Med. Chem. Lett., Vol. 13, pp.3905-3908 (2003)).

On the other hand, as a screening method for β-secretase inhibitor, for example, in WO 01/87293, there is described a method detecting the cleavage activity of a recombinant human β-secretase protein, by using a peptide labeled with both a fluorescence donor and a fluorescence quencher, as a substrate.

In WO 94/10569 and JP-A-H07-165606, there is described a method identifying an inhibitor of β-amyloid peptide (βAP) production, by using a βAP-producing cell line and the change in the amount of the produced soluble βAPs as an indication. In patent reference JP-A-2003-261596, there is described a screening method for β-secretase inhibitor, with the binding to the active site of β-secretase as an indication.

In WO 99/43711 peptides are disclosed inhibiting the function of guanine nucleotid-binding regulatory protein-coupled receptors (GPCR) by targeting the transmembrane region of these receptors. However, the document does not relate to the inhibition of β-secretase.

In WO 00/24780 antibodies are disclosed, which bind to an epitope present in the transmembrane domain of polycystin for a better characterization of the tissue distribution and intracellular localization of polycystin. However, no inhibitory action of the antibodies is disclosed nor does the document refer to β-secretase.

In GB 2367060 a screening method for modulators of β-secretase activity is disclosed. This method comprises following steps: (a) contacting a first composition comprising a mammalian β-secretase polypeptide or biologically active fragment thereof with a second composition comprising a substrate polypeptide having an amino acid sequence comprising a β-secretase cleavage site both in the presence and in the absence of a putative modulator compound; (b) measuring cleavage of the substrate polypeptide in the presence and in the absence of the putative modulator compound; and (c) identifying modulators of β-secretase activity from a difference in cleavage in the presence versus in the absence of the putative modulator compound.

Yan Riqiang et al. describe that the transmembrane domain of β-secretase determines its localization and access to β amyloid precursor protein and is therefore required for the activity of β-secretase (Journal of Biological Chemistry, September 28, 2001, vol. 276, no. 39, p. 36788-36796). However, no inhibition of the activity of β-secretase by substrates that bind on the transmembrane region of the enzyme is reported and furthermore, no advantages over the inhibition of the active center of β-secretase are mentioned.

### Disclosure of the Invention

As above-mentioned, a number of pharmaceutical companies have made a lot of effort to research and develop β-secretase inhibitors. To find a novel site of inhibitory action of β-secretase is also important to develop a compound with superior β-secretase inhibitory action.

Accordingly, the object of the present invention is to provide a screening method for a β-secretase inhibitor acting on a novel site for inhibition, as well as to provide a compound having a superior β-secretase inhibitory action and therefore a superior prophylactic and/or therapeutic activity on AD by acting on the site for inhibition.

The present inventors carried out a kinetic analysis of β-secretase inhibition for the various compounds having a β-secretase inhibitory activity described in the above-mentioned WO 01/87293, and as a result, found out that the inhibition mode of these compounds was non-competitive inhibition. Thus, we investigated for a site of the β-secretase protein to which these compounds bind, and unexpectedly it was revealed that these compounds inhibited the β-secretase activity by binding to a transmembrane region of the β-secretase protein. Although allosteric inhibitors which bind to a region other than the active region to non-competitively inhibit the enzyme activity are well known for many enzymes, there is no report to date regarding an inhibiting substance which exerts its inhibitory activity by binding to a transmembrane region of a transmembrane enzyme as well as β-secretase.

The present inventors produced a various mutant proteins with the transmembrane region gradually truncated from C terminal of a β-secretase protein by using genetic engineering procedures and successfully identified a site of β-secretase protein to which these compounds bind, and by using a β-secretase protein retaining the binding site and a β-secretase protein lacking the binding site, established a screening system which is able to detect the binding of a test compound to these proteins and the enzyme inhibitory activity of the test compound, which resulted in the completion of the present invention.

That is, the present invention provides:
[1] A screening method for a β-secretase inhibiting substance specifically binding to a transmembrane region of the secretase, comprising the following steps:
   (I) measurement of the activity of a first protein having a part or all of an amino acid sequence of the secretase, comprising
      (i) a region comprising an active center of the β-secretase and
      (ii) a region comprising a part or all of a transmembrane region of the β-secretase, comprising at least a binding site targeted by a substance having a subject enzyme inhibitory activity;
   (II) measuring the binding of a test substance to the first protein;
   (III) selection of a β-secretase inhibiting substance, which is the test substance that inhibits β-secretase activity by binding to the transmembrane region (ii) of the β-secretase,
   wherein region (i) comprises an amino acid sequence comprising a sequence homology of not less than 70% with the amino acid sequence shown by amino acid 93-292 in the amino acid sequence shown by SEQ ID NO: 2,
   the transmembrane region of the β-secretase has an amino acid sequence having a sequence homology of not less than 70% with the amino acid sequence shown by amino acid 455-480 in the amino acid sequence shown by SEQ ID NO: 2, and
   the region (ii) above comprises a region having an amino acid sequence having a sequence homology of not less than about 70% with the amino acid sequence shown by amino acid 466-471 in the amino acid sequence shown by SEQ ID NO: 2.
[2] The method of the above-mentioned [1], further comprising the following step:
   (I') measurement of the activity of a second protein having a part of an amino acid sequence of the β-secretase, comprising the region (i) above in the first protein and lacking the part or all of the region (ii) above in the first protein in the presence of the test substance,
      wherein the assessment in step (II) above comprises:
      (a) measuring the binding of the test substance to the first protein;
      (b) measuring the binding of the test substance to the second protein;
      (c) determining that the test substance binds to region (ii) above in the β-secretase if the test substance binds with the first protein but does not bind with the second protein
         and the selection in the step (III) above comprises:
      (d) selection of the β-secretase inhibiting substance, which inhibits β-secretase activity by binding to the transmembrane region (ii) of β-secretase, test substance which binds to the first protein and inhibits the enzyme activity of the first protein but does not bind and does not inhibit the enzyme activity of the second protein.
[3] The method of the above-mentioned [1], wherein region (i) above has an amino acid sequence having a sequence homology of not less than 70% with the amino acid sequence shown by amino acid 46-454 in the amino acid sequence shown by SEQ ID NO: 2.
[4] A kit for screening for a β-secretase inhibiting substance specifically binding to a transmembrane region of the β-secretase, comprising the following (a) and (b):
   (a) a first protein having a part or all of an amino acid sequence of the β-secretase, comprising
      (i) a region comprising an active center of the β-secretase and
      (ii) a region comprising a part or all of the transmembrane region of the β-secretase; and
   (b) a second protein having a part of an amino acid sequence of the β-secretase, comprising a region (i) above in the first protein and lacking region (ii) above in the first protein ,
   wherein region (i) comprises an amino acid sequence having a sequence homology of not less than 70% with the amino acid sequence shown by amino acid 93-292 in the amino acid sequence shown by SEQ ID NO: 2,
   the transmembrane region of the β-secretase has an amino acid sequence having a sequence homology of not less than 70% with the amino acid sequence shown by amino acid 455-480 in the amino acid sequence shown by SEQ ID NO: 2, and
   the region (ii) above comprises a region having an amino acid sequence having a sequence homology of not less than 70% with the amino acid sequence shown by amino acid 466-471 in the amino acid sequence shown by SEQ ID NO: 2.
[5] The kit of the above-mentioned [4], wherein region (i) above has an amino acid sequence having a sequence homology of not less than 70% with the amino acid sequence shown by amino acid 46-454 in the amino acid sequence shown by SEQ ID NO: 2.

Further it is described
[9] a β-secretase selective inhibitor comprising a β-secretase inhibiting substance binding to a transmembrane region of the enzyme, wherein the substance is other than the compounds presented by the following structural formulas: 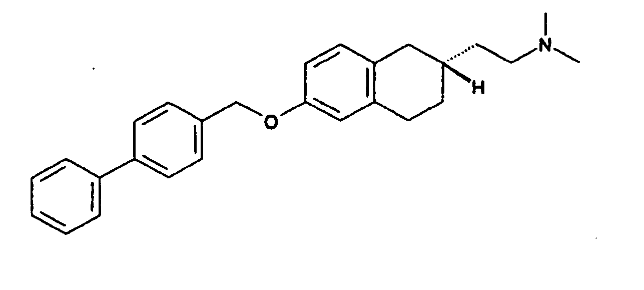 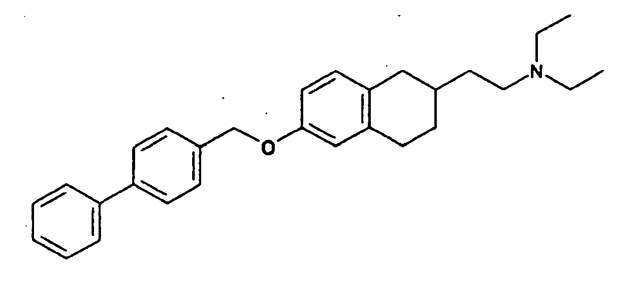 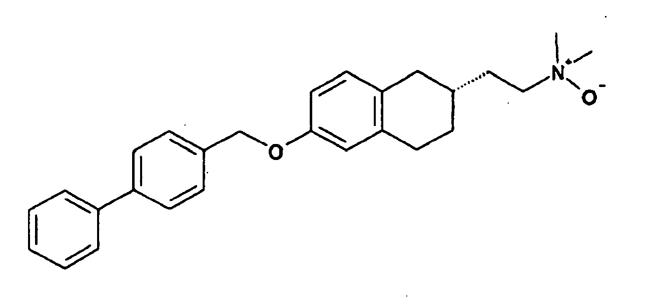 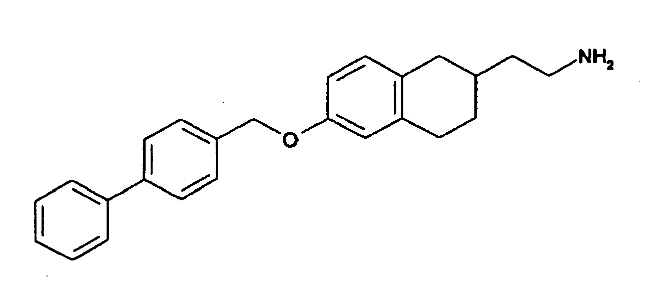 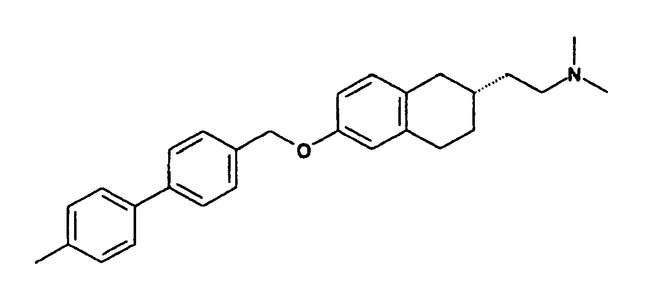
[10] the inhibitor of the above-mentioned [9], wherein a β-secretase binding site of the inhibiting substance is present in an amino acid sequence shown by amino acid 466-471 in an amino acid sequence shown by SEQ ID NO: 2;
[11] the inhibitor of the above-mentioned [9], which is used for the prophylaxis and/or treatment of a disease selected from the group consisting of Alzheimer's disease, Down syndrome and Age-Associated Memory Impairment;
[12] the inhibitor of the above-mentioned [11], characterized by causing no blood pressure reduction;
[13] a method of selectively inhibiting β-secretase, characterized by using a β-secretase inhibiting substance binding to a transmembrane region of the enzyme, wherein the substance is other than the compounds presented by the following structural formulas: 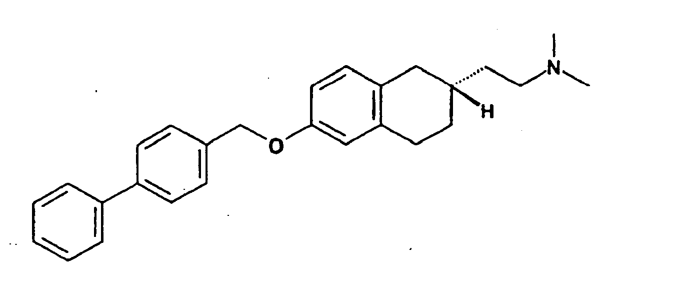 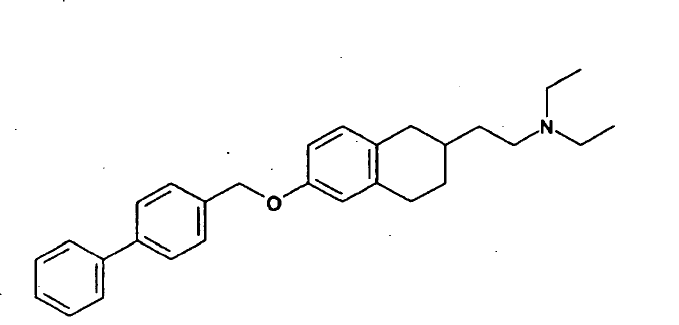 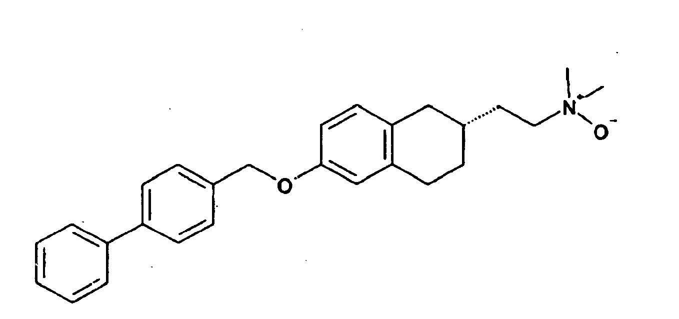 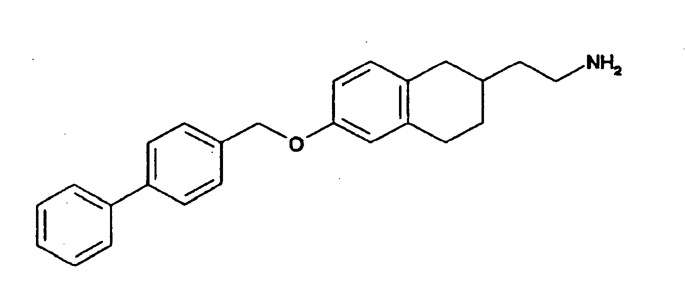 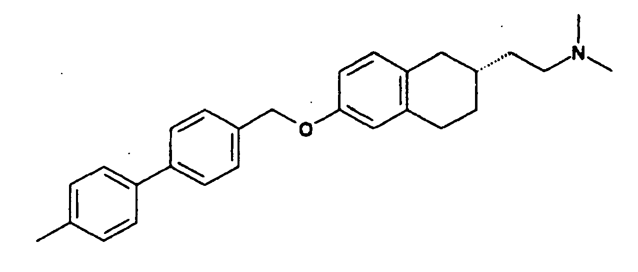
[14] the method of the above-mentioned [13], wherein a β-secretase binding site of the inhibiting substance is present in an amino acid sequence shown by amino acid 466-471 in an amino acid sequence shown by SEQ ID NO: 2;
[15] the method of the above-mentioned [13], which is for prophylaxis and/or treatment of a disease selected from the group consisting of Alzheimer's disease, Down syndrome and Age-Associated Memory Impairment;
[16] the method of the above-mentioned [15], characterized by causing no blood pressure reduction;
   Further described herein is the
[17] use of a β-secretase inhibiting substance binding to a transmembrane region of the enzyme for the production of a secretase selective inhibitor, wherein the substance is other than the compounds presented by the following structural formulas: 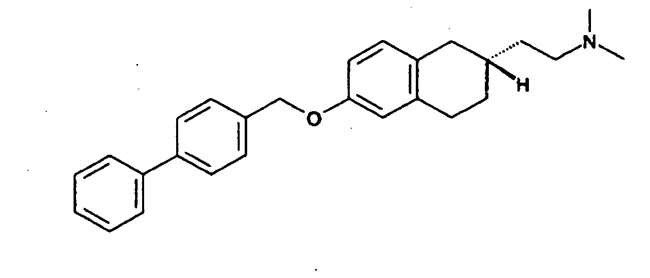 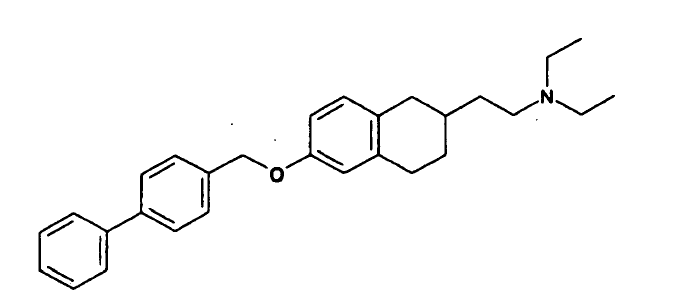 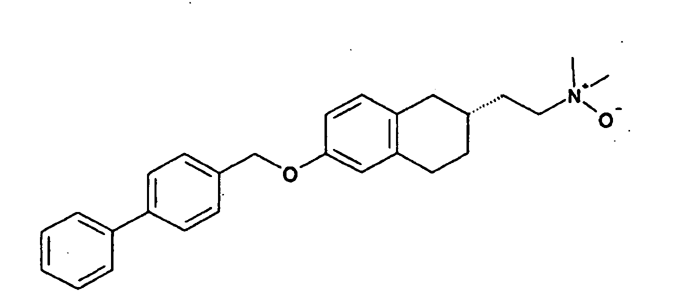 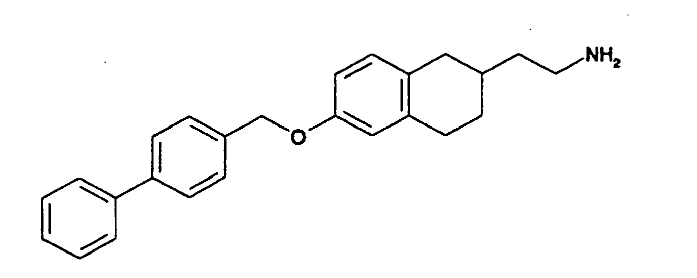 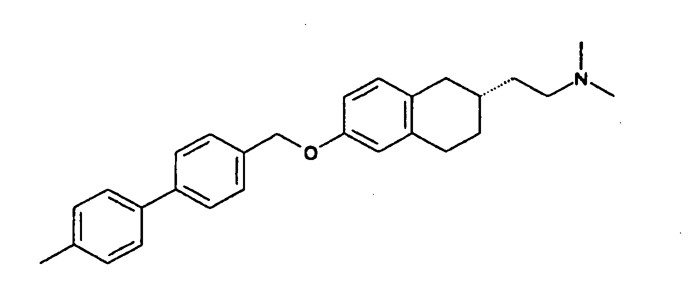
[18] the use of the above-mentioned [17], wherein a β-secretase binding site of the inhibiting substance is present in an amino acid sequence shown by amino acid 466-471 in an amino acid sequence shown by SEQ ID NO: 2;
[19] the use of the above-mentioned [17], wherein the inhibitor is used for the prophylaxis and/or treatment of a disease selected from the group consisting of Alzheimer's disease, Down syndrome and Age-Associated Memory Impairment; and
[20] the use of the above-mentioned [19], wherein the inhibitor is characterized by causing no blood pressure reduction.

A screening method or a kit for screening of the present invention can select a compound which inhibits a β-secretase activity by binding to the transmembrane region of the enzyme, such that it can provide an advantageous effect capable of selecting a compound which selectively acts on β-secretase and not inhibits other aspartic proteases, unlike a transition state mimic and other inhibitors acting on the active center.

### Brief Description of the Drawings

Fig. 1 shows a Lineweaver-Burk plot for analysis of the mode of compound D to inhibit BACE1-501. The concentrations of Compound D are: ●:30 µM, ▲:10 µM, and ■:0 µM.
Fig. 2 shows a signal of surface plasmon resonance (Resonance Unit) demonstrating the binding of BACE1-501 and Compound J.

### Best Mode for Embodying the Invention

A screening method of the present invention for a transmembrane enzyme inhibiting substance specifically binding to a transmembtrane region of the enzyme (hereinafter, sometimes to be abbreviated as "a screening method of the present invention") is characterized by using a protein having a part or all of an amino acid sequence of the enzyme, comprising a region comprising an active center and a part or all of a transmembrane region of the transmembrane enzyme, wherein the transmembrane enzyme is a β-secretase.

By "a region including an active center of a transmembrane enzyme" is meant a region which comprises an active center and comprises a partial amino acid sequence of the enzyme sufficient for the expression of the enzyme activity. The position of the region on the sequence is not limited, and the region may be at any position including N-terminal region, internal sequence and C-terminal region of the enzyme protein. The region may also be at any position including an extracellular (lumenal in the case of the endoplasmic reticulum, Golgi apparatus and others), cytoplasmic or transmembrane region (wherein, which is different from the binding site for the enzyme inhibiting substance). For example, when the active center is present within the extracellular (or lumenal) region, the cytoplasmic region or the transmembrane region, said "region comprising an active center" may be a whole of any region in which the active center is present, or if the 3D structure of the enzyme has been revealed by for example, X-ray crystal structure analysis and the like, only at least a part required for expression of the enzyme activity, within the region in which the active center is present, can also be used.

By "a part or all of a transmembrane region" is meant that it comprises at least a binding site targeted by a compound having a subject enzyme inhibitory activity. Although the binding site can be set optionally within the transmembrane region, if the active center of the transmembrane enzyme is present in the transmembrane region, the binding site is selected from within said transmembrane region other than the site at which the active center is present.

As aforementioned, the transmembrane enzymes to which a screening method of the present invention can be applied is a β-secretase. The β-secretase protein in the present invention is a protein comprising the same or substantially the same amino acid sequence as an amino acid sequence shown by amino acid 46-501 in an amino acid sequence shown by SEQ ID NO: 2.

The β-secretase protein may be a protein derived from a cell (e.g., splenocyte, nerve cell, glial cell, pancreatic β cell, myeloid cell, mesangial cell, Langerhans' cell, epidermal cell, epithelial cell, goblet cell, endothelial cell, smooth muscle cell, fibroblast, fibrocyte, myocyte, adipocyte, immune cell (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte or interstitial cell, or a corresponding precursor cell, stem cell or cancer cell thereof, and the like) of a warm-blooded animal (e.g., human, mouse, rat, guinea pig, hamster, rabbit, sheep, goat, swine, bovine, horse, bird, cat, dog, monkey, chimpanzee and the like), or any tissue where such cells are present (e.g., brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, muscle, lung, gastrointestinal tract (e.g., large intestine and small intestine), blood vessel, heart, thymus, spleen, submandibular gland, peripheral blood, prostate, testicle, ovary, placenta, uterus, bone, joint, adipose tissue, skeletal muscle, and the like). The β-secretase protein may also be a chemically synthesized protein or a protein synthesized using a cell-free translation system. Alternatively, the β-secretase protein may be a recombinant protein produced by a transformant introduced with a nucleic acid having the base sequence that encodes the above-described amino acid sequence.

As "substantially the same amino acid sequence" as an amino acid sequence shown by amino acid 46-501 in an amino acid sequence shown by SEQ ID NO: 2, an amino acid sequence having a homology of about 70% or more, preferably about 80% or more, more preferably about 90% or more, particularly preferably about 95% or more, and most preferably about 98% or more, with an amino acid sequence shown by amino acid 46-501 in an amino acid sequence shown by SEQ ID NO: 2 can be mentioned. As used herein, the "homology" means the ratio (%) in an optimal alignment (preferably, the algorithm can consider introduction of gap into one or both of sequences for optimal alignment) of the same amino acid and analogous amino acid residue relative to the overlapping whole amino acid residue, when two amino acid sequences are aligned using a mathematical algorithm known in the technique field. The "analogous amino acid" means amino acid analogous in physicochemical properties and, for example, amino acids classified into the same group such as aromatic amino acid (Phe, Trp, Tyr), aliphatic amino acid (Ala, Leu, Ile, Val), polar amino acid (Gln, Asn), basic amino acid (Lys, Arg, His), acidic amino acid (Glu, Asp), amino acid having a hydroxyl group (Ser, Thr), amino acid having a small side chain (Gly, Ala, Ser, Thr, Met) and the like can be mentioned. Substitution with such analogous amino acid is predicted to cause no change in the phenotype of protein (i.e., conservative amino acid substitution). Specific examples of the conservative amino acid substitution are well known in the technique field and described in various literatures (e.g., see Bowie et al., Science, 247: 1306-1310 (1990)).

The homology of amino acid sequence in the present specification can be calculated using the homology calculation algorithm NCBI BLAST (National Center for Biotechnology Information Basic Local Alignment Search Tool) under the following conditions (expectancy = 10; allowing gap; matrix=BLOSUM62;filtering=OFF). As other algorithm for determining the homology of amino acid sequence, for example, the algorithm described in Karlin et al., Proc. Natl. Acad. Sci. USA, 90: 5873-5877 (1993) [the algorithm is incorporated in NBLAST and XBLAST program (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in GCG software package], the algorithm described in Myers and Miller, CABIOS, 4: 11-17 (1988) [the algorithm is incorporated in ALIGN program (version 2.0) which is a part of the CGC sequence alignment software package], the algorithm described in Pearson et al., Proc. Natl. Acad. Sci. USA, 85: 2444-2448 (1988) [the algorithm is incorporated in the FASTA program in GCG software package] and the like can be mentioned, and they can be preferably used in a similar way.

More preferably, the "substantially the same amino acid sequence" is an amino acid sequence having a homology of not less than about 70%, preferably not less than about 80%, more preferably not less than about 90%, particularly preferably not less than about 95%, most preferably not less than about 98%, with an amino acid sequence shown by amino acid 46-501 in an amino acid sequence shown by SEQ ID NO: 2.

"The protein having substantially the same amino acid sequence" as an amino acid sequence shown by amino acid 46-501 in an amino acid sequence shown by SEQ ID NO: 2 refers a protein that comprises substantially the same amino acid sequence as the aforementioned amino acid sequence shown by amino acid 46-501 in an amino acid sequence shown by SEQ ID NO: 2, and that has substantially the same quality of activity as a protein that comprises an amino acid sequence shown by amino acid 46-501 in an amino acid sequence shown by SEQ ID NO: 2.

"Substantially the same quality of activity" refers (1) a β-secretase activity (that is, a protease activity that specifically cleave the isotype (APP695) consisting of 695 amino acids of APP, between Met596 and Asp597) and (2) a binding activity to a compound which binds to the transmembrane region to exert its β-secretase inhibitory activity. Substantially the same quality means that the activities thereof are qualitatively equivalent to each other. Accordingly, it is preferable that the proteins be equivalent to each other in terms of the β-secretase activity and the binding activity to an inhibiting substance, but quantitative factors such as the extent of these activities and the molecular weights of the proteins may be different (e.g., differences within the range of about 0.01 to 100 times, preferably about 0.1 to 10 times, more preferably 0.5 to 2 times, with respect to activity, can be mentioned).

Measurement of the β-secretase activity is performed with the known methods, for example, but not limited to, a method comprising reacting a synthetic peptide substrate comprising the same or substantially the same amino acid sequence of APP or a site thereof recognized by β-secretase with the β-secretase protein, and detecting one or both reaction products resulting from the cleavage, a method comprising reacting a synthetic substrate comprising a fluorescent substance and a quenching substance with the β-secretase protein as described in the above-mentioned WO 01/87293, and measuring the fluorescence resulting from the separation of the fluorescent substance and the quenching substance due to the enzymatic cleavage, or the like. On the other hand, the binding activity to the inhibiting substance can be measured with surface plasmon resonance (SPR) and the like, as shown in the following Example.

The β-secretase to be used in the present invention includes, for example, a protein having (1) an amino acid sequence shown by amino acid 46-501 in an amino acid sequence shown by SEQ ID NO: 2, wherein one or more (e.g., about 1-50, preferably about 1-30, more preferably about 1-10, more preferably about 1-5) amino acids have been deleted, (2) an amino acid sequence shown by amino acid 46-501 in an amino acid sequence shown by SEQ ID NO: 2, wherein one or more (e.g., about 1-50, preferably about 1-30, more preferably about 1-10, more preferably about 1-5) amino acids have been added, (3) an amino acid sequence shown by amino acid 46-501 in an amino acid sequence shown by SEQ ID NO: 2, wherein one or more (e.g., about 1-50, preferably about 1-30, more preferably about 1-10, more preferably about 1-5) amino acids have been inserted, (4) an amino acid sequence shown by amino acid 46-501 in an amino acid sequence shown by SEQ ID NO: 2, wherein one or more (e.g., about 1-50, preferably about 1-30, more preferably about 1-10, more preferably about 1-5) amino acids have been substituted by other amino acid(s), or (5) an amino acid sequence which is a combination thereof.

Wherein, if 1 or two or more amino acids are deleted, the site of the deletion is a site other than an amino acid sequence shown by at least amino acid 466-471 in an amino acid sequence shown by SEQ ID NO: 2, and preferably a site other than an amino acid sequence shown by amino acid 455-480. If 1 or two or more amino acids are inserted or substituted by other amino acid(s), the site of the insertion or substitution is a site other than an amino acid sequence shown by amino acid 466-471 in an amino acid sequence shown by SEQ ID NO: 2 (preferably, a site other than an amino acid sequence shown by amino acid 455-480), or even if such a site, the site has to be a site wherein the result of the insertion or substitution has no qualitative effect on the activity of the site (that is, the binding activity to a β-secretase inhibiting substance which binds to the transmembrane region).

The β-secretase is a single transmembrane aspartic protease and comprises an active center within the extracellular (or lumenal) region. More specifically, for example, in the human β-secretase shown by SEQ ID NO: 2, an amino acid sequence shown by amino acid 46-501 is an amino acid sequence of a mature β-secretase protein (β-secretase is produced as a preproenzyme, and a signal peptide consisting of an amino acid sequence shown by amino acid 1-21 and a propeptide consisting of an amino acid sequence shown by amino acid 22-45 are cleaved therefrom during the secretion process), for example, although the position is somewhat different among literatures, the extracellular (lumenal) region, the transmembrane region and the cytoplasmic region are consisting of amino acid sequences shown by amino acids 45-454, 455-480 and 481-501, respectively.

The consensus motifs (D-T/S-G-T/S) of the active center in the extracellular (lumenal) region are amino acid 93-96 (DTGS) and amino acid 289-292 (DSGT). Therefore, "a region comprising an active center" of β-secretase includes, for example, a region comprising the same or substantially the same amino acid sequence as an amino acid sequence shown by amino acid 93-292 in an amino acid sequence shown by SEQ ID NO: 2. Wherein, "substantially the same amino acid sequence" is as defined above, "a region comprising substantially the same amino acid sequence" is a region comprising substantially the same amino acid sequence and having the β-secretase activity. The region comprising the active center of β-secretase may be a whole extracellular (lumenal) region thereof (that is, the same or substantially the same amino acid sequence as an amino acid sequence shown by amino acid 46-454 in an amino acid sequence shown by SEQ ID NO: 2), or may be a sequence further comprising a prosequence or preprosequence at its N-terminal (that is, the same or substantially the same amino acid sequence as an amino acid sequence shown by amino acid 22-454 or 1-454 in an amino acid sequence shown by SEQ ID NO: 2).

"A part of a transmembrane region" of β-secretase is not particularly limited, as long as it is the same or substantially the same amino acid sequence as any partial sequence of an amino acid sequence shown by amino acid 455-480 in an amino acid sequence shown by SEQ ID NO: 2 (e.g., one consisting of continuous about 3-20 amino acids, preferably continuous about 5-10 amino acids), and preferably includes the same or substantially the same amino acid sequence as an amino acid sequence shown by amino acid 466-471. Wherein, "substantially the same amino acid sequence" refers an amino acid sequence with one to several amino acids substituted, deleted, inserted or added in any partial sequence of an amino acid sequence shown by amino acid 455-480 in an amino acid sequence shown by SEQ ID NO: 2, and wherein the binding ability of the partial sequence to a compound having β-secretase inhibitory activity is not changed at least qualitatively. Such amino acid mutations can be achieved by the aforementioned conservative amino acid substitution and the like. Particularly, it is believed that the substitution by an amino acid similar in the hydrophobicity is preferable without limitation.

A β-secretase to be used in a screening method of the present invention is not particularly limited as long as it includes the above-mentioned "region comprising an active center" at its N-terminal and "a part or all of a transmembrane region" at its C-terminal. For example, it may or may not comprise a sequence of the cytoplasmic region at C-terminal of a part or all of the transmembrane region.

With respect to the proteins and peptide mentioned herein, the left end is the N-terminal (amino terminal) and the right end is the C-terminal (carboxyl terminal) in accordance with the conventional peptide marking. For a transmembrane enzyme used in a screening method of the present invention, the C-terminal may be any of a carboxyl group (-COOH), a carboxylate (-COO⁻), an amide (-CONH₂), and an ester (-COOR).

Here, as R in the ester, a C₁₋₆ alkyl group such as methyl, ethyl, n-propyl, isopropyl and n-butyl; a C₃₋₈ cycloalkyl group such as cyclopentyl and cyclohexyl; a C₆₋₁₂ aryl group such as phenyl and α-naphthyl; a phenyl-C₁₋₂ alkyl group such as benzyl and phenethyl; a C₇₋₁₄ aralkyl group such as an _{α}-naphthyl-C₁₋₂ alkyl group such as α-naphthylmethyl; a pivaloyloxymethyl group; and the like can be used.

When the transmembrane enzyme has a carboxyl group (or a carboxylate) in addition to that on the C-terminal, one in which the carboxyl group is amidated or esterified is also included in the transmembrane enzyme of the present invention. In this case, as the ester, the above-described C-terminal ester and the like, for example, can be used.

Furthermore, the transmembrane enzyme also includes a protein wherein the amino group of the N-terminal amino acid residue thereof (e.g., methionine residue) is protected by a protecting group (e.g., a C₁₋₆ acyl group such as a C₁₋₆ alkanoyl group such as a formyl group or an acetyl group, and the like), a protein wherein the N-terminal glutamine residue, which is produced by cleavage *in vivo*, has been converted to pyroglutamic acid, a protein wherein a substituent (e.g., -OH, -SH, an amino group, an imidazole group, an indole group, a guadinino group and the like) on an amino acid side chain in the molecule is protected by an appropriate protecting group (e.g., a C₁₋₆ acyl group such as a C₁₋₆ alkanoyl group such as a formyl group or an acetyl group, and the like), a conjugated protein such as what is called a glycoprotein, which has a sugar chain bound thereto, and the like.

The partial peptide of the transmembrane enzyme to be used in the present invention is a peptide which has the above-mentioned partial amino acid sequence of the transmembrane enzyme (that is, the sequence of the region comprising an active center and the sequence of a part or all of the transmembrane region) and substantially the same quality of activity as the transmembrane enzyme. As used herein, the "substantially the same quality of activity" is as defined above. In addition, "substantially the same quality of activity" can be measured in the same manner as mentioned above. In the present specification, the partial peptide is hereinafter to be referred to as "an inhibiting substance binding peptide".

The inhibiting substance binding peptide has the above-mentioned properties, and for example, in the case of β-secretase, includes one lacking a partial amino acid sequence having no effect on the enzyme activity within the extracellular (lumenal) region, one lacking a partial amino acid sequence other than a target binding site within the transmembrane region, one lacking a part or all of the cytoplasmic region and the like.

On the other hand, a partial peptide of a transmembrane enzyme (1) retaining a region comprising an active center, and (2) lacking a binding site targeted by a subject enzyme inhibiting substance within a transmembrane region, retains the enzyme activity, however, has no binding activity to the subject enzyme inhibiting substance, such that when contacted with a compound exerting its enzyme inhibitory activity by specifically binding to the target binding site, it does not bind to the compound and the enzyme activity is not inhibited. In the present description, hereinafter, the partial peptide is called as "an inhibiting substance non-binding peptide".

With respect to the partial peptide of transmembrane enzyme (encompassing both an inhibiting substance binding peptide and an inhibiting substance non-binding peptide; hereinafter, such case is to be also referred to as "the partial peptide of the present invention"), the C-terminal may be any of a carboxyl group (-COOH), a carboxylate (-COO⁻), an amide (-CONH₂), and an ester (-COOR). Here, as R in the ester, the same as those mentioned for the transmembrane enzyme can be mentioned. When these peptides have a carboxyl group (or a carboxylate) in addition to that on the C-terminal, one in which the carboxyl group is amidated or esterified is also included in the partial peptide of the present invention. In this case, as the ester, the above-described C-terminal ester and the like, for example, can be used.

Furthermore, the partial peptide of the present invention also includes a peptide wherein the amino group of the N-terminal methionine residue is protected by a protecting group, a peptide wherein Gln, which is produced by cleavage on the N-terminal side *in vivo*, has been converted to pyroglutamic acid, a peptide wherein a substituent on an amino acid side chain in the molecule is protected by an appropriate protecting group, a conjugated peptide such as what is called a glycopeptide, which has a sugar chain bound thereto, and the like, as with the above-described transmembrane enzyme.

As the salt of the transmembrane enzyme or a partial peptide thereof, a physiologically acceptable salt with an acid or a base can be mentioned, with preference given to a physiologically acceptable acid addition salt. Useful salts include, for example, salts with inorganic acids (e.g., hydrochloric acid, phosphoric acid, hydrobromic acid, sulfuric acid) or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The transmembrane enzyme or salt thereof can be produced from cells or a tissue of the aforementioned warm-blooded animal by a method of protein purification known *per se*, as above mentioned in β-secretase as the example. Specifically, the transmembrane enzyme or salt thereof can be produced by homogenizing a tissue or cells of a warm-blooded animal, and separating and purifying the soluble fraction by a chromatography such as reversed-phase chromatography, ion exchange chromatography or affinity chromatography, and the like.

The transmembrane enzyme or a partial peptide thereof can also be produced according to a publicly known peptide synthesis process.

The peptide synthesis process may be any of, for example, a solid phase synthesis process and a liquid phase synthesis process. A desired protein can be produced by condensing a partial peptide or amino acids capable of constituting a transmembrane enzyme with the remaining portion, and removing the protecting group if any in the resultant product.

Here, the condensation and the removal of the protecting group are conducted according to methods known *per se,* for example, methods described in (1) to (5) below.
(1) M. Bodanszky and M.A. Ondetti: Peptide Synthesis, Interscience Publishers, New York (1966)
(2) Schroeder and Luebke: The Peptide, Academic Press, New York (1965)
(3) Nobuo Izumiya, et al.: Peptide Gosei-no-Kiso to Jikken, published by Maruzen Co. (1975);
(4) Haruaki Yajima and Shunpei Sakakibara: Seikagaku Jikken Koza 1, Tanpakushitsu no Kagaku IV, 205 (1977)
(5) Haruaki Yajima, ed.: Zoku Iyakuhin no Kaihatsu, Vol. 14, Peptide Synthesis, published by Hirokawa Shoten.

The transmembrane enzyme or a partial peptide thereof thus obtained can be isolated and purified by a publicly known method of purification. Here, as examples of the method of purification, solvent extraction, distillation, column chromatography, liquid chromatography, recrystallization, a combination thereof, and the like can be mentioned.

When the transmembrane enzyme or a partial peptide thereof obtained by the above-described method is a free form, the free form can be converted to an appropriate salt by a publicly known method or a method based thereon; conversely, when the transmembrane enzyme or a partial peptide thereof is obtained in the form of a salt, the salt can be converted to a free form or another salt by a publicly known method or a method based thereon.

For the synthesis of the transmembrane enzyme or a partial peptide thereof, an ordinary commercially available resin for protein synthesis can be used. As examples of such resins, chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenylacetamidomethyl resin, polyacrylamide resin, 4-(2',4'-dimethoxyphenyl-hydroxymethyl)phenoxy resin, 4-(2',4'-dimethoxyphenyl-Fmoc-aminoethyl)phenoxy resin and the like can be mentioned. Using such a resin, an amino acid having an appropriately protected α-amino group and side chain functional group is condensed on the resin in accordance with the sequence of the desired protein or peptide according to various methods of condensation known *per se*. At the end of the reaction, the protein (peptide) is cleaved from the resin, various protecting groups are removed simultaneously, and a reaction to form an intramolecular disulfide bond is carried out in a highly diluted solution to obtain the desired protein (peptide) or an amide thereof.

For the above-described condensation of protected amino acids, various activation reagents useful for protein synthesis can be used, with preference given to a carbodiimide. As the carbodiimide, DCC, N,N'-diisopropylcarbodiimide, N-ethyl-N'-(3-dimethylaminoprolyl) carbodiimide and the like can be used. For the activation using these carbodiimides, the protected amino acid, along with a racemation-suppressing additive (e.g., HOBt, HOOBt), may be added directly to the resin, or the protected amino acid may be activated in advance as a symmetric acid anhydride, or HOBt ester or HOOBt ester and then added to the resin.

A solvent used for activation of protected amino acids and condensation of protected amino acids with a resin can be appropriately selected from among solvents that are known to be usable for protein condensation reactions. Examples of such useful solvents include acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethyl sulfoxide; amines such as pyridine; ethers such as dioxane and tetrahydrofuran; nitriles such as acetonitrile and propionitrile; esters such as methyl acetate and ethyl acetate; suitable mixtures thereof; and the like. Reaction temperature is appropriately selected from the range that is known to be usable in protein binding reactions, and is normally from the range of about -20°C to about 50°C. An activated amino acid derivative is normally used from 1.5 to 4 times in excess. When the condensation is insufficient as the result of the test using a ninhydrin reaction, sufficient condensation can be carried out by repeating the condensation reaction without elimination of the protecting group. If the condensation is insufficient even though the condensation reaction is repeated, unreacted amino acids can be acetylated by using acetic anhydride or acetylimidazole.

A protecting method and a protecting group of a functional group that should not been involved in the reaction of raw materials, a method of removing the protecting group, a method of activating a functional group involved in the reaction, and the like can be appropriately selected from among publicly known groups or publicly known means.

As the protecting group for the amino group of the starting material, for example, Z, Boc, tertiary pentyloxycarbonyl, isobornyloxycarbonyl, 4-methoxybenzyloxycarbonyl, C1-Z, Br-Z, adamantyloxycarbonyl, trifluoroacetyl, phthaloyl, formyl, 2-nitrophenylsulfenyl, diphenylphosphinothioyl, Fmoc and the like can be used.

The carboxyl group can be protected by, for example, alkyl esterification (e.g., linear, branched or cyclic alkyl esterification with methyl, ethyl, propyl, butyl, tertiary butyl, cyclopentyl, cyclohexyl, cycloheptyl, cyclooctyl, 2-adamantyl, and the like), aralkyl esterification (e.g., benzyl esterification, 4-nitrobenzyl esterification, 4-methoxybenzyl esterification, 4-chlorobenzyl esterification, benzhydryl esterification), phenacyl esterification, benzyloxycarbonyl hydrazidation, tertiary butoxycarbonyl hydrazidation, trityl hydrazidation, and the like.

The hydroxyl group of serine can be protected by, for example, esterification or etherification. As the group suitable for this esterification, for example, lower alkanoyl groups such as an acetyl group, aroyl groups such as a benzoyl group, and groups derived from carbonic acid such as benzyloxycarbonyl group, ethoxycarbonyl group and the like can be used. In addition, as examples of the group suitable for etherification, for example, a benzyl group, a tetrahydropyranyl group, a t-butyl group and the like can be mentioned.

As the protecting group for the phenolic hydroxyl group of tyrosine, for example, Bzl, Cl₂-Bzl, 2-nitrobenzyl, Br-Z, tertiary butyl and the like can be used.

As the protecting group for the imidazole of histidine, for example, Tos, 4-methoxy-2,3,6-trimethylbenzenesulfonyl, DNP, benzyloxymethyl, Bum, Boc, Trt, Fmoc and the like can be used.

As the method of removing (eliminating) a protecting group, catalytic reduction in a hydrogen stream in the presence of a catalyst such as Pd-black or Pd-carbon; acid treatment by means of anhydrous hydrogen fluoride, methanesulfonic acid, trifluoromethane-sulfonic acid, trifluoroacetic acid, or a mixture solution thereof; base treatment by means of diisopropylethylamine, triethylamine, piperidine, piperazine or the like; and reduction with sodium in liquid ammonia, and the like, for example, can be used. The elimination reaction by the above-described acid treatment is generally carried out at a temperature of about -20°C to about 40°C; the acid treatment is efficiently conducted by adding a cation scavenger such as, for example, anisole, phenol, thioanisole, m-cresol, p-cresol, dimethyl sulfide, 1,4-butanedithiol or 1,2-ethanedithiol. Also, a 2,4-dinitrophenyl group used as a protecting group for the imidazole of histidine is removed by thiophenol treatment; a formyl group used as a protecting group for the indole of tryptophan is removed by acid treatment in the presence of 1,2-ethanedithiol, 1,4-butanedithiol, or the like, as well as by alkali treatment with a dilute sodium hydroxide solution, dilute ammonia, or the like.

As the raw material having an activated carboxyl group, for example, a corresponding acid anhydride, an azide, an activated ester [an ester with an alcohol (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, or HOBt)] and the like can be used. As the raw material having an activated amino group, for example, a corresponding phosphoric amide can be used.

As another method for obtaining an amide of a protein (peptide), for example, a method comprising protecting the α-carboxyl group of each C-terminal amino acid of partial peptide constituting a protein (peptide) by amidation, extending peptide chain to the amino group side in a desired chain length (amino acid to be joined with C-terminal amino acid of adjacent partial peptide). Producing a peptide only without α-amino-protecting group of N-terminal amino acid of C-terminal side peptide chain, and a peptide only without carboxyl group-protecting group of C-terminal amino acid of N-terminal side peptide chain, and condensing these peptides in the above-mentioned mixed solvent can be mentioned. The details of the condensation reaction are as mentioned above. After purification of the protected protein (protected peptide) obtained by condensation, the protecting group is eliminated by the above-mentioned method to give a desired crude protein (crude peptide). The crude protein (crude peptide) is purified by various known purification means, and the main fraction is lyophilized to give an amide of the desired protein (peptide).

An ester of the protein (peptide) can be obtained, for example, by condensing the α-carboxyl group of C-terminal amino acid with a desired alcohol to give an amino acid ester, and treating the ester in the same manner as in the above-mentioned amide.

The partial peptide of the present invention or a salt thereof can also be produced by cleaving the transmembrane enzyme or a salt thereof with an appropriate peptidase.

Moreover, a transmembrane enzyme or a partial peptide thereof can also be produced by culturing a transformant having the nucleic acid encoding same, and separating and purifying a transmembrane enzyme or a partial peptide thereof from the obtained culture.

The nucleic acid encoding a transmembrane enzyme or a partial peptide thereof may be a DNA or an RNA, or DNA/RNA chimera. DNA is preferable. The nucleic acid may be a double stranded or a single strand. In the case of a double stranded, it may be a double stranded DNA, a double stranded RNA or a DNA:RNA hybrid. In the case of a single strand, it may be a sense strand (i.e., coding strand) or an antisense chain (i.e., non-coding strand).

As the DNA encoding a transmembrane enzyme or a partial peptide thereof, genomic DNA, cDNA derived from any cell [for example, splenocyte, nerve cell, glial cell, pancreatic β cells, myeloid cell, mesangial cell, Langerhans' cell, epidermal cell, epithelial cell, endothelial cell, fibroblast, fibrocyte, myocytes, adipocyte, immune cell (e.g., macrophage, T cell, B cell, natural killer cell, mast cell, neutrophil, basophil, eosinophils, monocyte), megakaryocyte, synovial cell, chondrocytes, bone cell, osteoblast, osteoclast, mammary cell, hepatocyte or interstitial cell, or a corresponding precursor cell, stem cell, cancer cell and the like, blood cells] of warm-blooded animal (e.g., human, mouse, rat, guinea pig, hamster, rabbit, sheep, goat, swine, bovine, horse, bird, cat, dog, monkey, chimpanzee and the like), or any tissue where such cells are present [e.g., brain or any portion of brain (e.g., olfactory bulb, amygdaloid nucleus, basal ganglia, hippocampus, thalamus, hypothalamus, subthalamic nucleus, cerebral cortex, medulla oblongata, cerebellum, occipital lobe, frontal lobe, lateral lobe, putamen, caudate nucleus, callosum, substantia nigra), spinal cord, hypophysis, stomach, pancreas, kidney, liver, gonad, thyroid, gallbladder, bone marrow, adrenal gland, skin, muscle, lung, gastrointestinal tract (e.g., large intestine, small intestine), blood vessel, heart, thymus, spleen, submandibular gland, peripheral blood, peripheral blood cell, prostate, testicle, ovary, placenta, uterus, bone, joint, adipose tissue and the like], synthetic DNA and the like can be mentioned. A genomic DNA and cDNA encoding transmembrane enzyme or a partial peptide thereof can also be directly amplified by Polymerase Chain Reaction (hereinafter to be abbreviated as "PCR method") or Reverse Transcriptase-PCR (hereinafter to be abbreviated as "RT-PCR method"), using genomic DNA fraction or total RNA or mRNA fraction prepared from the above-mentioned cell/tissue as a template. Alternatively, genomic DNA or cDNA encoding transmembrane enzyme or a partial peptide thereof can also be cloned by colony or plaque hybridization method, PCR method and the like, from the genomic DNA library or cDNA library prepared by inserting, into a suitable vector, a fragment of genomic DNA and total RNA or mRNA prepared from the above-mentioned cell/tissue. The vector to be used for the library may be any of bacteriophage, plasmid, cosmid, phagemid and the like.

An example of the DNA that encodes the β-secretase includes, a DNA that has a base sequence shown by base 136-1503 in a base sequence shown by SEQ ID NO: 1, a DNA that has a base sequence hybridizing to a complementary strand sequence of a base sequence shown by base 136-1503 in a base sequence shown by SEQ ID NO: 1 under high stringent conditions and encodes a protein having substantially the same quality of activity as the aforementioned protein comprising an amino acid sequence shown by amino acid 46-501 in an amino acid sequence shown by SEQ ID NO: 2 (that is, the β-secretase activity and the binding activity to a β-secretase inhibiting substance at the target binding site etc.) or the like.

As the DNA capable of hybridizing to a complementary strand sequence of a base sequence shown by base 136-1503 in a base sequence shown by SEQ ID NO: 1 under high stringent conditions, for example, a DNA that has a base sequence having a homology of about 60% or more, preferably about 70% or more, more preferably about 80% or more, and particularly preferably about 90% or more, with a base sequence shown by base 136-1503 in a base sequence shown by SEQ ID NO: 1, and the like, can be used.

The homology of the base sequence in the present specification can be calculated using the homology calculation algorithm NCBI BLAST (National Center for Biotechnology Information Basic Local Alignment Search Tool) and under the following conditions (expectancy =10; allowing gap; filtering=ON; match score=1; mismatch score=-3). As other algorithm with which to determine the homology of the base sequence, the homology calculation algorithm of the above-mentioned amino acid sequence can be preferably used in the same manner.

Hybridization can be conducted according to a method known *per se* or a method based thereon, for example, a method described in Molecular Cloning, 2nd edition (J. Sambrook *et al*., Cold Spring Harbor Lab. Press, 1989) and the like. When a commercially available library is used, hybridization can be conducted according to the method described in the instruction manual attached to the library. Hybridization can preferably be conducted under high stringent conditions.

As the high stringent conditions, for example, the conditions of sodium salt concentration of about 19 - about 40 mM, preferably about 19 - about 20 mM, a temperature of about 50°C - about 70°C, preferably about 60°C - about 65°C, and the like can be mentioned. Particularly, a sodium salt concentration of about 19 mM and a temperature of about 65°C are preferable. Those of ordinary skill in the art can easily adjust to desired stringency by appropriately changing the salt concentration of hybridization solution, hybridization reaction temperature, probe concentration, probe length, number of mismatch, hybridization reaction time, salt concentration of washing, washing temperature and the like.

The DNA encoding β-secretase is preferably human β-secretase DNA having the base sequence shown by SEQ ID NO: 1, or its allele variant, or ortholog of other warm-blooded animal (e.g., mouse, rat, guinea pig, hamster, rabbit, sheep, goat, swine, bovine, horse, bird, cat, dog, monkey, chimpanzee and the like).

A DNA encoding an inhibiting substance binding peptide may be any DNA comprising a base sequence encoding a region comprising an active center and a base sequence encoding a part or all of a transmembrane region of a transmembrane enzyme. Also, a DNA encoding an inhibiting substance non-binding peptide may be any DNA comprising a base sequence encoding a region comprising an active center and lacking a base sequence encoding the above-mentioned "a part or all of a transmembrane region" of the transmembrane enzyme. The DNA may be any of a genomic DNA, a cDNA derived from the above-described cell or tissue, and a synthetic DNA.

Specifically, if the transmembrane enzyme is a β-secretase, as the DNA that encodes the inhibiting substance binding peptide, the following can be used, for example:
(1a) a DNA having a base sequence shown by base 277-876 and a part or all of a base sequence shown by base 1363-1440 in a base sequence shown by SEQ ID NO: 1, or
(2a) a DNA having a base sequence hybridizing to the above DNA of (1a) under high stringent conditions, and encoding a peptide having substantially the same quality of activity as a peptide comprising an amino acid sequence shown by amino acid 46-501 in an amino acid sequence shown by SEQ ID NO: 2 (that is, the β-secretase activity and the binding activity to the β-secretase inhibiting substance at the target binding site within the transmembrane region).

As the DNA that encodes an inhibiting substance non-binding peptide, the following is used:
(1b) a DNA having a base sequence shown by base 277-876 and lacking a part or all of a base sequence shown by base 1363-1440 in a base sequence shown by SEQ ID NO: 1 which is possessed by the above DNA of (1a), or
(2b) a DNA having a base sequence hybridizing to the above DNA of (1b) under high stringent conditions, and encoding a peptide having substantially the same quality of activity as a peptide comprising an amino acid sequence shown by amino acid 46-454 in an amino acid sequence shown by SEQ ID NO: 2 (that is, the β-secretase activity) but not the binding activity to the enzyme inhibiting substance at the target binding site within the transmembrane region.

As the DNA capable of hybridizing to the above-mentioned DNA of (1a) or (1b) under high stringent conditions, a DNA that has a base sequence having a homology of about 60% or more, preferably about 70% or more, more preferably about 80% or more, and particularly preferably about 90% or more, to the corresponding portion in the base sequence, and the like, for example, can be used.

The DNA that encodes the transmembrane enzyme or a partial peptide thereof can be cloned by amplifying it by the PCR method using a synthetic DNA primer having a portion of the base sequence that encodes the protein or peptide, or by hybridizing DNA incorporated in an appropriate expression vector to a labeled DNA fragment or synthetic DNA that encodes a portion or the entire region of the protein of the present invention. Hybridization can be conducted according to, for example, a method described in Molecular Cloning, 2nd edition (ibidem) and the like. When a commercially available library is used, hybridization can be conducted according to the method described in the instruction manual attached to the library.

The base sequence of DNA can be converted according to a method known *per se*, such as the ODA-LA PCR method, the Gapped duplex method, the Kunkel method and the like, or a method based thereon, using a publicly known kit, for example, Mutant^{™}-super Express Km (Takara Shuzo Co., Ltd.), Mutan^{™}-K (Takara Shuzo Co., Ltd.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added using an appropriate synthetic DNA adapter.

The protein or peptide can be produced by transforming a host with an expression vector containing a DNA encoding the above-mentioned transmembrane enzyme or a partial peptide thereof and cultivating the obtained transformant.

An expression vector containing a DNA encoding transmembrane enzyme or a partial peptide thereof can be produced, for example, by cleaving out an object DNA fragment from the DNA encoding transmembrane enzyme and connecting the DNA fragment with the downstream of a promoter in a suitable expression vector.

Useful expression vectors include plasmids derived from E. coli (e.g., pBR322, pBR325, pUC12, pUC13); plasmids derived from *Bacillus* subtilis (e.g., pUB110, pTP5, pC194); plasmids derived from yeast (e.g., pSH19, pSH15); bacteriophages such as λ phage; animal viruses such as retrovirus, vaccinia virus and baculovirus; pA1-11, pXT1, pRc/CMV, pRc/RSV, pcDNAI/Neo and the like.

The promoter may be any promoter, as long as it is appropriate for the host used to express the gene.

For example, when the host is an animal cell, the SR_{α} promoter, the SV40 promoter, the LTR promoter, the CMV (cytomegalovirus) promoter, the HSV-TK promoter and the like are used. Of these, the CMV promoter, the SR_{α} promoter and the like are preferred.

When the host is a bacterium of the genus *Escherichia,* the trp promoter, the lac promoter, the recA promoter, the λP_{L} promoter, the lpp promoter, the T7 promoter and the like are preferred.

When the host is a bacterium of the genus *Bacillus,* the SP01 promoter, the SP02 promoter, the penP promoter and the like are preferred.

When the host is yeast, the PH05 promoter, the PGK promoter, the GAP promoter, the ADH promoter and the like are preferred.

When the host is an insect cell, the polyhedrin promoter, the P10 promoter and the like are preferred.

Useful expression vectors include, in addition to the above, expression vectors that optionally comprises an enhancer, a splicing signal, a polyA addition signal, a selection marker, an SV40 replication origin (hereinafter also abbreviated as SV40ori), and the like. As examples of the selection markers, the dihydrofolate reductase (hereinafter also abbreviated as dhfr) gene [methotrexate (MTX) resistance], the ampicillin resistance gene (hereinafter also abbreviated as Amp^{r}), the neomycin resistance gene (hereinafter also abbreviated as Neo^{r}, G418 resistance), and the like can be mentioned. In particular, when a dhfr gene defective Chinese hamster cell is used and the dhfr gene is used as the selection marker, a target gene can also be selected using a thymidine-free medium.

Where necessary, a base sequence encoding (signal codon) a signal sequence suitable for the host may be added to the 5' end side of DNA encoding a transmembrane enzyme or a partial peptide thereof. When the host is the genus *Escherichia,* PhoA signal sequence, OmpA signal sequence and the like are used; when the host is the genus *Bacillus,* α-amylase signal sequence, subtilisin signal sequence and the like are used; when the host is yeast, MFα signal sequence, SUC2 signal sequence and the like are used; and when the host is an animal cell, insulin signal sequence, α-interferon signal sequence, antibody molecule signal sequence and the like are used.

As useful examples of the host, a bacterium of the genus *Escherichia,* a bacterium of the genus *Bacillus,* yeast, an insect cell, an insect, an animal cell, and the like can be mentioned.

As useful examples of the bacterium of the genus *Escherichia, Escherichia* coli K12 DH1 (Proc. Natl. Acad. Sci. U.S.A., Vol. 60, 160 (1968)), JM103 (Nucleic Acids Research, Vol. 9, 309 (1981)), JA221 (Journal of Molecular Biology, Vol. 120, 517 (1978)), HB101 (Journal of Molecular Biology, Vol. 41, 459 (1969)), C600 (Genetics, Vol. 39, 440 (1954)), and the like can be mentioned.

As useful examples of the bacterium of the genus *Bacillus, Bacillus* subtilis MI114 (Gene, Vol. 24, 255 (1983)), 207-21 (Journal of Biochemistry, Vol. 95, 87 (1984)) and the like can be mentioned.

As useful examples of the yeast, *Saccharomyces cerevisiae* AH22, AH22R⁻, NA87-11A, DKD-5D and 20B-12, *Schizosaccharomyces pombe* NCYC1913, and NCYC2036, *Pichia pastoris* KM71 and the like can be mentioned.

As useful examples of the insect cell, *Spodoptera frugiperda* cell (Sf cell), MG1 cell derived from the mid-intestine of *Trichoplusia ni*, High Five^{™} cell derived from an egg of *Trichoplusia ni,* cell derived from *Mamestra brassicae,* cell derived from *Estigmena acrea,* and the like can be mentioned when the virus is AcNPV. When the virus is BmNPV, useful insect cells include *Bombyx mori* N cell (BmN cell) and the like. As useful examples of the Sf cell, Sf9 cell (ATCC CRL1711), Sf21 cell (both in Vaughn, J. L. et al., In Vivo, 13, 213-217 (1977), and the like can be mentioned.

As useful examples of the insect, a larva of Bombyx mori (Maeda et al., Nature, 315, 592 (1985)), and the like can be mentioned.

As useful examples of the animal cell, monkey cell COS-7, Vero, Chinese hamster cell CHO (hereafter abbreviated as CHO cell), dhfr gene defective Chinese hamster cell CHO (hereafter abbreviated as CHO(dhfr⁻) cell), mouse L cell, mouse AtT-20, mouse myeloma cell, rat GH3, human FL cell, HEK293 cell, HeLa cell and the like can be mentioned.

Transformation can be carried out according to the kind of host in accordance with a publicly known method.

A bacterium of the genus *Escherichia* can be transformed, for example, in accordance with a method described in Proc. Natl. Acad. Sci. U.S.A., 69, 2110 (1972), Gene, 17, 107 (1982), and the like.

A bacterium of the genus *Bacillus* can be transformed, for example, according to a method described in Molecular and General Genetics, 168, 111 (1979), and the like.

Yeast can be transformed, for example, in accordance with a method described in Methods in Enzymology, 194, 182-187 (1991), Proc. Natl. Acad. Sci. USA, 75, 1929 (1978), and the like.

An insect cell and an insect can be transformed, for example, according to a method described in Bio/Technology, 6, 47-55 (1988), and the like.

An animal cell can be transformed, for example, in accordance with a method described in Saibo Kogaku, extra issue 8, Shin Saibo Kogaku Jikken Protocol, 263-267 (1995), published by Shujunsha, or Virology, 52, 456 (1973).

Cultivation of a transformant can be carried out according to the kind of host in accordance with a publicly known method.

For example, when a transformant whose host is a bacterium of the genus *Escherichia* or the genus *Bacillus* is cultivated, the culture medium is preferably a liquid medium. Also, the medium preferably comprises a carbon source, a nitrogen source, an inorganic substance, and the like, necessary for the growth of the transformant. Here, as examples of the carbon source, glucose, dextrin, soluble starch, sucrose, and the like can be mentioned; as examples of the nitrogen source, inorganic and organic substances such as an ammonium salt, a nitrate salt, corn steep liquor, peptone, casein, meat extract, soybean cake, potato extract, and the like can be mentioned; as examples of the inorganic substance, calcium chloride, sodium dihydrogen phosphate, magnesium chloride, and the like can be mentioned. In addition, the medium may be supplemented with yeast extract, vitamins, growth promoting factor, and the like. Preferably, the pH of the medium is about 5 to 8.

Examples of the medium used to cultivate a transformant whose host is a bacterium of the genus *Escherichia* include a M9 medium supplemented with glucose and a Casamino acid (Miller, Journal of Experiments in Molecular Genetics, 431-433, Cold Spring Harbor Laboratory, New York, 1972). As required, in order to increase promoter efficiency, a chemical such as 3β-indolylacrylic acid may be added to the medium.

Cultivation of a transformant whose host is a bacterium of the genus *Escherichia* is normally carried out at about 15°C to 43°C for about 3 to 24 hours. As necessary, the culture may be aerated or agitated.

Cultivation of a transformant whose host is a bacterium of the genus *Bacillus* is normally carried out at about 30°C to 40°C for about 6 to 24 hours. As necessary, the culture may be aerated or agitated.

As examples of the medium for cultivating a transformant whose host is yeast, Burkholder's minimum medium [Bostian, K.L. et al., Proc. Natl. Acad. Sci. USA, 77, 4505 (1980)] and SD medium supplemented with 0.5% casamino acid [Bitter, G.A. et al., Proc. Natl. Acad. Sci. USA, 81, 5330 (1984)] can be mentioned. The medium's pH is preferably about 5 to 8. Cultivation is normally carried out at about 20°C to 35°C for about 24 to 72 hours. As necessary, the culture may be aerated or agitated.

Useful medium for cultivating a transformant whose host is an insect cell or an insect include, for example, Grace's Insect Medium [Grace, T.C.C., Nature, 195, 788 (1962)] supplemented with additives such as inactivated 10% bovine serum as appropriate. The medium's pH is preferably about 6.2 to 6.4. Cultivation is normally carried out at about 27°C for about 3 to 5 days. As necessary, the culture may be aerated or agitated.

Useful medium for cultivating a transformant whose host is an animal cell include, for example, MEM medium supplemented with about 5 to 20% fetal bovine serum [Science, 122, 501(1952)], DMEM medium [Virology, 8, 396(1959)], RPMI 1640 medium [The Journal of the American Medical Association, 199, 519(1967)], 199 medium [Proceeding of the Society for the Biological Medicine, 73, 1(1950)] and the like. The medium's pH is preferably about 6 to 8. Cultivation is normally carried out at about 30°C to 40°C for about 15 to 60 hours. As necessary, the culture may be aerated or agitated.

As described above, the transmembrane enzyme or a partial peptide thereof can be produced in or outside the cells of the transformant.

The transmembrane enzyme or a partial peptide thereof can be separated and purified from the culture obtained by cultivating the aforementioned transformant according to a method known *per se.*

For example, when the transmembrane enzyme or a partial peptide thereof is extracted from cultivated bacteria or cells, a method is used as appropriate wherein the bacteria or cells are recovered from the culture by a known means, suspended in an appropriate buffer solution, and disrupted by means of sonication, lysozyme and/or freeze-thawing and the like, after which a crude extract of soluble protein is obtained by centrifugation or filtration. The buffer solution may contain a protein denaturant such as urea or guanidine hydrochloride and a surfactant such as Triton X-100^{™}.

Isolation and purification of the transmembrane enzyme or a partial peptide thereof contained in the thus-obtained soluble fraction can be conducted according to a method know *per se*. Useful methods include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing. These methods can be combined as appropriate.

When the thus-obtained transmembrane enzyme or a partial peptide thereof is a free form, the free form can be converted to a salt by a method known *per se* or a method based thereon; when the protein or the peptide is obtained as a salt, the salt can be converted to a free form or another salt by a method known *per se* or a method based thereon.

Note that the transmembrane enzyme or a partial peptide thereof produced by the transformant can also be optionally modified by the action of an appropriate protein-modifying enzyme, before or after purification, or can have a polypeptide thereof removed partially. As such, useful protein-modifying enzymes include, for example, trypsin, chymotrypsin, arginyl endopeptidase, protein kinase, glycosidase and the like.

The presence of the thus-obtained transmembrane enzyme or a partial peptide thereof can be confirmed by enzyme immunoassay, Western blotting and the like employing a specific antibody.

Furthermore, the transmembrane enzyme or a partial peptide thereof can also be synthesized by in vitro translation using a cell-free protein translation system that comprises a rabbit reticulocyte lysate, wheat germ lysate, *Escherichia coli* lysate and the like, with RNA corresponding to the above-described DNA that encodes transmembrane enzyme or a partial peptide thereof as the template. Alternatively, the transmembrane enzyme or a partial peptide thereof can be synthesized using a cell-free transcription/translation system containing RNA polymerase, with the DNA that encodes the transmembrane enzyme or a partial peptide thereof as the template. As the cell-free protein (transcription/) translation system, commercially available ones can be used, a method known *per se*, and specifically, an *Escherichia* coli extract can also be prepared by the method described in Pratt J. M. et al., Transcription and Tranlation, 179-209, Hames B. D. & Higgins S. J. eds., IRL Press, Oxford (1984) and the like. As the commercially available cell lysates, as those derived from *Escherichia* coli, *E. coli* S30 extract system (manufactured by Promega), RTS 500 Rapid Translation System (manufactured by Roche) and the like can be mentioned, as those derived from rabbit reticulocyte, Rabbit Reticulocyte Lysate System (manufactured by Promega) and the like can be mentioned, and as those derived from wheat germ, PROTEIOS^{™} (manufactured by TOYOBO) and the like can be mentioned. Of these, ones using a wheat germ lysate are preferable. As the production method of wheat germ lysate, for example, the methods described in Johnston F.B. et al., Nature, 179: 160-161 (1957) or Erickson A.H. et al., Meth. Enzymol., 96: 38-50 (1996) and the like can be used.

As the system or apparatus for protein synthesis, batch method (Pratt, J.M. *et al.* (1984), mentioned above), continuous cell-free protein synthesis system (Spirin A.S. et al., Science, 242: 1162-1164 (1988)) wherein amino acid, energy source and the like are continuously supplied to the reaction system, dialysis (Kikawa *et al.*, The 21st Annual Meeting of the Molecule Biology Society of Japan, WID6), or overlay method (manual of PROTEIOS^{™} Wheat germ cell-free protein synthesis core kit: manufactured by TOYOBO) and the like can be mentioned. Moreover, a method (JP-A-2000-333673) wherein template RNA, amino acid, energy source and the like are supplied to a synthesis reaction system as necessary and a synthesized substance and decomposed product are discharged as necessary, and the like can be used.

A screening method of the present invention is characterized by using a transmembrane enzyme protein or an inhibiting substance binding peptide obtained by any method described above, and measuring the binding of the test substance to the protein or peptide and the enzyme activity of the protein or peptide.

The measurement of the enzyme activity may be carried out with any method conventionally known depending on the transmembrane enzyme used. For example, if the transmembrane enzyme is a β-secretase, the measurement of the enzyme activity can be carried out with, for example, a method described in the above-mentioned WO 01/87293, a method described in Japanese Unexamined Patent Publication No. 11-507538, a method described in Science, 286, 735-741 (1999), Nature, 402, 533-537 (1999), Nature, 402, 537-540 (1999) and the like, and the like.

Specifically, for example, a method can be mentioned wherein a β-secretase protein or an inhibiting substance binding peptide is contacted with a natural or synthetic substrate of β-secretase [APP or a fragment thereof comprising a β-secretase cleavage site, or a synthetic peptide comprising the same or substantially the same amino acid sequence as the amino acid sequence of the cleavage site (such synthetic peptides are commercially available, or those of ordinary skill in the art can also be easily synthesize such peptides by the above-mentioned peptide synthesis method based on an amino acid sequence near the β-secretase cleavage site of APP)] in the presence of a test substance, and after the incubation under the suitable reaction condition for a given time, one or both products resulting from cleavage are detected. The detection methods of the reaction products can include, for example, a method wherein the reaction solution is subjected to SDS-polyacrylamide electrophoresis (SDS-PAGE), two bands (and a band of a uncleaved substrate peptide) are visualized by Coomassie Brilliant Blue (CBB) staining and the like, and the enzyme activity is quantified with a densitometer and the like, a method wherein SDS-PAGE is performed similarly with a substrate labeled with a labeling agent and the amount of the label of each band on the gel is detected, and the like. As examples of the labeling agent, a radioisotope, an enzyme, a fluorescent substance, a luminescent substance and the like can be used. As examples of the radioisotope, [¹²⁵I], [¹³¹I], [³H], [¹⁴C] and the like can be used. As the above-described enzyme, those that are stable and high in specific activity are preferred; for example, β-galactosidase, β-glucosidase, alkaline phosphatase, peroxidase, malate dehydrogenase and the like can be used. As examples of the fluorescent substance, fluorescamine, fluorescein isothiocyanate and the like can be used. As examples of the luminescent substance, luminol, luminol derivative, luciferin, lucigenin and the like can be used.

Preferably, as shown in the below-mentioned Example, βsecretase activity can be measured by reacting a peptide labeled with a fluorescence donor and a fluorescence quencher as a substrate (because there is a β-secretase cleavage site between the fluorescence donor and the fluorescence quencher, the fluorescence is not detected for a unreacted substrate due to the proximity of the donor and the quencher, however, when the substrate is cleaved at the cleavage site by the β-secretase activity, then the quencher is separated, as a result, the fluorescence is detected) with a β-secretase or an inhibiting substance binding peptide in the presence of a test substance as the above-mentioned, and measuring the fluorescence in the reaction solution. Here, as the fluorescence donor, N^{ε}-Methylanthranoyl group, (7-methoxycoumarin-4-yl)acetyl group, 4-((4-(dimethylamino)phenyl)azo)benzoic acid (dabcyl), Cy3B, Cy5 and the like, and as the fluorescence quencher, 2,4-dinitrophenyl group, 5-((2-(Fmoc)-γ-L-glutamylaminoethyl)amino)naphthalene-1-sulfonic acid (EDANS), Cy5Q, Cy7Q and the like are exemplified, respectively, but not limited thereto.

For the binding of a transmembrane enzyme or an inhibiting substance binding peptide and a test substance, the presence or absence of the binding of the test substance to the immobilized enzyme or inhibiting substance binding peptide can be determined, for example, using surface plasmon resonance (SPR) and the change in the resonance angle as an indication, wherein the transmembrane enzyme or the inhibiting substance binding peptide is immobilized onto the surface of a commercially available sensorchip (e.g., manufactured by Biacore) according to a conventional method, the test substance is contacted therewith, and then the sensorchip is illuminated with a light of a particular wavelength from a particular angle. Alternatively, the binding of a transmembrane enzyme or an inhibiting substance binding peptide and a test substance can also be measured by detecting the appearance of a peak corresponding to the test substance by a method wherein the transmembrane enzyme or the inhibiting substance binding peptide is immobilized onto the surface of a protein chip adaptable to a mass spectrometer, a test substance is contacted therewith, and then ionization method such as MALDI-MS, ESI-MS, FAB-MS and the like and mass spectrometer (e.g., double-focusing mass spectrometer, quadrupole mass spectrometer, time-of-flight mass spectrometer, Fourier transformation mass spectrometer, ion cyclotron mass spectrometer and the like) are combined. However, the methods for measuring are not limited thereto, and any other known methods are also available.

The confirmation whether the test substance binds to the target binding site within the transmembrane region in the transmembrane enzyme or the inhibiting substance binding peptide and whether it exerts its enzyme inhibitory activity by binding to the binding site can be performed by using an inhibiting substance non-binding peptide instead of the above-mentioned transmembrane enzyme or inhibiting substance binding peptide, and measuring the enzyme activity and the binding activity of the test substance in the same manner as above-mentioned.

As a result, a substance which binds to the transmembrane enzyme or the inhibiting substance binding peptide and inhibits the enzyme activity, but does not bind the inhibiting substance non-binding peptide and inhibit the enzyme activity, can be selected as a compound which exerts the enzyme inhibitory activity by binding to the target binding site within the transmembrane region.

The present invention also provides a kit for screening, preferable for caring out a screening method of the present invention. The kit comprises at least (a) a subject transmembrane enzyme or an inhibiting substance binding peptide, and (b) an inhibiting substance non-binding peptide, as its constituents. The kit may further comprise reagents or instruments (e.g., substrate, reaction buffer, sensorchip for SPR, protein chip for mass spectrometry etc.) needed for the measurement of the enzyme activity and the binding test.

In a screening method of the present invention, by using β-secretase as a transmembrane enzyme, a β-secretase inhibiting substance which binds to the transmembrane region of the enzyme can be selected. An example of such compound can include, but not limited to the compounds represented by A, D, H, I, J shown in the following Example and the like. Because the β-secretase inhibiting substance inhibits β-secretase activity by binding to the transmembrane region of the enzyme, it has high selectivity to β-secretase unlike a conventionally known transition state mimic and other inhibitors acting on the active center of β-secretase. That is, the β-secretase inhibiting substance has extremely low possibility to inhibit other aspartic proteases having a structurally similar pocket (e.g., a non-transmembrane aspartic protease such as rennin, cathepsin D and the like), such that the occurrence of side effects (e.g., blood pressure reduction due to inhibition by rennin, cathepsin D and the like) is less likely. Accordingly, a β-secretase selective inhibiting substance which can be selected by a screening method of the present invention can be use as a less toxic and safer agent for prophylaxis and/or treatment of AD, Down syndrome, Age-Associated Memory Impairment (AAMI) and the like (wherein, AAMI generally refers as an age-related memory decline, but not the name of disease or diagnosis, and as a condition accompanied by no pathological process and within the physiological range. However, the term "treatment" is comprehensively used herein including "improvement of condition" of AAMI).

When a β-secretase selective inhibiting substance is used as the above-mentioned prophylactic and/or therapeutic agent, it can be formulated according to a conventional means. For example, the inhibiting substance can be used orally as tablets coated with sugar as required, capsules, elixirs, microcapsules and the like, or can be used parenterally in the form of an injectable such as a sterile solution or suspension in water or another pharmaceutically acceptable liquid. For example, a preparation can be produced by blending the inhibiting substance with a known physiologically acceptable carrier, a sweetener, an excipient, a vehicle, an antiseptic, a stabilizer, a binder and the like, in a unit dosage form required for generally accepted preparation design. The active ingredient contents in these preparations are intended to ensure that an appropriate dose in the specified range is obtained.

As examples of additives that can be formulated in tablets, capsules and the like, a binder like gelatin, cornstarch, tragacanth and gum arabic, a excipient like crystalline cellulose, a swelling agent like cornstarch, gelatin, alginic acid and the like, a lubricant like magnesium stearate, a sweetener like sucrose, lactose or saccharin, a flavoring agent like peppermint, acamono oil or cherry and the like can be used. When the formulation unit form is a capsule, the above-described type of material can further contain a liquid carrier like an oil or fat. A sterile composition for injection can be formulated according to an ordinary preparation design such as dissolving or suspending an active substance, a naturally produced vegetable oil such as sesame oil or coconut oil, and the like in a vehicle like water for injection. As examples of aqueous solutions for injection, physiological saline, an isotonic solution containing glucose or other auxiliary agent (e.g., D-sorbitol, D-mannitol, sodium chloride and the like) and the like can be used, which may be used in combination with an appropriate solubilizer, for example, an alcohol (e.g., ethanol), a polyalcohol (e.g., propylene glycol, polyethylene glycol), a non-ionic surfactant (e.g., polysorbate 80^{™}, HCO-50) and the like. As examples of oily solutions, sesame oil, soybean oil and the like can be used, which may be used in combination with solubilizers benzyl benzoate, benzyl alcohol and the like.

Also, the above-described prophylactic or therapeutic agent may be formulated with, for example, a buffering agent (e.g., phosphate buffer solution, sodium acetate buffer solution), a soothing agent (e.g., benzalkonium chloride, procaine hydrochloride and the like), a stabilizer (e.g., human serum albumin, polyethylene glycol and the like), a preservative (e.g., benzyl alcohol, phenol and the like), an antioxidant and the like. The prepared injection solution is normally filled in an appropriate ampoule.

Since the preparation thus obtained is safe and of low toxicity, it can be administered to, for example, a human or another warm-blooded animal (e.g., rat, mouse, hamster, rabbit, sheep, goat, swine, bovine, horse, cat, dog, monkey, chimpanzee, bird and the like).

The dosage of β-secretase selective inhibiting substance varies depending on subject of administration, symptoms, method of administration and the like; in an AD patient (body weight 60 kg), for example, the usual oral dosage is about 0.1 mg to 100 mg, preferably about 1.0 to 50 mg, more preferably about 1.0 to 20 mg, per day. In the case of parenteral administration, the dosage per administration varies depending on subject of administration, symptoms, method of administration and the like; in an AD patient (body weight 60 kg), for example, it is convenient that the usual dosage in an injection is about 0.01 to 30 mg, preferably about 0.1 to 20 mg, more preferably about 0.1 to 10 mg, per day. In the case of another animal, a dosage converted per 60 kg body weight can be administered.

A β-secretase inhibiting substance which can be selected by a screening method of the present invention has also an advantage in that, in the optimization of the structure, it can be a lead compound whose structure can be developed without being limited by inhibitory action on other aspartic proteases, unlike a β-secretase inhibitor acting on the active center.

Abbreviations for bases, amino acids and the like used in the present specification and drawings are based on abbreviations specified by the IUPAC-IUB Commission on Biochemical Nomenclature or abbreviations in common use in relevant fields. Some examples are given below. When an enantiomer may be present in amino acid, it is of the L-configuration, unless otherwise stated.
DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediamine tetraacetic acid
SDS: Sodium dodecyl sulfate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine
pGlu: Pyroglutamic acid
Me: Methyl group
Et: Ethyl group
Bu: Butyl group
Ph: Phenyl group
TC: Thiazolidine-4(R)-carboxamide group
   Substituents, protecting groups and reagents frequently mentioned herein are represented by the symbols shown below.
Tos: p-Toluenesulfonyl
CHO: Formyl
Bzl: Benzyl
Cl₂Bzl: 2, 6-Dichlorobenzyl
Bom: Benzyloxymethyl
Z: Benzyloxycarbonyl
C1-Z: 2-Chlorobenzyloxycarbonyl
Br-Z: 2-Bromobenzyloxycarbonyl
Boc: t-Butoxycarbonyl
DNP: Dinitrophenol
Trt: Trityl
Bum: t-Butoxymethyl
Fmoc: N-9-Fluorenylmethoxycarbonyl
HOBt: 1-Hydroxybenztriazole
HOOBt: 3,4-Dihydro-3-hydroxy-4-oxo-1,2,3-benzotriazine
HONB: 1-Hydroxy-5-norbornane-2,3-dicarboximide
DCC: N,N'-Dicyclohexylcarbodiimide

The present invention is hereinafter described in more detail by means of the following Reference Examples and Examples, which examples, however, are not to be construed as limiting the scope of the present invention. A change can be made in the present invention without departing from the scope of the present invention.

### Example

Five compounds used in the following Example were produced according to the description of the above-mentioned WO 01/87293:

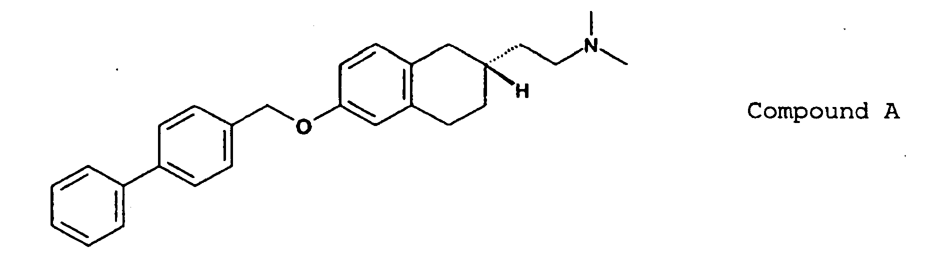

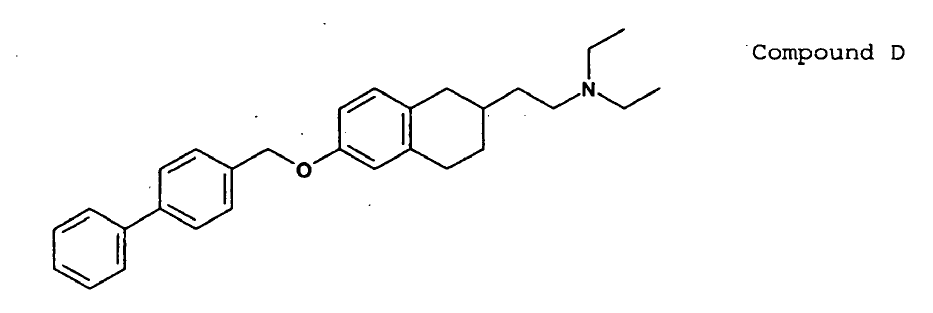

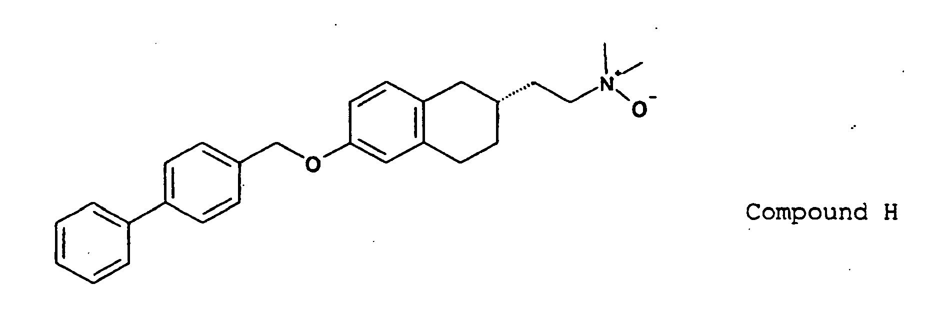

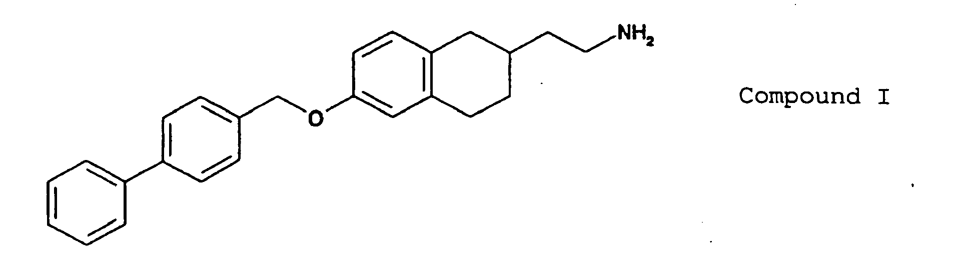

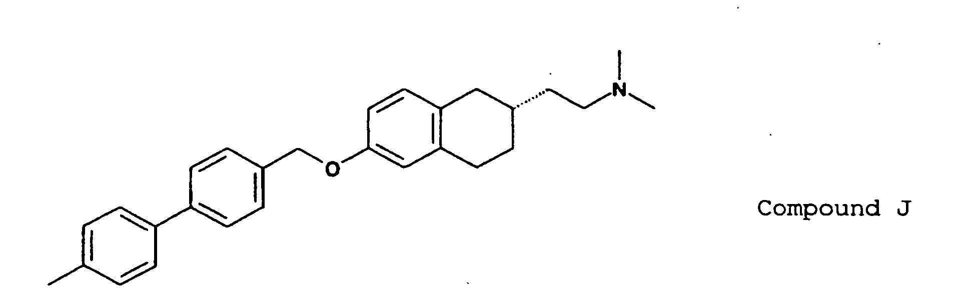

Genetic engineering methods using Escherichia coli were performed according to the methods described in Molecular cloning.

### Reference Example 1: Preparation of an expression plasmid for full-length β-secretase protein

The construction of an expression plasmid for preparation of full-length β-secretase protein was carried out as following. In a base sequence of a gene encoding β-secretase, it was revealed that a base sequence of Clone No. FG04087 (GenBank Accession No. AB032975, Kazusa DNA Research Institute) had 1 base insertion (at 102^{nd}) compared with a base sequence reported by Bennett et al. (Science 286,735-741(1999)). Therefore, a conversion was performed, and further, a base sequence (5'-GATTACAAGGATGACGACGATAAG-3' (SEQ ID NO: 3)) encoding Flag peptide (Asp-Tyr-Lys-Asp-Asp-Asp-Asp-Lys (SEQ ID NO: 4)) was added to the C-terminal for easy purification. First, using a gene of Clone No. FG04087 as a template, adding each 20 pmol of primer set: 5'-GGCACCACCAACCTTCGT-3' (SEQ ID NO: 5) and 5'-GGTACCTACTTATCGTCGTCATCCTTGTAATCCTTCAGCAGGGAGATGTCATCAG-3' (SEQ ID NO: 6) comprising the base sequence encoding Flag peptide, which are designed with reference to the base sequence of β-secretase gene reported by Bennett *et al.,* the PCR reaction was performed on MiniCycler^{™} (MJ research) using KOD (TOYOBO) (reaction condition: 94°C for 2 min. x 1 cycle; 98°C for 15 sec., 72°C for 2 sec., 74°C for 10 sec. x 3 cycles; 98°C for 15 sec., 68°C for 2 sec., 74°C for 10 sec. x 3 cycles; 98°C for 15 sec., 64°C for 2 sec., 74°C for 10 sec. x 3 cycles; 98°C for 15 sec., 60°C for 2 sec., 74°C for 10 sec. x 28 cycles). The PCR products were subjected to agarose gel electrophoresis to recover about 700 bp DNA fragment. The fragment was cloned using Zero Blunt TOPO PCR Cloning Kit (Invitrogen). Obtained plasmid was digested with restriction enzymes ApaI (TaKaRa) and KpnI (TaKaRa), and then subjected to agarose gel electrophoresis to recover about 250 bp DNA fragment. A plasmid comprising Clone No. FG04087 was digested with ApaI, and then subjected to agarose gel electrophoresis to recover about 1.2 kbp DNA fragment. These DNA fragments and an expression plasmid for animal cell, pcDNA3.1(-)(Funakoshi), digested with ApaI and KpnI were mixed, ligated using Ligation High (TOYOBO), and transformed into Escherichia coli JM109 competent cells (TaKaRa) to give a plasmid pBACE-F. Next, for the conversion of 1 base insertion, using a gene of Clone No. FG04087 as a template, adding each 20 pmol of primer set: 5'-TAATACGACTCACTATAGGG-3' (SEQ ID NO: 7) and 5'-GGCGCCCCCCAGACCACTTCTCAG-3' (SEQ ID NO: 8) which are designed with reference to the base sequence of β-secretase gene reported by Bennett *et al*., the PCR reaction was performed on MiniCycler^{™} using KOD (TOYOBO) (reaction condition: 94°C for 2 min. x 1 cycle; 98°C for 15 sec., 72°C for 2 sec., 74°C for 10 sec. x 3 cycles; 98°C for 15 sec., 68°C for 2 sec., 74°C for 5 sec. x 3 cycles; 98°C for 15 sec., 64°C for 2 sec., 74°C for 5 sec. x 3 cycles; 98°C for 15 sec., 60°C for 2 sec., 74°C for 5 sec. x 28 cycles). The PCR products were subjected to agarose gel electrophoresis to recover about 170 bp DNA fragment. The fragment was cloned using Zero Blunt TOPOPCR Cloning Kit (Invitrogen). Obtained plasmid was digested with restriction enzymes ApaI (TaKaRa) and BbeI (TaKaRa), and then subjected to agarose gel electrophoresis to recover about 120 bp DNA fragment. pBACE-F was digested with the same restriction enzymes, and then subjected to agarose gel electrophoresis to recover about 1.1 kbp DNA fragment. Further, pBACE-F was digested with ApaI, and then subjected to agarose gel electrophoresis to recover about 5.7 kbp DNA fragment. These three fragments were ligated using Ligation High, and transformed into Escherichia coli JM109 competent cells to give a plasmid pBACE1-501. Resulting cDNA fragment has a base sequence shown by SEQ ID NO: 9, and 1^{st} -1527^{th} of the base sequence encodes an amino acid sequence shown by SEQ ID NO: 10.

### Reference Example 2: Expression and purification of full-length β-secretase protein in HEK293 cells

Expression of full-length β-secretase protein (BACE1-501) was performed with FreeStyle 293 Expression System (Invitrogen). FreeStyle 293-F cells were seeded into 140 ml of FreeStyle 293 Expression Medium at 1.1 x cells/ml. 200 µl of 293fectin was diluted with 5 ml of Opti-MEM I medium, mixed with 150 µg of the expression plasmid diluted with 5 ml of Opti-MEM I medium, allowed to stand for 20 min. at room temperature, and then added to FreeStyle 293-F cells. After shaking culture at 37°C, under 8% CO₂ gas and at 125 rpm for 2 days, the cells were recovered, and disrupted by a ultrasonic disintegrator (TOMY SEIKO) (disruption condition: output of 5, 15 sec. x 4) after addition of 5 ml of suspending buffer (0.01 M Tris-HC1 (pH8), 0.15 M NaCl, 1 mM EDTA, 0.5 mM PMSF) to them. The disrupted solution was centrifuged (500 g, 10 min.), the supernatant was further centrifuged (100,000 g, 45 min.), and the precipitate was solubilized (4°C, 2.5 hr) in 0.5 ml of solubilizing buffer (0.01 M Tris-HC1 (pH 8), 0.05 M octyl-β-glucoside, 1 mM EDTA, 0.5 mM PMSF) and then centrifuged (100,000 g, 45 min.). The supernatant was purified with 100 µl of anti-Flag antibody (Sigma Ltd.). As a result, 395 µg of the subject BACE1-501 was obtained.

### Reference Example 3: Analysis of the inhibition mode of Compounds A, D, H, I and J on BACE1-501 protein

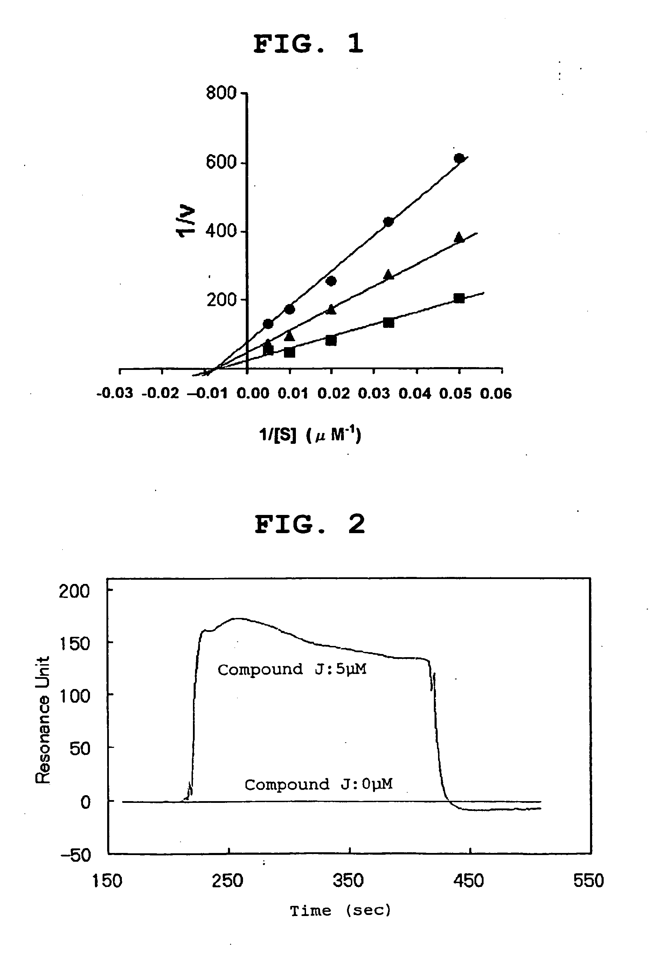

To 96 black well plate (Corning), 25 µl of 0.05 M acetate buffer (pH 5.5), each 10 µl of 0.1 mM, 0.15 mM, 0.25 mM, 0.5 mM, 1 mM substrate (Nma-Ser-Glu-Val-Lys-Met-Asp-Ala-Glu-Lys(Dnp)-Arg-Arg-NH₂;SEQ ID NO: 11), 10 µl of BACE1-501(0.07 mg/ml) obtained from the above-mentioned (2), and each 5 µl of 0.1 mM, 0.3 mM Compound A in 10% DMSO solution, and as a control, 5 µl of 10% DMSO were added respectively, and allowed to be reacted at 37°C for 20 hr. After completion of the reaction, the fluorescence intensity (excitation wavelength at 325 nm, measurement wavelength at 460 nm) was measured with Fluoroscan ASCENT (Labosystems). The reciprocal of the amount of the change in the fluorescence value in presence of the compound at each concentration was plotted on vertical axis, and the reciprocal of the substrate concentration was plotted on longitudinal axis (Lineweaver-Burk plot). As a result, the lines crossed on the longitudinal axis in all compounds, revealing that the inhibition mode of these compounds on full-length BACE1-501 is non-competitive inhibition. As an example, the graph for Compound D is shown in Fig. 1.

It is believed that because each compound inhibits full-length BACE1-501 non-competitively, it binds other than the active site. Next, to reveal the binding site, proteins consisting of each domain were prepared to perform inhibition and binding experiments.

### Reference Example 4: Preparation of expression plasmids for a protein consisting of an extracellular domain and a protein consisting of the extracellular domain and a transmembrane domain of β-secretase

An expression plasmid to prepare a protein consisting of the extracellular domain of β-secretase was prepared that has a base sequence with Flag peptide added to amino acid 1-454. First, using the plasmid pBACE1-501 as a template, adding each 20 pmol of primer set: 5'-GCTGGCTAGCGTTTAAACGGGCCCTCTAGA-3' (SEQ ID NO: 12) and 5'-TTTTGGTACCTACTTATCGTCGTCATCCTTGTAATCGGTTGACTCATCTGTC-3' (SEQ ID NO: 13), the PCR reaction was performed on Gene Amp PCR system 9700 (Applied BioSystems) using pfu Turbo (Stratagene) (reaction condition: 94°C for 1 min. x 1 cycle; 94°C for 30 sec., 58°C for 30 sec., 72°C for 2 min. x 20 cycles). The PCR products were subjected to agarose gel electrophoresis to recover about 1.4 kbp DNA fragment. Obtained DNA fragment was digested with restriction enzymes NheI (TaKaRa) and KpnI, and then recovered with spin column S-300 (Amersham Biosciences). These DNA fragments and pcDNA3.1(-) digested with NheI and KpnI were mixed, ligated using Ligation High, and transformed into Escherichia coli DH5-α competent cells (TOYOBO) to give a plasmid pBACE1-454. Resulting cDNA fragment has a base sequence shown by SEQ ID NO: 14, and 1^{st} - 1386^{th} of the base sequence encodes an amino acid sequence shown by SEQ ID NO: 15.

An expression plasmid to prepare a protein consisting of the extracellular domain and the transmembrane domain of β-secretase was prepared that has a base sequence with Flag peptide added to amino acid 1-474. First, using the plasmid pBACEl-501 as a template, adding each 20 pmol of primer set: 5'-GCTGGCTAGCGTTTAAACGGGCCCTCTAGA-3' (SEQ ID NO: 12) and 5'-TTTTGGTACCTACTTATCGTCGTCATCCTTGTAATCGCAGAGTGGCAGCATG-3' (SEQ ID NO: 16), the PCR reaction was performed on Gene Amp PCR system 9700 using pfu Turbo (reaction condition: 94°C for 1 min. x 1 cycle; 94°C for 30 sec., 58°C for 30 sec., 72°C for 2 min. x 20 cycles). The PCR products were subjected to agarose gel electrophoresis to recover about 1.5kbp DNA fragment. Obtained DNA fragment was digested with restriction enzymes NheI and KpnI, and then recovered with spin column S-300. These DNA fragments and pcDNA3.1(-) digested with NheI and KpnI were mixed, ligated using Ligation High, and transformed into Escherichia coli DH5-α competent cells to give the plasmid pBACE1-474. Resulting cDNA fragment has a base sequence shown by SEQ ID NO: 17, and 1^{st} - 1446^{th} of the base sequence encodes an amino acid sequence shown by SEQ ID NO: 18.

### Reference Example 5: Expression and purification of the protein consisting of the extracellular domain (BACE1-454) and the protein consisting of the extracellular domain and the transmembrane domain (BACE1-474) of β-secretase in HEK293 cells

Expression of each protein was performed with FreeStyle 293 Expression System (Invitrogen). FreeStyle 293-F cells were seeded into 140 ml of FreeStyle 293 Expression Medium at 1.1 x cells/ml. 200 µl of 293fectin was diluted with 5ml of Opti-MEM I medium, mixed with 150 µg of the expression plasmid diluted with 5ml of Opti-MEM I medium, allowed to stand for 20 min. at room temperature, and then added to FreeStyle 293-F cells. After shaking culture at 37°C, under 8% CO₂ gas and at 125 rpm for 2 days, for BACE1-474, the cells were recovered, and disrupted by a ultrasonic disintegrator (TOMY SEIKO) (disruption condition: output of 5, 15 sec. x 4) after addition of 5 ml of suspending buffer (0.01 M Tris-HC1 (pH 8), 0.15 M NaCl, 1 mM EDTA, 0.5 mM PMSF) to them. The disrupted solution was centrifuged (500 g, 10 min.), the supernatant was further centrifuged (100,000 g, 45 min.), and the precipitate was solubilized (4°C, 2.5 hr) in 0.5 ml of solubilizing buffer (0.01 M Tris-HCl (pH 8), 0.05 M octyl-β-glucoside, 1 mM EDTA, 0.5 mM PMSF) and then centrifuged (100,000 g, 45 min.). The supernatant was purified with 100 µl of anti-Flag antibody. As a result, 70 µg of the subject BACE1-474 was obtained. For BACE1-454, the culture supernatant was recovered, and purified with 100 µl of anti-Flag antibody. As a result, 14 µg of the subject BACE1-454 was obtained.

### Example 1: Measurement of the inhibitory action of Compounds A, D, H, I and J on each BACE protein

To 96 black well plate, 25 µl of 0.05 M acetate buffer (pH 5.5), 10 µl of 250 µM substrate (Nma-Ser-Glu-Val-Lys-Met-Asp-Ala-Glu-Lys(Dnp)-Arg-Arg-NH₂; SEQ ID NO: 11), 10 µl of BACE1-474 (0.02 mg/ml) or BACE1-454 (0.01 mg/ml) obtained from the above-mentioned Reference Example 5 or a recombinant human BACE-1 (amino acid residues 1-460, R & D Systems) (0.025 mg/ml) commercially available as an extracellular domain, and 5 µl of each compound in 10% DMSO solution, and as a control, 5 µl of 10% DMSO were added respectively, and allowed to be reacted at 37°C for 63 hr. After completion of the reaction, the fluorescence intensity (excitation wavelength at 325 nm, measurement wavelength at 460 nm) was measured with Fluoroscan ASCENT. Each compound (final concentration: 10 µM) exhibited the inhibition rates of 30% (Compound A), 38% (Compound D), 34 % (Compound H), 37% (Compound I), 37% (Compound J) on BACE1-474, but exhibited no inhibitory activity on BACE1-454 and the recombinant human BACE-1.

### Example 2: Detection of binding of each BACE protein to Compounds A, D, H, I and J by surface plasmon resonance

Biacore3000 (Biacore) was used for analysis by surface plasmon resonance. BACE1-501 and the recombinant human BACE-1 (amino acid residue 1-460) were immobilized to carboxyl groups on CM5 sensorchip (carboxymethylated dextran matrix chip) activated with N-hydrosuccinimide (NHS)/N-ethyl-N'-(3-dimethyl-aminopropyl)-carbodiimide hydrochloride (EDC) in acetate buffer at pH 4.5. BACE1-454 and BACE1-474 were immobilized by the same way in acetate buffer at pH 4.0. The binding of the compound and the enzymes was measured in PBS containing 10% DMSO and 0.005% Surfactant P20. The correction of the difference of Resonance Unit (RU) value among flow cells resulting from the bulk effect was carried out with a correction curve made using the buffers with the DMSO concentration varied from 9% to 11% in the five-graded. As a result, signals showing the binding of each compound to BACE1-501 and BACE1-474 were observed. On the other hand, for BACE1-454 and the recombinant human BACE-1 (amino acid residue 1-460), no signal was observed showing the binding. As an example, a signal diagram showing the binding of BACE1-501 and Compound J is shown in Fig. 2.

It was revealed that each compound inhibits full-length BACE1-501 by binding to any site between amino acid 461 and 474 in the transmembrane region. Next, to investigate in more detail which part of amino acid 461-474 in the transmembrane region the compound binds to for inhibition, a protein with amino acids deleted from carboxyl terminal of BACE1-474 was prepared to perform inhibition and binding experiments.

### Reference Example 6: Construction of expression plasmids for a β-secretase protein with amino acids deleted from carboxyl terminal of BACE1-474

Expression plasmids for β-secretase proteins with amino acids deleted gradually from carboxyl terminal of BACE1-474 were prepared. First, for preparation of a plasmid having a base sequence with Flag peptide added to amino acid 1-471, using the plasmid pBACE1-474 as a template, primer set:5'-CATCTGCGCCCTCTTCATGCTGGATTACAAGGATGACGACG-3' (SEQ ID NO: 19) and 5'-CGTCGTCATCCTTGTAATCCAGCATGAAGAGGGCGCAGATG-3' (SEQ ID NO: 20), and QuickChange Site-Directed Mutagenesis Kit (Stratagene), 9 bases were deleted to give a plasmid pBACE1-471. Resulting cDNA fragment has a base sequence shown by SEQ ID NO: 21, and 1^{st} -1437^{th} of the base sequence encodes an amino acid sequence shown by SEQ ID NO: 22.

Then, a plasmid having a base sequence with Flag peptide added to amino acid 1-469 was prepared. Using the plasmid pBACE1-474 as a template, adding each 20 pmol of primer set: 5'-GCTGGCTAGCGTTTAAACGGGCCCTCTAGA-3' (SEQ ID NO: 12) and 5'-TTTTGGTACCTACTTATCGTCGTCATCCTTGTAATCGAAGAGGGCGCAGATG-3' (SEQ ID NO: 23), the PCR reaction was performed on Gene Amp PCR system 9700 using pfu Turbo (reaction condition:94°C for 1 min. x 1 cycle; 94°C for 30 sec., 58°C for 30 sec., 72°C for 2 min. x 20 cycles). The PCR products were subjected to agarose gel electrophoresis to recover about 1.4 kbp DNA fragment. Obtained DNA fragment was digested with restriction enzymes NheI and KpnI, and then recovered with spin column S-300. These DNA fragments and pcDNA3.1(-) digested with NheI and KpnI were mixed, ligated using Ligation High, and transformed into Escherichia coli DH5-α competent cells to give a plasmid pBACE1-469. Resulting cDNA fragment has a base sequence shown by SEQ ID NO: 24, and 1^{st} - 1431^{st} of the base sequence encodes an amino acid sequence shown by SEQ ID NO: 25. Further, a plasmid having a base sequence with Flag peptide added to amino acid 1-465 was prepared. That is, using the plasmid pBACE1-469 as a template, primer set: 5'-GCCTATGTCATGGCTGCCATCGATTACAAGGATGACGACG-3' (SEQ ID NO: 26) and 5'-CGTCGTCATCCTTGTAATCGATGGCAGCCATGACATAGGC-3' (SEQ ID NO: 27), and QuickChange Site-Directed Mutagenesis Kit, 12 bases were deleted to give a plasmid pBACE1-465. Resulting cDNA fragment has a base sequence shown by SEQ ID NO: 28, and 1^{st} - 1419^{th} of the base sequence encodes an amino acid sequence shown by SEQ ID NO: 29.

### Reference Example 7: Expression and purification of BACE1-471 and BACE1-465 in HEK293 cells

Expression of each protein was performed with FreeStyle 293 Expression System (Invitrogen). FreeStyle 293-F cells were seeded into 140 ml of FreeStyle 293 Expression Medium at 1.1 x cells/ml. 200 µl of 293fectin was diluted with 5 ml of Opti-MEM I medium, mixed with 150 µg of the expression plasmid diluted with 5 ml of Opti-MEM I medium, allowed to stand for 20 min. at room temperature, and then added to FreeStyle 293-F cells. After shaking culture at 37°C, under 8% CO₂ gas and at 125 rpm for 2 days, for BACE1-471, the cells were recovered, and disrupted by a ultrasonic disintegrator (TOMY SEIKO) (disruption condition: output of 5, 15 sec. x 4) after addition of 5 ml of suspending buffer (0.01 M Tris-HC1 (pH 8), 0.15 M NaCl, 1 mM EDTA, 0.5 mM PMSF) to them. The disrupted solution was centrifuged (500 g, 10 min.), the supernatant was further centrifuged (100,000 g, 45 min.), and the precipitate was solubilized (4°C, 2.5 hr) in 0.5 ml of solubilizing buffer (0.01 M Tris-HCl (pH 8), 0.05 M octyl-β-glucoside, 1 mM EDTA, 0.5 mM PMSF) and then centrifuged (100,000 g, 45 min.). The supernatant was purified with 100 µl of anti-Flag antibody. As a result, 105 µg of the subject BACE1-471 was obtained. For BACE1-465, the culture supernatant was recovered, and purified with 100 µl of anti-Flag antibody. As a result, 91 µg of the subject BACE1-465 was obtained.

### Example 3: Measurement of the inhibitory action of Compounds A, D, H, I and J on BACE1-471 and 1-465

To 96 black well plate, 25 µl of 0.05M acetate buffer(pH5.5), 10 µl of 250 µM substrate (Nma-Ser-Glu-Val-Lys-Met-Asp-Ala-Glu-Lys(Dnp)-Arg-Arg-NH₂;SEQ ID NO: 11), 10 µl of BACE1-471 (0.05 mg/ml) or BACE1-465 (0.04 mg/ml) obtained from the above-mentioned Reference Example 7, and 5 µl of 0.1mM Compound A in 10% DMSO solution, and as a control, 5 µl of 10% DMSO were added respectively, and allowed to be reacted at 37°C for 50 hr for BACE1-471 and for 22 hr for BACE1-465. After completion of the reaction, the fluorescence intensity (excitation wavelength at 325 nm, measurement wavelength at 460 nm) was measured with Fluoroscan ASCENT. Each compound (final concentration: 10 µM) exhibited the inhibition rates of 29% (Compound A), 42% (Compound D), 31% (Compound H), 70% (Compound I), 48% (Compound J) on BACE1-471, but exhibited no inhibitory activity on BACE1-465.

### Example 4: Detection of binding of BACE1-471 and BACE1-465 to Compounds A, D, H, I and J by surface plasmon resonance

Biacore3000 was used for analysis by surface plasmon resonance. BACE1-465 was immobilized to carboxyl groups on CM5 sensorchip activated with NHS/EDC in acetate buffer at pH4.5. BACE1-465 was immobilized by the same way in acetate buffer at pH 4.0. The binding of the compound and the enzyme was measured in PBS containing 10% DMSO and 0.005% Surfactant P20. The correction of the difference of RU value among flow cells resulting from the bulk effect was carried out with a correction curve made using the buffers with the DMSO concentration varied from 9% to 11% in the five-graded. As a result, signals showing the binding of each compound to BACE1-471 were observed. However, no signal was observed showing the binding to BACE1-465.

The above results reveal that Compounds A, D, H, I and J bind to the amino acid position 466-471 of β-secretase to exhibit the inhibitory activity, suggesting that the amino acid position 466-471 of β-secretase is a site having important effect on regulation of its activity.

### Industrial Applicability

A compound inhibiting a β-secretase activity by binding to the transmembrane region, which can be obtained by a screening method or a kit for screening of the present invention, shows a different β-secretase inhibition mode from a conventionally known transition state mimic and other inhibitors acting on the active center. Therefore, it may have an advantageous property (e.g., selectively acting on β-secretase and not inhibiting other aspartic proteases such as rennin, cathepsin D and the like, etc.) in the prophylaxis and/or treatment for a disease expected to be prevented and/or treated by inhibiting the β-secretase, for example, a disease associated with an abnormal deposition of Aβ, such as AD, Down syndrome and the like, a condition like AAMI. Accordingly, the screening method of the present invention is useful for the search of a drug for prophylaxis and/or treatment of AD, Down syndrome, AAMI and the like, which is at a low risk of the side effect and has a novel site of inhibitory action.

This application is based on application No. 2004-290784 (filing date: October 1, 2004) filed in Japan, the contents of which are incorporated herein by reference.

### Free-text of Sequence Listing

[SEQ ID NO: 3]
   oligonucleotide encoding Flag peptide.
[SEQ ID NO: 4]
   synthetic construct.
[SEQ ID NO: 5]
   primer.
[SEQ ID NO: 6]
   primer.
[SEQ ID NO: 7]
   primer.
[SEQ ID NO: 8]
   primer.
[SEQ ID NO: 9]
   (1504)..(1527) Flag tag.
[SEQ ID NO: 11]
   synthetic substrate of β-secretase.
   (1)..(1) N-methylanthranoyl-L-serine
   (9)..(9) 2,4-dinitrophenyl-L-lysine
   (11)..(11) amidated
[SEQ ID NO: 12]
   primer.
[SEQ ID NO: 13]
   primer.
[SEQ ID NO: 14]
   (1363) .. (1386) Flag tag.
[SEQ ID NO: 16]
   primer.
[SEQ ID NO: 17]
   (1423) .. (1446) Flag tag.
[SEQ ID NO: 19]
   primer.
[SEQ ID NO: 20]
   primer.
[SEQ ID NO: 21]
   (1414)..(1437) Flag tag.
[SEQ ID NO: 23]
   primer.
[SEQ ID NO: 24]
   (1408)..(1431) Flag tag.
[SEQ ID NO: 26]
   primer.
[SEQ ID NO: 27]
   primer.
[SEQ ID NO: 28]
   (1396).. (1419) Flag tag.

## Claims

1. A screening method for a β-secretase inhibiting substance specifically binding to a transmembrane region of the β-secretase, comprising the following steps:
(I) measurement of the activity of a first protein having a part or all of an amino acid sequence of the β-secretase, comprising
(i) a region comprising an active center of the β-secretase and
(ii) a region comprising a part or all of a transmembrane region of the β-secretase, comprising at least a binding site targeted by a substance having a subject enzyme inhibitory activity;
(II) measuring the binding of a test substance to the first protein;
(III) selection of a β-secretase inhibiting substance, which is the test substance that inhibits β-secretase activity by binding to the transmembrane region (ii) of the β-secretase,
wherein region (i) comprises an amino acid sequence comprising a sequence homology of not less than 70% with the amino acid sequence shown by amino acid 93-292 in the amino acid sequence shown by SEQ ID NO: 2,
the transmembrane region of the β-secretase has an amino acid sequence having a sequence homology of not less than 70% with the amino acid sequence shown by amino acid 455-480 in the amino acid sequence shown by SEQ ID NO: 2, and
the region (ii) above comprises a region having an amino acid sequence having a sequence homology of not less than about 70% with the amino acid sequence shown by amino acid 466-471 in the amino acid sequence shown by SEQ ID NO: 2.

2. The method of claim 1, further comprising the following steps:
(I') measurement of the activity of a second protein having a part of an amino acid sequence of the β-secretase, comprising the region (i) above in the first protein and lacking the part or all of the region (ii) above in the first protein in the presence of the test substance,
wherein the assessment in step (II) above comprises:
(a) measuring the binding of the test substance to the first protein;
(b) measuring the binding of the test substance to the second protein;
(c) determining that the test substance binds to region (ii) above in the β-secretase if the test substance binds with the first protein but does not bind with the second protein
and the selection in the step (III) above comprises:
(d) selection of the β-secretase inhibiting substance, which inhibits β-secretase activity by binding to the transmembrane region (ii) of β-secretase, test substance which binds to the first protein and inhibits the enzyme activity of the first protein but does not bind and does not inhibit the enzyme activity of the second protein.

3. The method of claim 1, wherein region (i) above has an amino acid sequence having a sequence homology of not less than 70% with the amino acid sequence shown by amino acid 46-454 in the amino acid sequence shown by SEQ ID NO: 2.

4. A kit for screening for a β-secretase inhibiting substance specifically binding to a transmembrane region of the β-secretase, comprising the following (a) and (b):
(a) a first protein having a part or all of an amino acid sequence of the β-secretase, comprising
(i) a region comprising an active center of the β-secretase and
(ii) a region comprising a part or all of the transmembrane region of the β-secretase; and
(b) a second protein having a part of an amino acid sequence of the β-secretase, comprising a region (i) above in the first protein and lacking region (ii) above in the first protein ,
wherein region (i) comprises an amino acid sequence having a sequence homology of not less than 70% with the amino acid sequence shown by amino acid 93-292 in the amino acid sequence shown by SEQ ID NO: 2,
the transmembrane region of the β-secretase has an amino acid sequence having a sequence homology of not less than 70% with the amino acid sequence shown by amino acid 455-480 in the amino acid sequence shown by SEQ ID NO: 2, and
the region (ii) above comprises a region having an amino acid sequence having a sequence homology of not less than 70% with the amino acid sequence shown by amino acid 466-471 in the amino acid sequence shown by SEQ ID NO: 2.

5. The kit of claim 4, wherein region (i) above has an amino acid sequence having a sequence homology of not less than 70% with the amino acid sequence shown by amino acid 46-454 in the amino acid sequence shown by SEQ ID NO: 2.

## Patentansprüche

1. Screeningverfahren nach einer β-Sekretase inhibierende Substanz, die spezifisch an eine Transmembranregion der β-Sekretase bindet, umfassend die folgenden Schritte:
(I) Messen der Aktivität eines ersten Proteins mit einem Teil von oder der gesamten Aminosäuresequenz der β-Sekretase, umfassend
(i) eine Region, umfassend ein aktives Zentrum der β-Sekretase, und
(ii) eine Region, umfassend einen Teil von oder die gesamte Transmembranregion der β-Sekretase, umfassend zumindest eine Bindungsstelle, die von einer Substanz mit der gegenständlichen Enzyminhibierungsaktivität targetiert wird;
(II) Messen der Bindung einer Testsubstanz an das erste Protein;
(III)Auswahl einer β-Sekretase inhibierenden Substanz, welche die Testsubstanz ist, die die β-Sekretaseaktivität durch Binden an die Transmembranregion (ii) der β-Sekretase inhibiert,
wobei Region (i) eine Aminosäuresequenz, umfassend eine Sequenzhomologie von nicht weniger als 70 % mit der Aminosäuresequenz, gezeigt von Aminosäure 93 - 292 in der von SEQ ID NR.: 2 gezeigten Aminosäuresequenz, umfasst,
die Transmembranregion der β-Sekretase eine Aminosäuresequenz mit einer Sequenzhomologie von nicht weniger als 70 % mit der Aminosäuresequenz, gezeigt von Aminosäure 455 - 480 in der von SEQ ID NR.: 2 gezeigten Aminosäuresequenz, aufweist, und
die Region (ii) oben eine Region mit einer Aminosäuresequenz mit einer Sequenzhomologie von nicht weniger als etwa 70 % mit der Aminosäuresequenz, gezeigt von Aminosäure 466 - 471 in der von SEQ ID NR.: 2 gezeigten Aminosäuresequenz, umfasst.

2. Verfahren nach Anspruch 1, ferner umfassend die folgenden Schritte:
(I') Messen der Aktivität eines zweiten Proteins mit einem Teil einer Aminosäuresequenz der β-Sekretase, umfassend die Region (i) oben in dem ersten Protein und ohne einen Teil von oder der gesamten Region (ii) oben in dem ersten Protein in Gegenwart der Testsubstanz,
wobei die Bewertung in Schritt (II) oben:
(a) das Messen der Bindung der Testsubstanz an das erste Protein;
(b) das Messen der Binding der Testsubstanz an das zweite Protein;
(c) das Bestimmen, dass die Testsubstanz an Region (ii) oben in der β-Sekretase bindet, wenn die Testsubstanz mit dem ersten Protein, aber nicht mit dem zweiten Protein bindet, umfasst
und die Auswahl in Schritt (III) oben:
(d) die Auswahl der β-Sekretase inhibierenden Substanz, die β-Sekretaseaktivität durch Binden an die Transmembranregion (ii) der β-Sekretase inhibiert, nämlich der Testsubstanz, die an das erste Protein bindet und die Enzymaktivität des ersten Proteins inhibiert, aber nicht an das zweite Protein bindet und dessen Enzymaktivität nicht inhibiert, umfasst.

3. Verfahren nach Anspruch 1, wobei Region (i) oben eine Aminosäuresequenz mit einer Sequenzhomologie von nicht weniger als 70 % mit der Aminosäuresequenz, gezeigt von Aminosäure 46 - 454 in der von SEQ ID NR.: 2 gezeigten Aminosäuresequenz, aufweist.

4. Kit zum Screenen nach einer β-Sekretase inhibierende Substanz, die spezifisch an eine Transmembranregion der β-Sekretase bindet, umfassend die folgenden (a) und (b)
(a) ein erstes Protein mit einem Teil von oder der gesamten Aminosäuresequenz der β-Sekretase, umfassend
(i) eine Region, die ein aktives Zentrum der β-Sekretase umfasst, und
(ii) eine Region, die einen Teil von oder die gesamte Transmembranregion der β-Sekretase umfasst; und
(b) ein zweites Protein mit einem Teil einer Aminosäuresequenz der β-Sekretase, umfassend eine Region (i) oben in dem ersten Protein und ohne Region (ii) oben in dem ersten Protein,
wobei Region (i) eine Aminosäuresequenz mit einer Sequenzhomologie von nicht weniger als 70 % mit der Aminosäuresequenz, gezeigt von Aminosäure 93 - 292 in der von SEQ ID NR.: 2 gezeigten Aminosäuresequenz, umfasst,
die Transmembranregion der β-Sekretase eine Aminosäuresequenz mit einer Sequenzhomologie von nicht weniger als 70 % mit der Aminosäuresequenz, gezeigt von Aminosäure 455 - 480 in der von SEQ ID NR.: 2 gezeigten Aminosäuresequenz, aufweist, und
die Region (ii) oben eine Region mit einer Aminosäuresequenz mit einer Sequenzhomologie von nicht weniger als 70 % mit der Aminosäuresequenz, gezeigt von Aminosäure 466 - 471 in der von SEQ ID NR.: 2 gezeigten Aminosäuresequenz, umfasst.

5. Kit nach Anspruch 4, wobei Region (i) oben eine Aminosäuresequenz mit einer Sequenzhomologie von nicht weniger als 70 % mit der Aminosäuresequenz, gezeigt von Aminosäure 46 - 454 in der von SEQ ID NR.: 2 gezeigten Aminosäuresequenz, aufweist.

## Revendications

1. Procédé de recherche par criblage d'une substance inhibant la β-sécrétase qui se lie spécifiquement à un domaine trans-membranaire de la (3-sécrétase, lequel procédé comporte les étapes suivantes :
I) évaluer l'activité d'une première protéine comportant tout ou partie de la séquence d'acides aminés de la β-sécrétase et comprenant :
i) une région comprenant un centre actif de la β-sécrétase,
ii) et une région comprenant tout ou partie d'un domaine transmembranaire de la β-sécrétase, comprenant au moins un site de liaison qui est la cible d'une substance présentant l'activité d'inhibition d'enzyme en question ;
II) évaluer la liaison de la substance testée à cette première protéine ;
III) et sélectionner, en tant que substance inhibant la β-sécrétase, une substance testée qui inhibe l'activité de la β-sécrétase en se liant au domaine transmembranaire (ii) de la β-sécrétase ;
étant entendu
que la région (i) comprend une séquence d'acides aminés qui présente un degré d'homologie de séquence d'au moins 70 % avec la séquence des acides aminés n° 93 à 292 de la séquence d'acides aminés présentée en tant que Séquence N° 2,
que le domaine transmembranaire de la β-sécrétase comporte une séquence d'acides aminés qui présente un degré d'homologie de séquence d'au moins 70 % avec la séquence des acides aminés n° 455 à 480 de la séquence d'acides aminés présentée en tant que Séquence N° 2,
et que la région (ii) définie ci-dessus comprend une région comportant une séquence d'acides aminés qui présente un degré d'homologie de séquence d'au moins environ 70 % avec la séquence des acides aminés n° 466 à 471 de la séquence d'acides aminés présentée en tant que Séquence N° 2.

2. Procédé conforme à la revendication 1, qui comporte en outre l'étape suivante :
I') évaluer l'activité d'une deuxième protéine comportant une partie de la séquence d'acides aminés de la β-sécrétase, comprenant la région (i) définie plus haut de la première protéine, mais privée de tout ou partie de la région (ii) définie plus haut de la première protéine, en présence de la substance testée ;
et dans lequel l'évaluation réalisée dans l'étape (II) indiquée plus haut comprend les opérations suivantes :
a) évaluer la liaison de la substance testée à la première protéine,
b) évaluer la liaison de la substance testée à la deuxième protéine,
c) et déterminer que la substance testée se lie à la β-sécrétase dans la région (ii) définie plus haut si la substance testée se lie à la première protéine, mais ne se lie pas à la deuxième protéine,
et la sélection réalisée dans l'étape (III) indiquée plus haut comprend l'opération suivante :
d) sélectionner, en tant que substance inhibant la β-sécrétase qui inhibe l'activité de la β-sécrétasse en se liant au domaine transmembranaire (ii) de la β-sécrétase, une substance testée qui se lie à la première protéine et en inhibe l'activité enzymatique, mais ne se lie pas à la deuxième protéine et n'en inhibe pas l'activité enzymatique.

3. Procédé conforme à la revendication 1, dans lequel la région (i) définie plus haut possède une séquence d'acides aminés qui présente un degré d'homologie de séquence d'au moins 70 % avec la séquence des acides aminés n° 46 à 454 de la séquence d'acides aminés présentée en tant que Séquence N° 2.

4. Trousse pour recherche par criblage d'une substance inhibant la β-sécrétase qui se lie spécifiquement à un domaine trans-membranaire de la β-sécrétase, laquelle trousse comporte les composants suivants (a) et (b) :
a) une première protéine comportant tout ou partie de la séquence d'acides aminés de la β-sécrétase et comprenant :
i) une région comprenant un centre actif de la β-sécrétase,
ii) et une région comprenant tout ou partie du domaine transmembranaire de la β-sécrétase ;
b) et une deuxième protéine comportant une partie de la séquence d'acides aminés de la β-sécrétase, comprenant la région (i) définie ci-dessus de la première protéine, mais privée de la région (ii) définie ci-dessus de la première protéine ;
étant entendu
que la région (i) comprend une séquence d'acides aminés qui présente un degré d'homologie de séquence d'au moins 70 % avec la séquence des acides aminés n° 93 à 292 de la séquence d'acides aminés présentée en tant que Séquence N° 2,
que le domaine transmembranaire de la β-sécrétase comporte une séquence d'acides aminés qui présente un degré d'homologie de séquence d'au moins 70 % avec la séquence des acides aminés n° 455 à 480 de la séquence d'acides aminés présentée en tant que Séquence N° 2,
et que la région (ii) définie ci-dessus comprend une région comportant une séquence d'acides aminés qui présente un degré d'homologie de séquence d'au moins 70 % avec la séquence des acides aminés n° 466 à 471 de la séquence d'acides aminés présentée en tant que Séquence N° 2.

5. Trousse conforme à la revendication 4, dans laquelle la région (i) définie plus haut possède une séquence d'acides aminés qui présente un degré d'homologie de séquence d'au moins 70 % avec la séquence des acides aminés n° 46 à 454 de la séquence d'acides aminés présentée en tant que Séquence N° 2.
